# EUROPÄISCHE PATENTANMELDUNG

(11) **EP 3 862 078 A1**
(43) Veröffentlichungstag der Anmeldung: **11.08.2021**
(21) Anmeldenummer: 20155753.5
(22) Anmeldetag: 05.02.2020
(51) Int. Cl.: B01J 20/34, A61M 1/34, B01J 20/288, B01J 20/32

(54) **VERWENDUNG EINER ALKALIHYDROXID-LÖSUNG ZUR REGENERATION EINER APHERESESÄULE**

(71) Anmelder: Pentracor GmbH, 16761 Hennigsdorf (DE)
(72) Erfinder: SHERIFF, Ahmed, 10713 Berlin (DE); VOGT, Birgit, 13353 Berlin (DE); MATTECKA, Stephan, 13053 Berlin (DE)
(74) Vertreter: Arth, Hans-Lothar

(57) **Zusammenfassung**

Die vorliegende Erfindung betrifft eine Verwendung einer Alkalihydroxid-Lösung zur Regeneration von Apheresesäulen sowie ein Verfahren zur vereinfachten Regeneration von Apheresesäulen unter der Verwendung einer Alkalihydroxid-Lösung und Apheresevorrichtungen die derart ausgestaltet sind, dass diese gegenüber Alkalihydroxid-Lösungen beständig sind.

## Beschreibung

Die vorliegende Erfindung betrifft eine Verwendung von Alkalihydroxid zur Regeneration von Apheresesäulen sowie ein Verfahren zur vereinfachten Regeneration von Apheresesäulen unter der Verwendung einer Alkalihydroxid-Lösung und Apheresevorrichtungen die derart ausgestaltet sind, dass diese gegenüber Alkalihydroxid-Lösungen beständig sind.

### Hintergrund der Erfindung

Nach Angaben der Weltgesundheitsorganisation (World Health Organization, WHO) starben im Jahr 2008 ca. 17.000.000 Menschen an kardiovaskulären Erkrankungen. Damit sind kardiovaskuläre Erkrankungen die häufigste Todesursache unter den nichtübertragbaren Krankheiten und verantwortlich für ungefähr ein Drittel der jährlich weltweit auftretenden Todesfälle. Nach Schätzungen wird diese Zahl bis zum Jahr 2030 auf ungefähr 23.000.000 Todesfälle pro Jahr ansteigen.

Somit sind und bleiben kardiovaskuläre Erkrankungen nicht nur die Haupttodesursache weltweit, sondern verursachen auch enorme medizinische Kosten für die nationalen Gesundheitssysteme und Krankenkassen. Zwei der häufigsten und schädlichsten Erscheinungen kardiovaskulärer Erkrankungen sind das Auftreten von Arteriosklerose und Thrombose, welche wiederum unter anderem ursächlich für Herzinfarkte und Schlaganfälle sind.

In den letzten Jahren wurden große Fortschritte in der Behandlung von kardiovaskulären Erkrankungen erzielt. Dieser Fortschritt wurde nicht nur durch wachsende Erkenntnisse hinsichtlich der Krankheits-auslösenden Mechanismen ermöglicht, sondern auch durch die frühzeitige Identifizierung von Risiko-Patienten. Tatsächlich sind die Identifizierung von Erkrankungsrisiken und deren frühzeitige Behandlung wichtige Merkmale der modernen medizinischen Praxis. In den letzten 25 Jahren wurde eine Vielzahl an Faktoren und klinischen Parametern identifiziert, die entweder mit dem gegenwärtigen Zustand der Erkrankung oder mit der zukünftigen Wahrscheinlichkeit für eine kardiovaskuläre Erkrankung korrelieren. Solche Risikofaktoren können messbare biochemische oder physiologische Parameter wie z.B. Serum-Cholesterin-, HDL-, LDL-, und Fibrinogen-Spiegel sein aber auch Verhaltensmuster wie z.B. Übergewicht und Rauchen beinhalten. In Fällen, in denen ein Risikofaktor nicht lediglich auf eine Erkrankung oder deren Entstehung hinweist, sondern tatsächlich ursächlich an deren Entstehung beteiligt ist, kann eine therapeutische Beeinflussung dieses Risikofaktors den Verlauf der Erkrankung beeinflussen bzw. das Risiko ihrer Entstehung verringern.

CRP ist als Akute-Phase-Protein Teil des angeborenen Immunsystems und wird in der Leber im Zuge von Entzündungsreaktionen gebildet und ins Blut abgegeben. Die Bildung von CRP wird hierbei in erster Linie durch Cytokine induziert, die im Rahmen einer akuten oder chronischen Entzündungsreaktion exprimiert werden. Der stärkste Stimulus zur Bildung von CRP ist hierbei das Interleukin-6 (IL-6). Deshalb sind die Spiegel von CRP ebenso wie von IL-6 im Blut Indikatoren für eine lokale oder systemische Entzündungsreaktion. Chronische Entzündungen werden als eine der zugrundeliegenden und unterstützenden pathologischen Erscheinungen bei kardiovaskulären Erkrankungen vermutet. Hierbei wird zunehmend davon ausgegangen, dass CRP nicht nur prädiktiv für kardiovaskuläre Erkrankungen ist, sondern auch kausal an deren Entstehung beteiligt ist bzw. deren Verlauf beeinflussen kann.

Der Normalwert für CRP im Blut von Menschen variiert von Person zu Person, liegt aber im Median bei ungefähr 0,8 mg CRP pro Liter Blut, kann aber im Fall von akuten oder chronischen Entzündungsreaktionen (z.B. bakterielle Infektionen, Atherosklerose, nach einem Herzinfarkt) auf deutlich über 100 mg CRP pro Liter Blut steigen. Da die Halbwertszeit von CRP im Blut (ca. 19 Stunden) konstant und damit unabhängig vom gesundheitlichen Zustand des Patienten ist, ist allein die Syntheserate von CRP für die Regulation des CRP-Spiegels im Blut verantwortlich (Pepys & Hirschfield, J. Clin. Invest., 2003, 111: 1805-1812). Die stark erhöhte Synthese an CRP bei akuten pathologischen Konditionen stellt folglich besondere Anforderungen an therapeutische Ansätze zur CRP-Entfernung von Patienten (Risikopatienten oder Akut-Patienten), da eine erhebliche Menge an CRP entfernt werden muss, um den CRP-Spiegel im Blut auf Normalwerte zu senken. Es besteht also ein Bedarf an besonders effizienten Vorrichtungen und Randbedingungen zur CRP-Entfernung aus dem Blut von Patienten.

In der DE 4338858 C1 wird eine Vorrichtung zur Regeneration einer Apheresesäule offenbart. Die DE 4338858 C1 lehrt die Verwendung eines Speichers worin das Plasma während der Regeneration der Apheresesäule zwischengespeichert wird. Die Regeneration der Apheresesäule findet über die aus dem Stand der Technik bekannte Kombination von Glycin-/HCL, PBS und NaCI-Lösung statt. Ferner offenbart die DE 4338858 C1 keine Bypass-Leitung die eine Umleitung des Plasmaflusses unter Umgehung der Apheresesäule, während der Regeneration derselben ermöglicht.

Zur Regeneration der Apheresesäule insbesondere von CRP-Apheresesäulen werden Glycin/HCl oder EDTA-Lösungen eingesetzt, die durch eine sprunghafte pH-Wert-Änderung in den sauren Bereich gebundene Proteine denaturieren oder durch die Komplexierung der bindungsvermittelnden Kationen insbesondere des Calciums die Bindung zwischen dem adsorbierten zu entfernenden Molekül insbesondere CRP und dem Trägermaterial der Apheresesäule lösen. Im Stand der Technik sind keine Verbindungen bekannt, die über einen anderen Mechanismus die Abtrennung von gebundenen zu entfernenden Molekülen insbesondere CRP an einem Trägermaterial der Apheresesäule ermöglichen.

Die Apheresesäulen werden für einen Patienten in mehreren Behandlungssitzungen verwendet. Dazu werden die Apheresesäulen nach der Behandlung in einer Konservierungsflüssigkeit gelagert. Im Stand der Technik wird am Ende der Apheresebehandlung die Apheresesäule mit einer Regenerationslösung behandelt, anschließend mit einer Spüllösung wie physiologische Kochsalzlösung nachgespült und zum Schluss eine Konservierungsflüssigkeit wie eine 0,04% Polyhexmethylen-BiguanidLösung (PHMB-Lösung) oder eine Lösung aus Natriumazid und PBS (Phosphate Buffered Saline) in die Apheresesäule eingebracht. Daher ist der Konservierungsschritt mit einem erheblichen zeitlichen Aufwand für das klinische Personal verbunden. Sofern es sich bei der Regenerationslösung und dem Konservierungsmittel um dieselbe Lösung handeln würde, könnten zwei zusätzliche Schritte für die Konservierung der Apheresesäule vermieden werden.

Es wurde von den Anmeldern gefunden, dass sich bei der Regeneration mit Glycin/HCl eine Proteinschicht um die Matrixpartikel (z.B. Agarosepartikel) bildet. Dies beruht vermutlich auf einer sauren Proteinfällung. Wenn das zu reinigende Blut des Patienten eine hohe Konzentration von zellfreier DNA/RNA enthält, kann dies zu einer Verstärkung des Effekts führen. Durch die Ausbildung der Proteinschicht in der Apheresesäule werden Bindungsstellen maskiert und die Leistung des Apheresematerials verringert sich. Der ursprüngliche Zustand kann mit bekannten Maßnahmen wie weiteren Regenerationsversuchen mit einer Glycin/HCl-Lösung nicht wiederhergestellt werden. Dieses Problem wurde im Stand der Technik bislang nicht beschrieben. Mit fortschreitender Schädigung der Apheresesäule erhöht sich die Behandlungszeit für den Patienten und somit die Leidenszeit des Patienten. Zudem sind die beschädigten Apheresesäulen für einen weiteren Einsatz oft nicht mehr brauchbar, sodass die Behandlungskosten erheblich steigen.

Weiterhin kann es durch die Proteinschicht bzw. Protein-DNA sowie Protein-RNA Schicht zu einer Verstopfung der feinen Poren kommen, wodurch der Systemdruck bei gleichbleibender Flussrate steigt. Eine weitere Erhöhung der Flussrate geht mit einer weiteren Druckerhöhung einher. Dies kann zu einem Abbruch der Behandlung führen. Diese Apheresesäulen sind ebenfalls für einen weiteren Einsatz nicht mehr brauchbar.

Daher ist die Aufgabe der vorliegenden Erfindung Verwendungen, Verfahren und Vorrichtungen bereitzustellen, die es ermöglichen, die Maskierung der Bindungsstellen und die Erhöhung der Behandlungszeit der Apherese zu verhindern und insbesondere den reibungslosen Ablauf zu gewährleisten.

Eine weitere Aufgabe der vorliegenden Erfindung ist es, Verwendungen, Verfahren und Vorrichtungen zur vereinfachten Regeneration von Apheresesäulen bereitzustellen, wobei die angeführten Nachteile der aus dem Stand der Technik bekannten Vorrichtungen minimiert werden. Anders formuliert besteht die Aufgabe der vorliegenden Erfindung darin eine Vorrichtung zur vereinfachten Regeneration von Apheresesäulen zur Verfügung zu stellen, welche mit reduziertem Trainingsaufwand, und damit verbunden mit einem reduzierten Personalaufwand und reduzierten Gesamtkosten bedient werden können.

Diese Aufgabe wird durch die Lehre der unabhängigen Ansprüche gelöst. Weitere vorteilhafte Ausgestaltungen ergeben sich aus der Beschreibung, den Beispielen und den anhängigen Patentansprüchen.

### Beschreibung der Erfindung

Überraschenderweise konnte gefunden werden, dass eine verwendete Apheresesäule, d.h. eine Apheresesäule, nach der Behandlung zur Entfernung von Substanzen aus dem Blut, mithilfe einer Alkalihydroxid-Lösung vorzugsweise einer Natriumhydroxid-Lösung regeneriert werden kann und die auf diese Weise regenerierte Apheresesäule für die Apherese weiterhin nutzbar ist. Vor allem lassen sich so Apheresesäulen regenerieren, welche durch ihre Verwendung Proteinablagerungen und/oder Protein-DNA- und Protein-RNA-Ablagerungen aufweisen, welche durch die im Stand der Technik verwendeten Regenerationslösungen nicht mehr entfernt werden können und diese Apheresesäulen damit unbrauchbar geworden sind.

Daher betrifft die zugrundeliegende Erfindung die Verwendung einer Alkalihydroxid-Lösung vorzugsweise einer Natriumhydroxid-Lösung zur Regeneration einer Apheresesäule. Anders formuliert ist die vorliegende Erfindung gerichtet auf die Verwendung einer Alkalihydroxid-Lösung vorzugsweise einer Natriumhydroxid-Lösung als Regenerationslösung bei der Apherese. Noch anders formuliert betrifft die vorliegende Erfindung die Verwendung einer Alkalihydroxid-Lösung vorzugsweise einer Natriumhydroxid-Lösung als Regenerationslösung für eine Apheresesäule.

Überraschenderweise wurde gefunden, dass eine basische Regeneration mit einer Alkalihydroxid-Lösung vorzugsweise einer Natriumhydroxid-Lösung auch eine bereits geschädigte Adsorbermatrix in der Apheresesäule regenerieren kann. Wird nur Alkalihydroxid-Lösung vorzugsweise Natriumhydroxid-Lösung als Regenerationsmittel verwendet, kann keine saure Proteinfällung stattfinden. Dies ist ein wichtiger Aspekt bei der Wiederherstellung der Funktion einer Apheresesäule.

Des Weiteren ist vorteilhaft, dass die Apheresesäule über einer Alkalihydroxid-Lösung vorzugsweise einer Natriumhydroxid-Lösung als Konservierungsmittel gelagert werden kann. In bevorzugten Ausführungsformen kann die Alkalihydroxid-Lösung vorzugsweise die Natriumhydroxid-Lösung zur Konservierung einer Apheresesäule verwendet werden.

Dadurch ist es möglich Apheresebehandlungen einschließlich der Vorbereitung der Lagerung der Apheresesäule möglichst effizient zu gestalten, da nach der Behandlung aufwändige Waschschritte der Apheresesäule vermieden werden können. Alkalihydroxid-Lösung ist geeignet für die Konservierung der Apheresesäule, insbesondere ist Natriumhydroxid-Lösung perfekt geeignet für die Konservierung der Apheresesäule, da es sehr gut sterilisiert. Mikrobenwachstum ist praktisch ausgeschlossen.

Ein Weiterer Aspekt der vorliegenden Erfindung betrifft daher die Verwendung einer Alkalihydroxid-Lösung vorzugsweise einer Natriumhydroxid-Lösung zur Regeneration und Konservierung einer Apheresesäule. Daher ist die vorliegende Erfindung ebenfalls gerichtet auf die Verwendung einer Alkalihydroxid-Lösung vorzugsweise einer Natriumhydroxid-Lösung zur Regeneration und Konservierung einer Apheresesäule. Mit anderen Worten ist die vorliegende Erfindung auch gerichtet auf die Verwendung einer Alkalihydroxid-Lösung vorzugsweise einer Natriumhydroxid-Lösung als Regenerationslösung bei der Apherese und zur Konservierung der Apheresesäule.

Im Gegensatz zu den im Stand der Technik bekannten Mitteln zur Regeneration einer Apheresesäule zeichnet sich eine Alkalihydroxid-Lösung vorzugsweise eine Natriumhydroxid-Lösung dadurch aus, dass es sich nicht um einen Chelator für Kationen handelt. Es wird angenommen, dass das Alkalihydroxid vorzugsweise Natriumhydroxid einerseits die Bindungsaffinität zum Trägermaterial des abzulösenden Stoffes verringert und andererseits evtl. gebundene Proteine denaturiert und somit die Bindung zum Trägermaterial herabsetzt. Durch die schnelle Regeneration kann die Säule während der Apheresebehandlung wieder in einen verwendbaren Zustand überführt werden. Die Regeneration der Apheresesäule kann während einer Apheresebehandlung durchgeführt werden, hat aber auf die Apheresebehandlung selbst keinen Einfluss und ist daher auch kein Diagnose- oder Therapieverfahren, sondern ein Reinigungsverfahren für die Apheresesäule, welche den Apheresepatienten nicht involviert, selbst wenn die Regeneration der Apheresesäule während der Apheresebehandlung stattfindet.

In einer hierin beschriebenen erfindungsgemäßen Verwendung einer Alkalihydroxid-Lösung vorzugsweise einer Natriumhydroxid-Lösung zur Regeneration einer Apheresesäule kann die Regeneration der Apheresesäule während der Apheresebehandlung stattfinden. Bevorzugt findet in einer hierin beschriebenen erfindungsgemäßen Verwendung einer Alkalihydroxid-Lösung vorzugsweise einer Natriumhydroxid-Lösung zur Regeneration einer Apheresesäule die Regeneration der Apheresesäule während der Entfernung der Zielverbindung insbesondere von CRP statt. Eine bevorzugte Ausführungsform der vorliegenden Erfindung ist mithin gerichtet auf die Verwendung einer Alkalihydroxid-Lösung vorzugsweise einer Natriumhydroxid-Lösung zur Regeneration einer Apheresesäule, wobei die Regeneration während einer Apheresebehandlung erfolgt.

"Während einer Apheresebehandlung" bzw. "während der extrakorporalen Entfernung von Zielverbindungen insbesondere CRP aus Blut", bedeutet hierin, dass die Regeneration der Apheresesäule während des Behandlungsverfahrens in der gleichen Apheresevorrichtung erfolgt. Dabei kann die extrakorporale Entfernung der Zielverbindung insbesondere von CRP aus Blut unterbrochen und mittels der gleichen Apparatur die Apheresesäule regeneriert werden. Alternativ kann die Regeneration einer Apheresesäule ausgeführt werden, während zeitgleich über eine weitere Apheresesäule die extrakorporale Entfernung der Zielverbindung insbesondere von CRP aus Blut fortgesetzt wird. Die Regeneration der Apheresesäule erfolgt in der Apheresevorrichtung. Unter dem Begriff "während einer Apheresebehandlung" ist jedoch nicht zu verstehen, dass die Regeneration und Entfernung von CRP aus Blut zeitgleich auf derselben Apheresesäule stattfindet.

Des Weiteren wurde, wie bereits erwähnt, von den Erfindern erkannt, dass sich bei der Regeneration mit Glycin/HCl eine Ablagerung in der Apheresesäule ausbildet (Proteinschicht). Wenn das zu reinigende Blut des Patienten eine hohe Konzentration von zellfreier DNA/RNA enthält, kann dies nochmals zu einer Verstärkung des Effekts führen. Durch die Ausbildung der Ablagerung in der Apheresesäule verklebt die Matrix irreversibel, d.h. der Zustand kann mit bekannten Maßnahmen wie weiteren Regenerationsversuchen mit einer Glycin/HCl-Lösung nicht wiederhergestellt werden. In Fällen der Ablagerung von Proteinen auf dem Säulenmaterial der Apheresesäule bis hin zur Ausbildung einer Proteinschicht auf dem Säulenmaterial der Apheresesäule ist eine Regeneration der Apheresesäule mit bekannten Maßnahmen, wie z.B. der Spülung mit einer Glycin/HCl-Lösung nicht mehr möglich, da die Proteinablagerungen oder die Proteinschicht mit herkömmlichen Regenerationslösungen nicht mehr entfernt werden können. Überraschend wurde gefunden, dass eine 0,01 M bis 1 M Alkalihydroxid-Lösung, bevorzugt eine 0,04 M bis 0,4 M Alkalihydroxid-Lösung in der Lage ist, die Proteinablagerungen wieder vorzugsweise vollständig zu entfernen. Überraschend wurde gefunden, dass insbesondere eine 0,01 M bis 1 M Natriumhydroxid-Lösung, bevorzugt eine 0,04 M bis 0,4 M Natriumhydroxid-Lösung in der Lage ist, die Proteinablagerungen wieder vorzugsweise vollständig zu entfernen.

Daher beziehen sich die erfindungsgemäßen Verwendungen vorzugsweise auf eine DNA/RNA-enthaltende Apheresesäule (freie DNA bzw. freie RNA), vorzugsweise auf eine Apheresesäule, an deren Matrix gebundene DNA bzw. RNA vorhanden ist, und insbesondere bevorzugt auf Apheresesäulen, an denen DNA bzw. RNA an die Liganden der Matrix gebunden ist.

Eine bevorzugte Ausführungsform der vorliegenden Erfindung bezieht sich daher auf die Verwendung einer Alkalihydroxid-Lösung vorzugsweise einer Natriumhydroxid-Lösung zur Regeneration einer Apheresesäule, wobei die Apheresesäule DNA- und/oder RNA-Ablagerungen enthält, d.h. auf dem Adsorber oder wie auch hierin bezeichnet auf der Adsorbermatrix enthält.

"Freie DNA" bzw. "Freie RNA", wie hierin verwendet, bezieht sich auf Desoxyribonukleinsäure bzw. Ribonukleinsäure, die sich außerhalb von Zellen befindet. An Liganden der Apheresesäule gebundene DNA/RNA wird ebenfalls als freie DNA/freie RNA bezeichnet, da diese sich auch außerhalb von Zellen befindet.

### Regenerationslösung

Regeneration, wie hierin verwendet, bezeichnet den Prozess bei dem die Matrix der Apheresesäule umfassend ein Matrixsubstratmaterial und einen Liganden, wovon angelagerte Substanzen zu entfernen sind, wieder in einen therapeutisch verwendbaren Zustand gebracht werden, d.h. regeneriert werden.

Der Begriff **"Regenerationslösung",** wie hierin verwendet, bezeichnet bei einem affinitätschromatographischen Verfahren zur Entfernung einer Substanz aus einer Probe (hier biologische Flüssigkeiten wie z.B. Blut oder Blutplasma) eine Lösung, die nach der Auftragung der Probe auf das Säulenmaterial und der erfolgten spezifischen Bindung der zu entfernenden Substanz an das Säulenmaterial, aufgetragen wird, um diese spezifische Bindung wieder zu lösen und somit die zu entfernende Substanz wieder von dem Säulenmaterial zu lösen (bzw. zu eluieren).

Glycin-HCI-Lösungen für die Verwendung als Regenerationslösung sind aus dem Stand der Technik bekannt. Die Glycin/HCl-Lösung weisen einen pH-Wert im Bereich von 2-3 auf z.B. pH = 2,8. Die Konzentration der Glycin-HCI-Lösung kann zwischen 0,1 M bis 1 M liegen. Zur Herstellung einer Glycin-HCI-Lösung oder synonym verwendet einem Glycin-Salzsäure-Puffer werden 15,01g Glycin in einem Liter Wasser gelöst und mit 25%iger Salzsäure versetzt. Der pH kann mittels Salzsäure oder Natriumhydroxid eingestellt werden. Die Herstellung der Glycin-HCI-Lösung ist dem Fachmann bekannt.

"Natriumhydroxid-Lösung" bezeichnet hierin, eine Lösung umfassend Natriumhydroxid in einem Lösungsmittel wie Wasser oder Alkohol wie Methanol, Ethanol, Propanol, etc. oder in einem Lösungsmittelgemisch aus Wasser und mindestens einem Alkohol. Bevorzugt handelt es sich um eine Lösung umfassend oder bestehend aus Natriumhydroxid in Wasser. Bevorzugt ist somit eine wäßrige Natriumhydroxid-Lösung (auch bezeichnet als NaOH_{aq}).

Erhältlich ist Natriumhydroxid in unterschiedlichen Reinheitsgraden, bevorzugt ist ein Reinheitsgrad von 98%, weiter bevorzugt 99%, weiter bevorzugt 99.5%, weiter bevorzugt von 99,9% und am bevorzugtesten von 99,99%. Bevorzugt ist ein Reinheitsgrad von 98% bis 100%.

Grundsätzlich kann das Natriumhydroxid in der Natriumhydroxid-Lösung in allen verfügbaren Konzentrationen (bezogen auf Natriumhydroxid in einem Lösungsmittel oder Lösungsmittelgemisch) vorliegen. In der Regel handelt es sich um eine wäßrige Natriumhydroxid-Lösung. Bevorzugt ist jedoch eine Konzentration des Natriumhydroxids in der Natriumhydroxid-Lösung in einem Bereich von 0,005 mol/l bis 1,0 mol/l, noch weiter bevorzugt zwischen 0,01 mol/l bis 1,0 mol/l, noch weiter bevorzugt zwischen 0,02 mol/l bis 0,80 mol/l, noch weiter bevorzugt zwischen 0,03 mol/l bis 0,60 mol/l, noch weiter bevorzugt zwischen 0,04 mol/l bis 0,50 mol/l, noch weiter bevorzugt zwischen 0,05 mol/l bis 0,40 mol/l, noch weiter bevorzugt zwischen 0,06 mol/l bis 0,30 mol/l, noch weiter bevorzugt zwischen 0,07 mol/l bis 0,20 mol/l, noch weiter bevorzugt zwischen 0,08 mol/l bis 0,10 mol/l.

Eine bevorzugte Ausführungsform der vorliegenden Erfindung betrifft die Verwendung einer Alkalihydroxid-Lösung, bevorzugt einer Natriumhydroxid-Lösung zur Regeneration einer Apheresesäule, wobei die Konzentration des Alkalihydroxids, vorzugsweise des Natriumhydroxids in der Lösung in einem Bereich von 0,01-1,0 mol/l liegt.
Eine andere bevorzugte Ausführungsform der vorliegenden Erfindung betrifft die Verwendung einer Alkalihydroxid-Lösung, bevorzugt einer Natriumhydroxid-Lösung zur Regeneration einer Apheresesäule, wobei die Konzentration des Alkalihydroxids, vorzugsweise des Natriumhydroxids in der Lösung in einem Bereich von 0,05 - 0,20 mol/l, bevorzugt von 0,05 - 0,10 mol/l, weiter bevorzugt von 0,07 - 0,10 mol/l liegt.

Der basische pH-Wert der Alkalihydroxid-Lösung vorzugsweise der Natriumhydroxid-Lösung ist nicht beschränkt. Bevorzugt weist die Alkalihydroxid-Lösung vorzugsweise die Natriumhydroxid-Lösung einen pH-Wert in einem Bereich von pH 7 bis 14, mehr bevorzugt von 7,5 - 14, mehr bevorzugt von 8,0 bis 14,0, mehr bevorzugt von 8,5 bis 14, mehr bevorzugt von 9,0 bis 14,0, mehr bevorzugt von 9,5 bis 14,0, mehr bevorzugt von 10,5 bis 14,0, mehr bevorzugt von 11 bis 14,0, mehr bevorzugt von 11,5 bis 14,0, mehr bevorzugt von 12 bis 14,0, mehr bevorzugt von 12,5 bis 14,0 und noch bevorzugter von 13 bis 14 auf. Besonders bevorzugt besitzt eine Natriumhydroxid-Lösung einen pH Wert von 12 bis 13,7.

Eine Ausführungsform der vorliegenden Erfindung ist daher die Verwendung einer Alkalihydroxid-Lösung vorzugsweise einer Natriumhydroxid-Lösung zur Regeneration einer Apheresesäule, wobei die Alkalihydroxid-Lösung vorzugsweise die Natriumhydroxid-Lösung einen pH-Wert in einem Bereich von 12 bis 14 aufweist.

Der Begriff "Alkalihydroxid-Lösung" oder alternativ auch "Alkalimetallhydroxid-Lösung" wie hierin verwendet bezeichnet eine "Lithiumhydroxid-Lösung", "Natriumhydroxid-Lösung", "Kaliumhydroxid-Lösung" oder eine Mischung von zwei oder drei der vorgenannten Lösungen.

"Lithiumhydroxid-Lösung" bezeichnet hierin, eine Lösung umfassend Lithiumhydroxid in einem Lösungsmittel wie Wasser oder Alkohol wie Methanol, Ethanol, Propanol, etc. oder in einem Lösungsmittelgemisch aus Wasser und mindestens einem Alkohol. Bevorzugt handelt es sich um eine Lösung umfassend oder bestehend aus Lithiumhydroxid in Wasser.

"Kaliumhydroxid-Lösung" bezeichnet hierin, eine Lösung umfassend Kaliumhydroxid in einem Lösungsmittel wie Wasser oder Alkohol wie Methanol, Ethanol, Propanol, etc. oder in einem Lösungsmittelgemisch aus Wasser und mindestens einem Alkohol. Bevorzugt handelt es sich um eine Lösung umfassend oder bestehend aus Kaliumhydroxid in Wasser.

Grundsätzlich kann das Lithiumhydroxid in der Lithiumhydroxid-Lösung bzw. das Kaliumhydroxid in der Kaliumhydroxid-Lösung in allen verfügbaren Konzentrationen (bezogen auf Lithiumhydroxid in einem Lösungsmittel oder Lösungsmittelgemisch bzw. bezogen auf Kaliumhydroxid in einem Lösungsmittel oder Lösungsmittelgemisch) vorliegen. Bevorzugt ist jedoch eine Konzentration des Lithiumhydroxids in der Lithiumhydroxid-Lösung bzw. des Kaliumhydroxids in der Kaliumhydroxid-Lösung in einem Bereich 0,005 mol/l bis 1,0 mol/l, noch weiter bevorzugt zwischen 0,01 mol/l bis 1,0 mol/l, noch weiter bevorzugt zwischen 0,02 mol/l bis 0,80 mol/l, noch weiter bevorzugt zwischen 0,03 mol/l bis 0,60 mol/l, noch weiter bevorzugt zwischen 0,04 mol/l bis 0,50 mol/l, noch weiter bevorzugt zwischen 0,05 mol/l bis 0,40 mol/l, noch weiter bevorzugt zwischen 0,06 mol/l bis 0,30 mol/l, noch weiter bevorzugt zwischen 0,07 mol/l bis 0,20 mol/l, noch weiter bevorzugt zwischen 0,08 mol/l bis 0,10 mol/l.

Werden zwei der drei vorgenannten Alkalihydroxid-Lösungen verwendet, so sollte die kumulierte Konzentration der beiden Alkalihydroxid-Lösungen in einem Bereich 0,005 mol/l bis 1,0 mol/l, noch weiter bevorzugt zwischen 0,01 mol/l bis 1,0 mol/l, noch weiter bevorzugt zwischen 0,02 mol/l bis 0,80 mol/l, noch weiter bevorzugt zwischen 0,03 mol/l bis 0,60 mol/l, noch weiter bevorzugt zwischen 0,04 mol/l bis 0,50 mol/l, noch weiter bevorzugt zwischen 0,05 mol/l bis 0,40 mol/l, noch weiter bevorzugt zwischen 0,06 mol/l bis 0,30 mol/l, noch weiter bevorzugt zwischen 0,07 mol/l bis 0,20 mol/l, noch weiter bevorzugt zwischen 0,08 mol/l bis 0,10 mol/l liegen. Die bedeutet z.B., dass die Konzentration von Kaliumhydroxid in der Lösung beispielsweise 0,06 mol/l beträgt und von Natriumhydroxid in derselben Lösung beispielsweise 0,08 mol/l beträgt, so dass sich daraus die kumulierte Alkalihydroxid-Konzentration in der Lösung von 0,14 mol/l ergibt. Gleiches gilt, wenn alle drei vorgenannten Alkalihydroxide in einer Lösungen verwendet werden, z.B. Lithiumhydroxid mit 0,04 mol/l, Kaliumhydroxid mit 0,02 mol/l und Natriumhydroxid mit 0,05 mol/l, so dass sich eine kumulierte Alkalihydroxid-Konzentration in der Lösung von 0,11 mol/l ergibt.

Eine Ausführungsform der vorliegenden Erfindung betrifft daher die Verwendung einer Alkalihydroxid-Lösung ausgewählt aus einer Gruppe umfassend oder bestehend aus Natriumhydroxid-Lösung, Lithiumhydroxid-Lösung und/oder Kaliumhydroxid-Lösung zur Regeneration einer Apheresesäule.

Besonders bevorzugt ist jedoch die Verwendung die Verwendung einer Natriumhydroxid-Lösung zur Regeneration einer Apheresesäule.

Zudem sind die Alkalihydroxid-Lösungen in der Lage, Adsorber zu regenerieren, auf denen sich bereits Proteinablagerungen gebildet haben, welche durch herkömmliche Regenerationsmittel nicht mehr zu entfernen sind.

### Apheresesäule

Wie eine derartige Apheresesäule (bzw. Kartusche oder Patrone) prinzipiell gestaltet bzw. aufgebaut sein kann, gehört zum Stand der Technik und geht u.a. aus EP 0237659 B1 hervor. Die genauen Dimensionen der erfindungsgemäß verwendeten Kartusche, Säule oder Patrone (als Vorrichtung zur selektiven Entfernung der Zielverbindung insbesondere von CRP) hängt hierbei maßgeblich von dem angestrebten Verwendungszweck der erfindungsgemäßen Vorrichtung ab. Die Apheresesäule umfasst hierbei in der Regel ein Gehäuse, z.B. in Form einer Kartusche oder einer Patrone, das über mindestens einen Zugang und mindestens einen Ausgang in fluidtechnischer Verbindung zu einem extrakorporalen Zirkulationssystem steht und welches eine Matrix zur affinitätschromatographischen bzw. zur adsorptiven Entfernung der Zielverbindung insbesondere von CRP enthält.

"Zielverbindung" bezeichnet hierin die Substanz, die mittels der Apherese aus Blut entfernt werden soll, d.h. die Substanz, die während der Apherese an der Matrix binden soll.

Die Matrix zur affinitätschromatographischen (oder adsorptiven) Entfernung der Zielverbindung insbesondere von CRP umfasst ein Matrixsubstratmaterial (Säulenmaterial), an welches wiederum Verbindungen (Liganden) gebunden sind, die die Eigenschaft haben, die Zielverbindung insbesondere CRP spezifisch zu binden. Die Matrix ist gemäß einer bevorzugten Ausführungsform der vorliegenden Erfindung derart in die Apheresesäule zur affinitätschromatographischen Entfernung der Zielverbindung insbesondere von CRP integriert bzw. in dieser immobilisiert, dass sie nicht mit dem Blutplasmafluss aus der Säule herausgespült werden kann. Dies kann je nach Ausführungsform z.B. in Form von Filtern am Eingang und Ausgang der Vorrichtung realisiert werden.

Die erfindungsgemäße Verwendung wird vorzugsweise bei der Regeneration von Apheresesäulen zur affinitätschromatographischen Entfernung von CRP genutzt.

Grundsätzlich sind für die Herstellung der Matrix sämtliche inerten Chromatographie- oder Säulenmaterialien als Matrixsubstratmaterialien (Säulenmaterial) geeignet, welche insbesondere nicht mit Blut oder Blutplasma reagieren oder Blut bzw. Blutplasma derart verändern oder kontaminieren, dass das Blut bzw. Blutplasma nach Kontakt mit der Matrix einem Patienten nicht mehr zurückgeführt werden kann. Die erfindungsgemäß geeigneten Matrixsubstratmaterialien umfassen daher, ohne jedoch hierauf eingeschränkt zu sein, Eupergit®, Polyvinylpyrollidon, Methacrylat, Methacrylat-Harze, Agarose, Quervernetzte Agarose wie z.B. Sepharose® (Separation-Pharmacia-Agarose), Acryl-Beads, Zellulosematrizen, Keramikmatrizen, Glas-Beads und/oder Festphasen-Kieselerde oder Mischungen und/oder Derivate dieser Stoffe. Bevorzugt wird das Matrixsubstratmaterial ausgewählt aus der Gruppe umfassend oder bestehend aus Agarose und Sepharose. Besonders bevorzugt handelt es sich bei dem Matrixsubstratmaterial um Agarose. Die Festphasen-Kieselerde-Matrix kann nahezu jede Form von partikulärer Kieselerde umfassen, einschließlich amorpher Kieselerden, wie kolloidale Kieselerde, Kieselgele, präzipitierte Kieselerden, und geräucherte oder pyrogene Kieselerden; mikrokristalline Kieselerden wie Kieselgur; und kristalline Kieselerden wie Quartz.

Während die Flussgeschwindigkeit während der Blutreinigung durch den Blutfluss des Patienten determiniert wird, könnte grundsätzlich bei der Regeneration die Flussrate erhöht werden, um den Vorgang zu beschleunigen, sodass die Apheresesäule für eine weitere Anwendung zur Verfügung steht. Aus einer höheren Flussrate resultiert jedoch zeitgleich eine Erhöhung des Druckes auf die Apherese und somit auf die Matrix bzw. das Säulenmaterial. Da in Abhängigkeit vom Säulenmaterial die Form und Festigkeit des Materials ab einem bestimmten Druck verändert und dadurch die Trennleiste des Säulenmaterials reduziert wird, ist die Flussrate für die Regeneration einer Apheresesäule von der Druckbeständigkeit des verwendeten Säulenmaterials abhängig.

Hierin konnte gefunden werden, dass die Verwendung einer Alkalihydroxid-Lösung vorzugsweise einer Natriumhydroxid-Lösung nicht nur höhere Drücke zulässt, sondern sogar derartig hohe Drücke verwendet werden können, dass Flussraten möglich sind, die eine Reinigung der Apheresesäule in nur wenigen Minuten bzw. Sekunden ermöglichen. Insbesondere eignet sich eine Kombination aus einer Alkalihydroxid-Lösung vorzugsweise einer Natriumhydroxid-Lösung mit Agarose und deren Derivate für eine schnelle Regeneration. Daher bezieht sich eine Ausführungsform der zugrundeliegenden Erfindung auf die Verwendung einer Alkalihydroxid-Lösung vorzugsweise einer Natriumhydroxid-Lösung zur Regeneration einer Apheresesäule, wobei die Apheresesäule Agarose und ihre Derivate enthält. Besonders bevorzugt ist Agarose oder quervernetzte Agarose wie z.B. Sepharose®. Durch die schnelle Regenerationszeit kann gewährleistet werden, dass die Behandlungszeit des Patienten und damit seine Leidenszeit stark herabgesetzt wird.

### Matrixsubstratmaterialien

Erfindungsgemäß werden die an die Matrixsubstratmaterialien gebundenen Verbindungen (Liganden), die die Eigenschaft haben die Zielverbindung insbesondere CRP spezifisch zu binden, ausgewählt aus der Gruppe umfassend oder bestehend aus Lipide, Lysophospholipide, Lysophosphatidylcholin, Peptide, Peptide mit geladenen Aminosäuren, Peptide enthaltend die Sequenz ArgProArg, Phosphocholin, Derivate von Phosphocholin, DNA, DNA-Derivate, RNA, RNA-Derivate, L-Ribonukleinsäureaptamere, wie Spiegelmere® (ein RNA-ähnliches Molekül, welches aus L-Ribose Einheiten besteht), Glycoside, Saccharide und Aptamere.

Der Begriff **"CRP",** wie hierin verwendet, ist gleichbedeutend mit "C-reaktives Protein". Er bezieht sich hierin bevorzugt auf das humane C-reaktive Protein.

In einer hierin beschriebenen erfindungsgemäßen Verwendung einer Alkalihydroxid-Lösung vorzugsweise einer Natriumhydroxid-Lösung zur Regeneration einer Apheresesäule handelt es sich daher bevorzugt um eine CRP-Apheresesäule.

Eine Ausführungsform der vorliegenden Erfindung betrifft daher die Verwendung einer Alkalihydroxid-Lösung vorzugsweise einer Natriumhydroxid-Lösung zur Regeneration einer Apheresesäule, wobei es sich bei der Apheresesäule um eine CRP-Apheresesäule handelt. Zudem betrifft eine weitere Ausführungsform der vorliegenden Erfindung die Verwendung einer Alkalihydroxid-Lösung vorzugsweise einer Natriumhydroxid-Lösung zur Regeneration einer Apheresesäule zur affinitätschromatographischen Entfernung von CRP.

Des Weiteren betrifft eine weitere Ausführungsform der vorliegenden Erfindung die Verwendung einer Alkalihydroxid-Lösung vorzugsweise einer Natriumhydroxid-Lösung zur Regeneration einer Apheresesäule, wobei es sich bei der Apheresesäule um eine CRP-Apheresesäule handelt und die Regeneration während der Entfernung von CRP aus Blut erfolgt.

Zudem betrifft eine weitere Ausführungsform der vorliegenden Erfindung die Verwendung einer Alkalihydroxid-Lösung vorzugsweise einer Natriumhydroxid-Lösung zur Regeneration einer Apheresesäule zur affinitätschromatographischen Entfernung von CRP, wobei die Regeneration während der Entfernung von CRP aus Blut erfolgt.

### Ca²⁺-abhängige Liganden für CRP

Für die affinitätschromatographische Entfernung von CRP aus biologischen Flüssigkeiten, z.B. aus Blut oder Blutplasma, kann ein Säulenmaterial verwendet, dass Phosphocholin und/oder Phosphoethanolamin bzw. deren Derivate enthält, wodurch eine Ca²⁺-abhängige Bindung von CRP an das besagte funktionalisierte Säulenmaterial möglich ist.

Hierzu wird Phosphocholin, Phosphoethanolamin oder deren Derivate an einem Säulenmaterial immobilisiert. Dies geschieht in der Regel über eine organische Linker-Gruppe, über die das Phosphocholin, Phosphoethanolamin bzw. deren Derivate adsorptiv oder noch bevorzugter kovalent mit dem Säulenmaterial verbunden ist. Die resultiert in einem so genannten **"funktionalisierten Säulenmaterial"** (funktionalisiertes Matrixsubstratmaterial), wobei die für die Ca²⁺-abhängige Bindung von CRP verantwortliche chemische Gruppe nach außen exponiert ist, so dass CRP, das sich in einer biologischen Flüssigkeit befindet, auch Zugang zu der besagten chemischen Gruppe hat.

Anders ausgedrückt, bezeichnet der Begriff **"funktionalisiertes Säulenmaterial",** wie hierin verwendet, ein Säulenmaterial zur Affinitätschromatographie, welches mit einer funktionalen chemischen Gruppe (Ligand) versehen wurde. Hierbei kann die funktionale chemische Gruppe über adsorptive oder ionische Wechselwirkungen aber bevorzugt über eine kovalente Bindung mit dem Säulenmaterial verbunden sein. Es ist natürlich von Bedeutung, dass die funktionale chemische Gruppe (Ligand) derart mit dem Säulenmaterial verbunden ist, dass die funktionale Gruppe aktiv und exponiert ist, so dass ihre Funktionalität erhalten bleibt. Hierdurch ist es möglich, dass die am Säulenmaterial befestigte Gruppe (hier: ω-Phosphonooxyalkylammoniumgruppe und/oder ω-Ammoniumalkoxy-hydroxy-phosphoryloxygruppe) mit einer Zielverbindung (hier CRP) aus der Probe (hier: biologische Flüssigkeit wie z.B. Blut oder Blutplasma) interagieren kann bzw. diesen binden kann.

Je nachdem, ob das Phosphocholin, Phosphoethanolamin bzw. deren Derivat über die Ammoniumgruppe oder über die Phosphatgruppe über einen organischen Linker mit dem Säulenmaterial verbunden ist, so unterscheidet man zwischen einem Säulenmaterial, das mit einer ω-Phosphonooxyalkylammoniumgruppe funktionalisiert wurde (Verknüpfung über die Ammoniumgruppe), und einem Säulenmaterial, das mit einer ω-Ammoniumalkoxy-hydroxy-phosphoryloxygruppe funktionalisiert wurde (Verknüpfung über die Phosphatgruppe).

Daher ist eine bevorzugte Ausführungsform der vorliegenden Erfindung die Verwendung einer Alkalihydroxid-Lösung vorzugsweise einer Natriumhydroxid-Lösung zur Regeneration einer Apheresesäule, wobei die Apheresesäule ein Matrixsubstratmaterial funktionalisiert mit mindestens einer ω-Phosphonooxyalkylammoniumgruppe und/oder mit mindestens einer ω-Ammoniumalkoxy-hydroxy-phosphoryloxygruppen umfasst.

Die Verknüpfung mit dem Säulenmaterial (gegebenenfalls über einen organischen Linker) wird in den unten aufgeführten Formeln (**I**) und (**II**) über eine gestrichelte Linie entweder am Stickstoffatom der Ammoniumgruppe oder am Sauerstoffatom der Phosphatgruppe dargestellt.

Der Begriff **"ω-Phosphonooxyalkylammoniumgruppe"** wie hierin verwendet, kann gleichbedeutend mit "omega-Phosphonooxyalkylammonium" verwendet werden und beschreibt Verbindungen der folgenden allgemeinen Formel (**I**) 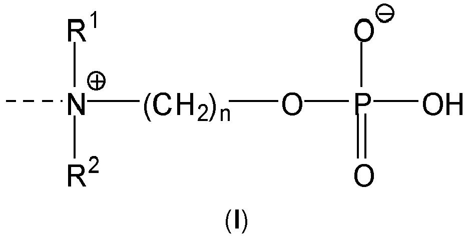 wobei
n ausgewählt wird aus 2 und 3;
R¹ und R² unabhängig voneinander ausgewählt werden aus: -H, -CH₃, -C₂H₅, -C₃H₇, -C₄H₉, -C₅H₁₁, -C₆H₁₃, oder R¹ und R² zusammen mit dem Stickstoffatom mit dem sie verbunden sind einen Heterozyklus bilden können, der ausgewählt wird aus: 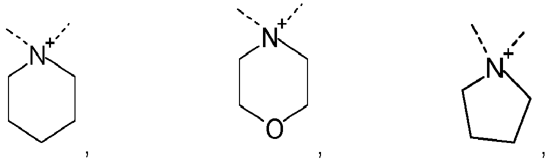 wobei ein oder mehrere Wasserstoffatom(e) durch (ein) Fluoratom(e) ersetzt werden können.

Daher ist eine Ausführungsform der vorliegenden Erfindung gerichtet auf die Verwendung einer Alkalihydroxid-Lösung vorzugsweise einer Natriumhydroxid-Lösung zur Regeneration einer Apheresesäule, wobei die Apheresesäule ein Matrixsubstratmaterial funktionalisiert mit mindestens einer ω-Phosphonooxyalkylammoniumgruppe der folgenden allgemeinen Formeln (I) umfasst: 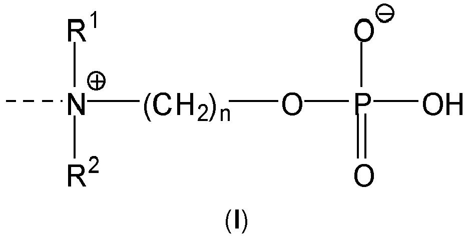 wobei
n ausgewählt wird aus 2 und 3;
R¹ und R² unabhängig voneinander ausgewählt werden aus: -H, -CH₃, -C₂H₅, -C₃H₇, -C₄H₉, -C₅H₁₁, -C₆H₁₃, oder R¹ und R² zusammen mit dem Stickstoffatom mit dem sie verbunden sind einen Heterozyklus bilden können, der ausgewählt wird aus: 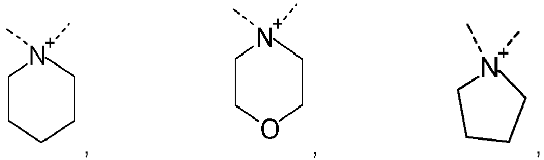 wobei ein oder mehrere Wasserstoffatom(e) durch (ein) Fluoratom(e) ersetzt werden können.

Bevorzugt entspricht die mindestens eine ω-Phosphonooxyalkylammoniumgruppe einer Gruppe der allgemeinen Formel (**I**) 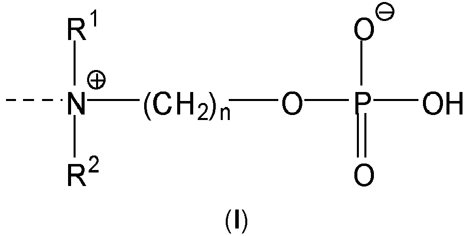 wobei
n = 2 oder 3 ist;
R¹ und R² unabhängig voneinander ausgewählt werden aus: -H, -CH₃, -C₂H₅, -C₃H₇ oder R¹ und R² zusammen mit dem Stickstoffatom mit dem sie verbunden sind einen Heterozyklus bilden können, der ausgewählt wird aus:

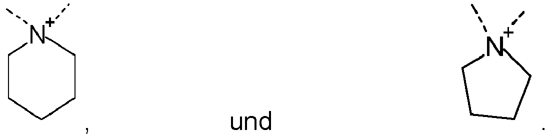

Besonders bevorzugt entspricht die mindestens eine ω-Phosphonooxyalkylammoniumgruppe eine Gruppe der allgemeinen Formel (**I**) 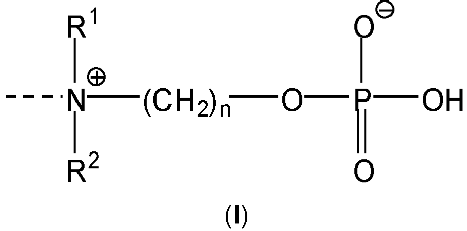 wobei
n = 2 ist;
R¹ und R² ausgewählt werden aus: -H, -CH₃, -C₂H₅, und besonders bevorzugt aus -CH₃ und -C₂H₅ oder R¹ und R² zusammen mit dem Stickstoffatom mit dem sie verbunden sind einen Heterozyklus bilden können, der ausgewählt wird aus:

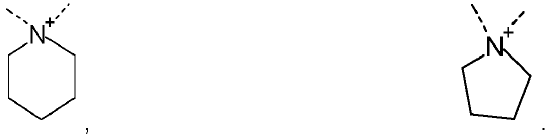

Bevorzugte Verbindungen, die eine wie oben beschriebene ω-Phosphonooxyalkylammoniumgruppe enthalten und für die Funktionalsierung eines entsprechendes Säulenmaterials (Matrixsubstratmaterial) geeignet sind umfassen beispielsweise:
2-[2-(2-Aminoethoxy)ethyl-diethyl-ammonio]ethyl-hydrogenphosphat, 2-[4-[2-(2-Aminoethoxy)ethyl]morpholin-4-ium-4-yl]ethyl-hydrogenphosphat, 2-[1-[2-(2-Aminoethoxy)ethyl]piperidin-1-ium-1-yl]ethyl-hydrogenphosphat, 2-[2-(2-Aminoethoxy)ethyl-dimethyl-ammonio]ethyl-hydrogenphosphat, 2-[3-Aminopropyl-(dimethyl)ammonio]ethyl-hydrogenphosphat, 2-[Dimethyl(4-sulfanylbutyl)ammonio]ethyl-hydrogenphosphat, 2-[4-Azidobutyl(dimethyl)ammonio]ethyl-hydrogenphosphat, 2-[Dimethyl(pent-4-ynyl)ammonio]ethyl-hydrogenphosphat, 2-[3-(6-Aminohexanoyl-amino)propyl-diethyl-ammonio]ethyl-hydrogenphosphat, 2-[1-[2-[2-(6-Aminohexanoyl-amino)ethoxy]ethyl]piperidin-1-ium-1-yl]ethyl-hydrogenphosphat, 2-[4-[2-[2-[3-(6-Aminohexanoylamino)propanoylamino]ethoxy]ethyl]morpholin-4-ium-4-yl]ethyl-hydrogenphosphat, 2-[1-[2-[2-[6-(6-Aminohexanoylamino)hexanoylamino]ethoxy]ethyl]pyrrolidin-1-ium-1-yl]ethyl-hydrogenphosphat, 2-[2-Allyloxyethyl(dimethyl)ammonio]ethyl-hydrogenphosphat, 2-[2-Allyloxyethyl(diethyl)ammonio]ethyl-hydrogenphosphat, 2-[4-(2-Allyloxyethyl)morpholin-4-ium-4-yl]ethyl-hydrogenphosphat, 2-[1-(2-Allyloxyethyl)piperidin-1-ium-1-yl]ethyl-hydrogenphosphat, 2-[2-[2-(6-Aminohexanoylamino)ethoxy]ethyl-dimethyl-ammonio]ethyl-hydrogenphosphat, 2-[2-[2-[3-(6-Aminohexanoylamino)propanoylamino]ethoxy]ethyl-dimethyl-ammonio]ethyl-hydrogenphosphat, 2-[3-Azidopropyl(dimethyl)ammonio]ethyl-hydrogenphosphat, 2-[Dimethyl-[2-[2-(prop-2-ynoxycarbonylamino)ethoxy]ethyl]ammonio]ethyl-hydrogenphosphat, 2-[2-[2-(Allyloxycarbonylamino)ethoxy]ethyl-dimethyl-ammonio]ethyl-hydrogenphosphat, 2-[2-[2-[6-(Allyloxycarbonylamino)hexanoylamino]ethoxy]ethyl-dimethyl-ammonio]ethyl-hydrogenphosphat, 2-[2-(6-Aminohexanoylamino)ethyl-dimethyl-ammonio]ethyl-hydrogenphosphat, 2-[Dimethyl-[3-[6-(prop-2-ynoxycarbonylamino)hexanoylamino]propyl]ammonio]ethyl-hydrogenphosphat, sowie 2-[3-(6-Aminohexanoylamino)propyl-dimethyl-ammonio]ethyl-hydrogenphosphat.

Der Begriff **"ω-Ammoniumalkoxy-hydroxy-phosphoryloxygruppen"** wie hierin verwendet, kann gleichbedeutend mit "omega-Ammoniumalkoxy-hydroxy-phosphoryloxygruppen" verwendet werden und beschreibt Verbindungen der folgenden allgemeinen Formel (**II**) 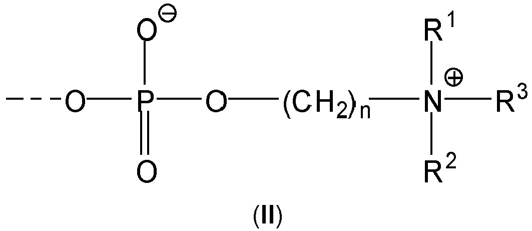 wobei
n ausgewählt wird aus 2 und 3;
R¹, R² und R³ unabhängig voneinander ausgewählt werden aus: -H, -CH₃, -C₂H₅, -C₃H₇, -C₄H₉, -C₅H₁₁, -C₆H₁₃,
oder R¹ und R² zusammen mit dem Stickstoffatom mit dem sie verbunden sind einen Heterozyklus bilden können, der ausgewählt wird aus: 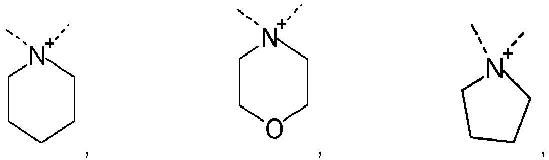 und
   R³ ausgewählt wird aus: -H, -CH₃, -C₂H₅, -C₃H₇, -C₄H₉, -C₅H₁₁, -C₆H₁₃, und vorzugsweise -H;
   wobei ein oder mehrere Wasserstoffatom(e) durch (ein) Fluoratom(e) ersetzt werden können.

Bevorzugte ω-Ammoniumalkoxy-hydroxy-phosphoryloxygruppen umfassen Verbindungen der allgemeinen Formel (**II**) 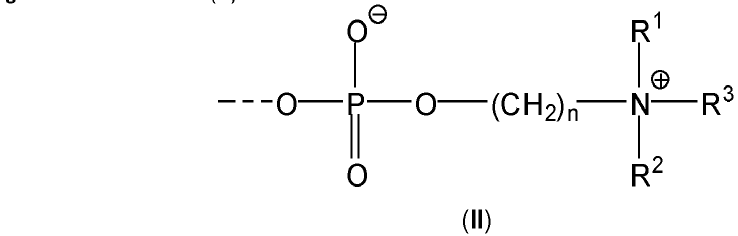 wobei
n ausgewählt wird aus 2 und 3;
R¹, R² und R³ unabhängig voneinander ausgewählt werden aus: -H, -CH₃, -C₂H₅, -C₃H₇,
oder R¹ und R² zusammen mit dem Stickstoffatom mit dem sie verbunden sind einen Heterozyklus bilden können, der ausgewählt wird aus: 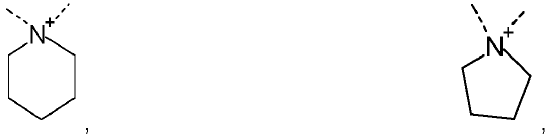 und R³ = -H ist.

Im Rahmen der vorliegenden Erfindung ist besonders bevorzugt, wenn es sich bei der ω-Ammoniumalkoxy-hydroxy-phosphoryloxygruppe um eine ω-Trialkylammoniumalkoxyhydroxy-phosphoryloxygruppe handelt.

Daher umfassen besonders bevorzugte ω-Ammoniumalkoxy-hydroxy-phosphoryloxygruppen Verbindungen der allgemeinen Formel (**II**) 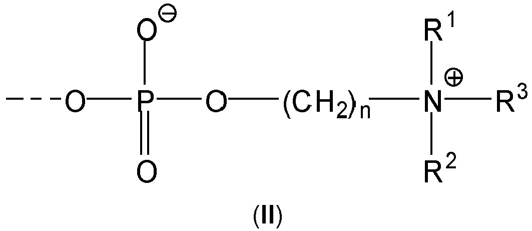 wobei
n = 2 ist;
und R¹, R² und R³ ausgewählt werden aus: -H, -CH₃, -C₂H₅ und besonders bevorzugt aus -CH₃ und -C₂H₅.

Ebenso besonders bevorzugt ist daher, wenn es sich bei den ω-Ammoniumalkoxy-hydroxy-phosphoryloxygruppen um ω-Trimethylammoniumethoxy-hydroxy-phosphoryloxy-gruppen oder um ω-Trimethylammoniupropoxy-hydroxy-phosphoryloxy-gruppen handelt.

Bevorzugte Verbindungen, die eine wie oben beschriebene ω-Ammoniumalkoxy-hydroxy-phosphoryloxygruppe enthalten und für die Funktionalsierung eines entsprechendes Säulenmaterials geeignet sind, umfassen beispielsweise: p-Aminophenylphosphocholin (APPC), 4-[[Hydroxy[2-(trimethylammonio)ethoxy]phosphinyl]oxy]-benzoldiazonium (p-Diazonium-phenylphosphocholin) oder p-Nitrophenyl-6-(O-Phosphocholin)hydroxy-hexanoat.

Eine bevorzugte Ausführungsform betrifft daher die Verwendung einer Alkalihydroxid-Lösung vorzugsweise einer Natriumhydroxid-Lösung zur Regeneration einer Apheresesäule, wobei die Apheresesäule ein Matrixmaterial funktionalisiert mit mindestens einer ω-Ammoniumalkoxy-hydroxy-phosphoryloxygruppe einer Gruppe der folgenden allgemeinen Formel (II) entspricht: 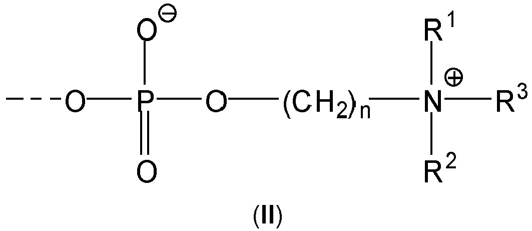 wobei
n ausgewählt wird aus 2 und 3;
R¹, R² und R³ unabhängig voneinander ausgewählt werden aus: -H, -CH₃, - C₂H₅,
-C₃H₇, -C₄H₉, -C₅H₁₁, -C₆H₁₃,
oder R¹ und R² zusammen mit dem Stickstoffatom mit dem sie verbunden sind einen Heterozyklus bilden können, der ausgewählt wird aus: 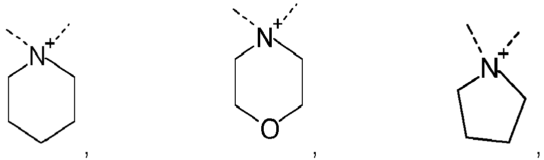 und
   R³ ausgewählt wird aus: -H, -CH₃, -C₂H₅, -C₃H₇, -C₄H₉, -C₅₀H₁₁, -C₆H₁₃, und vorzugsweise -H;
   wobei ein oder mehrere Wasserstoffatom(e) durch (ein) Fluoratom(e) ersetzt werden können.

Ein weiterer Aspekt der vorliegenden Erfindung betrifft ein Verfahren zur Regeneration einer Apheresesäule zur affinitätschromatographischen Entfernung von CRP umfassend den Schritt:
(I) Einleitung einer Alkalihydroxid-Lösung vorzugsweise einer Natriumhydroxid-Lösung in eine Apheresesäule zur Regeneration der Apheresesäule.

Die Alkalihydroxid-Lösung vorzugsweise die Natriumhydroxid-Lösung kann durch eine Neutralisationslösung aus der Apheresesäule entfernt werden, um diese für eine Apheresebehandlung vorzubereiten. Die erfindungsgemäßen Verfahren können daher einen Schritt (II) Einleitung einer Neutralisationslösung umfassen.

Daher betrifft eine Ausführungsform der vorliegenden Erfindung ein Verfahren zur Regeneration einer Apheresesäule zur affinitätschromatographischen Entfernung von CRP umfassend die Schritte:
(I) Einleitung einer Alkalihydroxid-Lösung vorzugsweise einer Natriumhydroxid-Lösung in eine Apheresesäule zur Regeneration der Apheresesäule; und
(II) Einleitung einer Neutralisationslösung.

Weiterhin betrifft eine Ausführungsform der vorliegenden Erfindung ein Verfahren zur Regeneration einer Apheresesäule zur affinitätschromatographischen Entfernung von CRP umfassend die Schritte:
(I) Einleitung einer Alkalihydroxid-Lösung vorzugsweise einer Natriumhydroxid-Lösung in eine Apheresesäule zur Regeneration der Apheresesäule;
(II') Stoppen der Einleitung der Alkalihydroxid-Lösung vorzugsweise der Natriumhydroxid-Lösung nach Schritt (I), und
(II) Einleitung einer Neutralisationslösung.

Die Regeneration der Apheresesäule kann während der Apheresebehandlung erfolgen. In einem solchen Fall kann der Plasmaverlust minimiert werden, indem das Plasma vor der Einleitung der Alkalihydroxid-Lösung vorzugsweise der Natriumhydroxid-Lösung aus Teilen der Apheresevorrichtung insbesondere aus der Apheresesäule entfernt wird, wofür eine Spüllösung verwendet werden kann. Die erfindungsgemäßen Verfahren können somit einen Schritt (I') Spülung der Apheresesäule vorzugsweise enthaltend Blutplasma umfassen.

Eine Ausführungsform der vorliegenden Erfindung betrifft daher ein Verfahren zur Regeneration einer Apheresesäule zur affinitätschromatographischen Entfernung von CRP umfassend die Schritte:
(I') Einleitung einer Spüllösung in eine Apheresesäule vorzugsweise enthaltend Blutplasma;
(I) Einleitung einer Alkalihydroxid-Lösung vorzugsweise einer Natriumhydroxid-Lösung in die Apheresesäule zur Regeneration der Apheresesäule;
(II') Stoppen der Einleitung der Alkalihydroxid-Lösung vorzugsweise der Natriumhydroxid-Lösung nach Schritt (I); und
(II) Einleitung einer Neutralisationslösung.

### Spüllösung

Die Spüllösung kann, muss aber nicht, zur Regeneration der Apheresesäule dienen, sondern hat neben der oben genannten Funktion die Aufgabe, das Blutplasma aus der Apheresesäule zu verdrängen oder zur Neutralisation der Apheresesäulenmatrix beizutragen. Als Spüllösung kann eine Natriumchlorid-Lösung insbesondere eine physiologische Natriumchlorid-Lösung oder eine PBS-Lösung (phosphate buffered saline) eingesetzt werden. Als Spüllösung wird bevorzugt eine NaCI-Lösung und besonders bevorzugt eine physiologische NaCI-Lösung verwendet.

Der Begriff "NaCl-Lösung" (Natriumchlorid-Lösung), wie hierin verwendet, umfasst wässrige Lösungen, die Natriumchlorid (d.h. NaCl, auch als Kochsalz bezeichnet) als Hauptbestandteil enthalten. "Hauptbestandteil" bedeutet hierbei, dass die molare Konzentration an Natriumchlorid in der NaCI-Lösung höher ist als die jeweilige molare Konzentration aller sonstigen Verbindungen innerhalb der NaCl-Lösung, ausgenommen jedoch Wasser. Bevorzugt umfasst die NaCI-Lösung 0,1 bis 5 Gew.% Natriumchlorid, besonders bevorzugt 0,9 Gew.%. Bei der Spüllösung handelt es sich vorzugsweise um eine solche NaCl-Lösung. Unter einer physiologischen NaCl-Lösung (PBS-Lösung) wird eine Natriumchlorid-Lösung umfassend Wasser und 0.9 Gew% Natriumchlorid (NaCl) verstanden.

### Neutralisationslösung

Der Begriff "Neutralisationslösung", wie hierin verwendet, bezeichnet eine wässrige Lösung, die dazu dient, einen pH-Bereich von 6,5 bis 7,6, bevorzugt von 7,30 bis 7,50 und insbesondere bevorzugt von 7,35 bis 7,45 einzustellen.

Als Neutralisationslösung kommen grundsätzlich alle wässrigen Lösungen in Betracht, die im medizinischen Bereich eingesetzt werden dürfen. Die wässrige Lösung besitzt vorzugsweise einen pH ≤ 7, d.h. die wässrige Lösung kann einen neutralen pH-Wert von oder einen pH-Wert <7 besitzen. Bevorzugte Neutralisationslösungen werden ausgewählt aus der Gruppe umfassend oder bestehend aus einer PBS-Lösung oder NaCl-Lösung oder Citrat-Lösung. Besonders bevorzugt handelt es sich bei der Neutralisationslösung um eine Citrat-Lösung. Ein weiterer Aspekt der vorliegenden Erfindung ist somit gerichtet auf die Verwendung einer Citrat-Lösung zur Neutralisation einer Apheresesäule mit einem Säulenmaterial, wobei das Säulenmaterial in einem basischen Medium vorliegt. Die erfindungsgemäßen Verfahren können daher einen Schritt (II) Einleitung einer Neutralisationslösung umfassen, wobei die Neutralisationslösung eine PBS, NaCl- oder Citrat-Lösung, vorzugsweise eine Citrat-Lösung ist.

Weiterhin ist eine Ausführungsform der vorliegenden Erfindung gerichtet auf ein Verfahren zur Regeneration einer Apheresesäule zur affinitätschromatographischen Entfernung von CRP umfassend die Schritte:
(I) Einleitung einer Alkalihydroxid-Lösung vorzugsweise einer Natriumhydroxid-Lösung in die Apheresesäule zur Regeneration der Apheresesäule;
(II') Stoppen der Einleitung der Alkalihydroxid-Lösung vorzugsweise der Natriumhydroxid-Lösung nach Schritt (I); und
(II) Einleitung einer PBS-Lösung oder NaCI-Lösung oder Citrat-Lösung.

Der Begriff "Citrat-Lösung", wie hierin verwendet, umfasst wässrige Lösungen die zumindest eine Citrat-Verbindung enthalten.

Der Begriff **"Citrat",** wie hierin verwendet, bezieht sich auf das Citrat-Anion, also das Salz der Zitronensäure. Bevorzugt ist, wenn die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält.
Eine Citrat-Lösung bestehend aus Zitronensäure, Trinatriumcitrat, D-Glucose und Wasser wird auch als **"Acid-Citrat-Dextrose-Lösung (ACD-Lösung)"** bezeichnet. Bevorzugte Varianten der erfindungsgemäß verwendeten Citrat-Lösung betreffen ACD-Lösungen, welche zwischen 22,9 mM und 38,0 mM Zitronensäure, zwischen 44,9 mM und 74,8 mM Trinatriumcitrat, zwischen 74,2 mM und 123,6 mM D-Glucose und Wasser enthalten. Eine besonders bevorzugte Variante der erfindungsgemäß verwendeten Citrat-Lösung betrifft eine ACD-Lösung, welche 38 mM Zitronensäure, 74,8 mM Trinatriumcitrat, 123,6 mM D-Glucose und Wasser enthält. Diese wird auch als "ACD-A Lösung" bezeichnet.

Eine Citrat-Lösung bestehend aus Zitronensäure, Trinatriumcitrat, Natriumhydrogenphosphat, D-Glucose und Wasser wird auch als **"Citrat-Phosphat-Dextrose-Lösung (CPD)"** bezeichnetEine Citrat-Lösung bestehend aus Zitronensäure, Trinatriumcitrat, Natriumhydrogenphosphat, D-Glucose, Adenin und Wasser wird auch als **"Citrat-Phosphat-Dextrose-Lösung mit Adenin (CPDA)"** bezeichnet.

Bevorzugt ist, dass die Citrat-Lösung eine Konzentration von 2-40%, bevorzugt 4% aufweist und einen pH-Wert im Bereich von 6,4 - 7,5 aufweist. Bevorzugt ist die Verwendung einer Flussrate von 80ml/min. Die Vorteile der Verwendung einer Citrat-Lösung gegenüber einer PBS-Lösung liegen in der verringerten Neutralisationszeit und in dem verringerten benötigten Spülvolumen.

### Vorrichtung

Ein weiterer Aspekt der vorliegenden Erfindung betrifft eine Apheresevorrichtung (1) zur extrakorporalen Entfernung von CRP aus Blut eines Patienten, wobei Apheresevorrichtung (1) derart ausgestaltet ist, dass diese gegenüber einer Alkalihydroxid-Lösung, wie z.B. einer Natriumhydroxid-Lösung beständig ist. Selbstverständlich müssen nur diejenigen Teile der Apheresevorrichtung, welche mit der Alkalihydroxid-Lösung in Berührung kommen, d.h. durch welche die Alkalihydroxid-Lösung aufbewahrt wird oder fließt, beständig gegen die verwendete Alkalihydroxid-Lösung sein. Beispielsweise kommen Zellseparator und Bypass-Leitung nicht mit der Alkalihydroxid-Lösung in Kontakt und müssen daher auch nicht zwingend beständig gegen die verwendete Alkalihydroxid-Lösung sein.

Der Begriff "beständig" wie hierin verwendet meint, dass keine Veränderung der Produkteigenschaften auftritt, z. B. bezüglich der Biokompatibilität, wie auch der Leistung.

Chemische Beständigkeit bezeichnet im Allgemeinen die Widerstandsfähigkeit eines Materials gegen die Einwirkung von Chemikalien. Ein Material ist chemisch beständig, wenn die charakteristischen mechanischen, physikalischen und chemischen Eigenschaften eines Materials auch bei langen Kontaktzeiten mit der zu testenden chemischen Substanz unverändert bleiben oder nur sehr langsam angegriffen werden. Ein Material ist bedingt chemisch beständig, wenn die charakteristischen Eigenschaften des Materials für eine begrenzte, für den Einsatzzweck akzeptable Zeitspanne oder innerhalb spezieller Grenzen der Einsatzbedingungen unverändert bleiben. Chemisch unbeständig sind hingegen Materialien, die ihre charakteristischen Eigenschaften innerhalb sehr kurzer Zeit bzw. schneller als der Einsatzzweck es erlaubt verlieren. Beständig wie hierin verwendet bedeutet daher bevorzugt auch, dass die charakteristischen Eigenschaften der Materialien der Teile der Apheresevorrichtung, welche mit der Alkalihydroxid-Lösung bevorzugt mit Natriumhydroxid-Lösung in Berührung kommen, bei einer Kontaktzeit von mindestens 20 h unverändert bleiben. In der Regel liegt eine für den Einsatzzweck akzeptable Zeitspanne zwischen 4h und 8h.

Daher müssen die mit der Alkalihydroxid-Lösung vorzugsweise der Natriumhydroxid-Lösung in Kontakt kommenden Teile aus einem Alkalihydroxid- vorzugsweise einem Natriumhydroxid-beständigen Material gefertigt sein. Geeignete Materialien schließen ein, sind aber nicht beschränkt auf: Edelstahl, Polypropylen (PP), Polyvinylchlorid (PVC), Polyethylen (PE), Polyethylenterephthalat (PET) und Polycarbonat (PC). Bevorzugt sind Edelstahl, Polypropylen (PP), Polyvinylchlorid (PVC), Polyethylen (PE), Polyethylenterephthalat (PET). Als gegen Alkalihydroxid vorzugsweise Natriumhydroxid beständige Materialien gelten hierin, sind aber nicht beschränkt auf Edelstahl, Polypropylen (PP), Polyvinylchlorid (PVC), Polyethylen (PE), Polyethylenterephthalat (PET) und Polycarbonat (PC). Bevorzugt sind Alkalihydroxid vorzugsweise Natriumhydroxid beständige Materialien ausgewählt aus Edelstahl, Polypropylen (PP), Polyvinylchlorid (PVC), Polyethylen (PE), Polyethylenterephthalat (PET).

Mit anderen Worten ist ein weiterer Aspekt der vorliegenden Erfindung gerichtet auf eine Apheresevorrichtung (1) zur extrakorporalen Entfernung von CRP aus Blut eines Patienten, wobei die Apheresevorrichtung an den Blutkreislauf des Patienten anschließbar ist, und wobei Apheresevorrichtung (1) derart ausgestaltet ist, dass diese gegenüber einer Alkalihydroxid-Lösung vorzugsweise einer Natriumhydroxid-Lösung beständig. D.h. sich die mechanischen, physikalischen und chemischen Eigenschaften der mit der Alkalihydroxid-Lösung in Berührung kommenden Teile der Apheresevorrichtung innerhalb der Betriebszeit der Apheresevorrichtung für eine Apheresebehandlung nicht ändern.

Zur Regeneration der Apheresesäule kann eine Alkalihydroxid-Lösung vorzugsweise eine Natriumhydroxid-Lösung verwendet werden. Alkalihydroxid-Lösungen vorzugsweise eine Natriumhydroxid-Lösung sind für ihre Reaktionsfähigkeit bekannt, die jedoch im erheblichen Maße von der Konzentration des Alkalihydroxids in der Alkalihydroxid-Lösung, vorzugsweise des Natriumhydroxids in der Natriumhydroxid-Lösung abhängen. Zudem beeinflussen weitere Verbindungen in der Alkalihydroxid-Lösung vorzugsweise der Natriumhydroxid-Lösung die Reaktivität des Alkalihydroxids vorzugsweise des Natriumhydroxids (bzw. Natriumhydroxid in dissoziierter Form).

Daher kann eine Alkalihydroxid-Lösung vorzugsweise eine Natriumhydroxid-Lösung Materialien angreifen. Beispielsweise reagieren starke Basen wie Alkalihydroxid-Lösung vorzugsweise Natriumhydroxid mit Glas, wodurch Glasbestandteile in Lösung gehen können. Ebenso existieren Materialien aus organischen Polymeren, die in Anwesenheit von Alkalihydroxid-Lösung vorzugsweise Natriumhydroxid bei Raumtemperatur oder höheren Temperaturen nicht beständig sind.

Eine erfindungsgemäße Ausführung betrifft eine Apheresevorrichtung (1) zur extrakorporalen Entfernung von CRP aus Blut eines Patienten, wobei die Apheresevorrichtung an den Blutkreislauf des Patienten anschließbar ist. Das Blut wird über einen Teil des extrakorporalen Zirkulationssystems (2) der erfindungsgemäßen Apheresevorrichtung (1) zu einem Zellseparator (7) zur Auftrennung des Bluts in Blutplasma und zelluläre Bestandteile gepumpt. Über einen ersten Ausgang des Zellseparators (7) wird das abgetrennte Blutplasma über eine Plasmaleitung (8A) zu einer Apheresesäule (4) zur affinitätschromatographischen Entfernung von CRP aus dem Blutplasma geleitet. Nach der Entfernung des CRPs aus dem Blutplasma des Patienten wird dieses nun behandelte Blutplasma über eine Plasmaleitung (8B) mit den zellulären Bestandteilen des Bluts zusammengeführt. Des Weiteren umfasst die erfindungsgemäße Apheresevorrichtung (1) eine Bypass-Leitung (12), die von der Plasmaleitung (8A) in die Plasmaleitung (8B) führt unter Umgehung der Apheresesäule (4). Ferner umfasst die erfindungsgemäße Apheresevorrichtung (1) mindestens eine Regenerationsleitung (14), die in Flussrichtung nach der Bypass-Leitung (12) in die Plasmaleitung (8A) oder direkt in die Apheresesäule (4) mündet. Die Apheresevorrichtung (1) ist derart ausgestaltet, dass diese gegenüber einer Alkalihydroxid-Lösung vorzugsweise einer Natriumhydroxid-Lösung beständig ist.

D.h. die Teile der Apheresevorrichtung, welche mit der Alkalihydroxid-Lösung in Kontakt kommen, sind beständig gegenüber der verwendeten Alkalihydroxid-Lösung.

Eine Ausführungsform der vorliegenden Erfindung betrifft eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14), die in Flussrichtung an oder nach der Abzweigung der Bypass-Leitung (12) zu der Plasmaleitung (8A) führt oder direkt in die Apheresesäule (4) mündet, wobei die Apheresevorrichtung (1) derart ausgestaltet ist, dass diese gegenüber einer Alkalihydroxid-Lösung vorzugsweise einer Natriumhydroxid-Lösung beständig ist.
Bevorzugt umfasst die Apheresevorrichtung (1) weiterhin eine zentrale Recheneinheit (10) zur Steuerung der Apheresevorrichtung (1).

Eine Ausführungsform der vorliegenden Erfindung betrifft eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F1) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht,
zumindest eine Regenerationsleitung (14), die von dem zumindest einen Flüssigkeitsbehälter (F1) oder der zumindest einen Anschlussleitung (11) abgeht und in Flussrichtung an oder nach der Abzweigung der Bypass-Leitung (12) zu der Plasmaleitung (8A) führt oder direkt in die Apheresesäule (4) mündet, und
zumindest eine zweite Regenerationsleitung (14), die von zumindest einem Flüssigkeitsbehälter (F2) abgeht und wobei die zweite Regenerationsleitung (14) keinen Anschluss an die arterielle Leitung (5) oder den Zellseparator (7) aufweist und in Flussrichtung an oder nach der Abzweigung der Bypass-Leitung (12) zu der Plasmaleitung (8A) führt oder direkt in die Apheresesäule (4) mündet,
wobei die Apheresevorrichtung (1) derart ausgestaltet ist, dass diese gegenüber einer Alkalihydroxid-Lösung vorzugsweise einer Natriumhydroxid-Lösung beständig ist.
Bevorzugt umfasst die Apheresevorrichtung (1) weiterhin eine zentrale Recheneinheit zur Steuerung der Apheresevorrichtung (1).

Bevorzugt umfasst die Apheresevorrichtung (1) daher zumindest zwei Regenerationsleitungen (14', 14"), die unabhängig voneinander in Flussrichtung an oder nach der Abzweigung der Bypass-Leitung (12) zu der Plasmaleitung (8A) führen oder direkt in die Apheresesäule (4') münden, wobei zumindest eine der Regenerationsleitungen (14', 14") von dem zumindest einen Flüssigkeitsbehälter (F1) oder der zumindest einen Anschlussleitung (11) abgeht.

Eine Ausführungsform der vorliegenden Erfindung betrifft eine Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile, mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F1) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F1) oder der zumindest einen Anschlussleitung (11) abgeht und in Flussrichtung an oder nach der Abzweigung der Bypass-Leitung (12) zu der Plasmaleitung (8A) führt oder direkt in die Apheresesäule (4) mündet, wobei die zumindest eine Regenerationsleitung (14) zumindest einen zusätzlichen Anschluss für einen Flüssigkeitsbehälter (F2) aufweist, wobei die Apheresevorrichtung (1) derart ausgestaltet ist, dass diese gegenüber einer Alkalihydroxid-Lösung vorzugsweise einer Natriumhydroxid-Lösung beständig ist.

Bevorzugt umfasst die Apheresevorrichtung (1) weiterhin eine zentrale Recheneinheit zur Steuerung der Apheresevorrichtung (1),

Wie bereits oben ausgeführt, ist unter der Verwendung des Begriffs "beständig" zu verstehen, dass nur die Teile der Apheresevorrichtung, welche mit der Alkalihydroxid-Lösung in Kontakt kommen, gegenüber der verwendeten Alkalihydroxid-Lösung beständig sein müssen bzw. beständig sind. Die restlichen Teile der Apheresevorrichtung können, müssen jedoch nicht gegen die verwendete Alkalihydroxid-Lösung beständig sein.

Beständig bedeutet, dass die Biokompatibilität gemäß der Normenreihe DIN EN ISO 10993-1 bis -12 nicht verändert wird. Die Normenreihe DIN EN ISO 10993-1 bis -12 beinhaltet ISO 10993-1 Beurteilung und Prüfung im Rahmen eines Risikomanagementverfahrens, ISO 10993-2 Tierschutzbestimmungen, ISO 10993-3 Prüfungen auf Gentoxizität, Karzinogenität und Reproduktionstoxizität, ISO 10993-4 Auswahl von Prüfungen zur Wechselwirkung mit Blut, ISO 10993-5 Prüfungen auf In-vitro-Zytotoxizität, ISO 10993-6 Prüfungen auf lokale Effekte nach Implantationen, ISO 10993-7 Ethylenoxid- Sterilisationsrückstände, ISO 10993-8 Auswahl und Eignung von Referenzmaterialien für biologische Prüfungen, ISO 10993-9 Rahmen zur Identifizierung und Quantifizierung von möglichen Abbauprodukten, ISO 10993-10 Prüfungen auf Irritation und Hautsensibilisierung, ISO 10993-11 Prüfungen auf systemische Toxizität, ISO 10993-12 Probenvorbereitung und Referenzmaterialien. Dies kann durch die Überprüfung von Extrakten erfolgen. Zur qualitativen Analyse der gewonnenen Extrakte legt die Norm eine Gaschromatographie (GC) oder eine (Hochleistungs-) Flüssigchromatographie (LC bzw. HPLC) in Kombination mit der Massenspektrometrie (MS) nahe. Zur weiteren Analyse wird eine Identifizierung von typischen extrahierten Verbindungen mittels Ion Pair Chromatography (IPC) bzw. eine Identifizierung extrahierbarer Metallionen mittels Inductively Coupled Plasma (ICP) empfohlen. Zytotoxizitätstests, Prüfungen auf Hämokompatibilität und extrahierbare Bestandteile sind eine Auswahl aus den vielen möglichen Prüfverfahren nach DIN EN ISO 10993. Die Untersuchung der Zellverträglichkeit überprüft, ob ein Produkt einen toxischen/ schädlichen Einfluss auf Zellen hat. Die Untersuchung erfolgt im direkten und/oder indirekten Kontakt und ermöglicht toxische Materialien sicher zu erkennen. Durch Blutverträglichkeitsprüfungen in-vitro werden in einem frühen Stadium unerwünschte Materialeigenschaften erkannt, bevor das Produkt dem Patienten schaden kann. Geprüft wird mit humanem Blut in einfachen statischen oder komplexen dynamischen Systemen. Biomaterialien und Medizinprodukte lösen im Körper unspezifische Fremdkörperreaktionen aus. Bei der Untersuchung der lokalen Gewebereaktion werden Parameter zur Bewertung der Tauglichkeit und Wertigkeit eines Ersatzstoffes bestimmt.

Daher ist es bevorzugt, wenn die hierin beschriebenen erfindungsgemäßen Apheresevorrichtungen derart ausgestaltet sind, dass diese gegenüber einer Alkalihydroxid-Lösung vorzugsweise einer Natriumhydroxid-Lösung beständig sind, wobei eine Beständigkeit gegenüber einer Alkalihydroxid-Lösung vorzugsweise einer Natriumhydroxid-Lösung vorliegt, wenn die Biokompatibilität gemäß der Normenreihe DIN EN ISO 10993-1 bis -12 nicht verändert wird.

Daher ist es bevorzugt, wenn die hierin beschriebenen erfindungsgemäßen Apheresevorrichtungen derart ausgestaltet sind, dass diese gegenüber einer Alkalihydroxid-Lösung vorzugsweise einer Natriumhydroxid-Lösung beständig sind, wobei eine Beständigkeit gegenüber einer Alkalihydroxid-Lösung vorzugsweise einer Natriumhydroxid-Lösung vorliegt, wenn die mit der Alkalihydroxid-Lösung vorzugsweise der Natriumhydroxid-Lösung in Kontakt kommenden Teile aus einem Alkalihydroxid vorzugsweise einem Natriumhydroxid beständigen Material gefertigt sind, wobei die Alkalihydroxid vorzugsweise Natriumhydroxid beständigen Materialien ausgewählt sind aus einer Gruppe umfassend oder bestehend aus Edelstahl, Polypropylen (PP), Polyvinylchlorid (PVC), Polyethylen (PE), Polyethylenterephthalat (PET) und/oder Polycarbonat (PC), bevorzugt Edelstahl, Polypropylen (PP), Polyvinylchlorid (PVC), Polyethylen (PE), und/oder Polyethylenterephthalat (PET).

Vorzugsweise besteht die Plasmaleitung aus Edelstahl oder einem Polymer ausgewählt aus der Gruppe umfassend oder bestehend aus, Polypropylen (PP), Polyvinylchlorid (PVC), Polyethylen (PE), Polyethylenterephthalat (PET) und Polycarbonate (PC). Besonders bevorzugt besteht die Plasmaleitung aus Edelstahl oder einem Polymer ausgewählt aus der Gruppe umfassend oder bestehend aus Polypropylen (PP), Polyvinylchlorid (PVC), Polyethylen (PE), Polyethylenterephthalat (PET).

Bevorzugt besteht der Flüssigkeitsbehälter zur Aufnahme der Regenerationslösung aus Edelstahl oder einem Polymer ausgewählt aus der Gruppe umfassend oder bestehend aus Polypropylen (PP), Polyvinylchlorid (PVC), Polyethylen (PE), Polyethylenterephthalat (PET) und Polycarbonate (PC). Besonders bevorzugt besteht der Flüssigkeitsbehälter zur Aufnahme der Regenerationslösung aus Edelstahl oder einem Polymer ausgewählt aus der Gruppe umfassend oder bestehend aus Polypropylen (PP), Polyvinylchlorid (PVC), Polyethylen (PE), Polyethylenterephthalat (PET).

Weiterhin bevorzugt besteht das Gehäuse der Apheresesäule aus Edelstahl oder einem Polymer ausgewählt aus der Gruppe umfassend oder bestehend aus Polypropylen (PP), Polyvinylchlorid (PVC), Polyethylen (PE), Polyethylenterephthalat (PET) und Polycarbonate (PC). Besonders bevorzugt besteht das Gehäuse der Apheresesäule aus Edelstahl oder einem Polymer ausgewählt aus der Gruppe umfassend oder bestehend aus Edelstahl, Polypropylen (PP), Polyvinylchlorid (PVC), Polyethylen (PE), Polyethylenterephthalat (PET).

Wie bereits oben erläutert, ist die erfindungsgemäße Apheresevorrichtung (1) zur extrakorporalen Entfernung von CRP aus Blut an den Blutkreislauf eines Patienten anschließbar. Von einem Gefäßzugang am Patienten (in der Regel ein venöser Zugang) wird das Blut über einen Teil des extrakorporalen Zirkulationssystems (2) der vorliegenden Erfindung zu einem Zellseparator (7) gepumpt. Der Teil des extrakorporalen Zirkulationssystems (2), der das Blut aus dem Patienten und zu dem Zellseparator (7) leitet, leitet das Blut vom Patienten und damit auch von dessen Herzen weg und wird daher in Anlehnung an die Gefäßnomenklatur im menschlichen Körper als **"arterielle Leitung"** (5) bezeichnet.

Durch einen Eingang des Zellseparators (7) wird das Blut des Patienten in den Zellseparator (7) geleitet und dort von diesem in Blutplasma (zum Teil auch einfach als "Plasma" bezeichnet) und die zellulären Bestandteile des Bluts getrennt. Hierbei muss berücksichtigt werden, dass die Trennung in Blutplasma und zelluläre Bestandteile nicht vollständig erfolgt, sondern lediglich vorzugsweise 10 bis 90 % des gesamten Blutplasmas von den zellulären Bestandteilen getrennt wird. Über einen ersten Ausgang des Zellseparators (7) wird das abgetrennte Blutplasma über eine Plasmaleitung (8A) zu der Apheresesäule (4) zur affinitätschromatographischen Entfernung von CRP aus dem Blut (bzw. aus dem Blutplasma) geleitet. Nach der Entfernung bzw. der Reduzierung des CRPs im Blutplasma des Patienten wird dieses nun behandelte Blutplasma (auch als **"abgereichertes Blutplasma"** bezeichnet) über eine Plasmaleitung (8B) zu dem Punkt (P1) geleitet. Über einen zweiten Ausgang des Zellseparators (7) und eine sich anschließende Leitung (die so genannte Zellleitung (9)) werden die zellulären Bestandteile des Bluts an der Apheresesäule (4) vorbeigeführt und zum Punkt (P1) geleitet. Dort werden die zellulären Bestandteile mit dem abgereicherten Blutplasma zusammengeführt. Nach der Vereinigung der zellulären Bestandteile mit dem abgereicherten Blutplasma wird das nun behandelte Blut über einen weiteren Teil des extrakorporalen Zirkulationssystems (2) der vorliegenden Erfindung zurück zum Patienten geführt. Der Teil des extrakorporalen Zirkulationssystems (2), der das behandelte Blut von dem Punkt (P1) des extrakorporalen Zirkulationssystems (2) zurück zum Patienten leitet, leitet das Blut zum Patienten und damit auch zu dessen Herzen und wird daher in Anlehnung an die Gefäßnomenklatur im menschlichen Körper als **"venöse Leitung"** (6) bezeichnet.

In einer alternativen Ausführungsform der vorliegenden Erfindung ist es möglich, dass die zellulären Bestandteile auch direkt nach der Abtrennung vom Plasma dem Patienten wieder über den zweiten Ausgang am Zellseparator und eine sich anschließende Leitung zugeführt werden und lediglich das behandelte Plasma dem Patienten über die venöse Leitung zugeführt wird.

Um die Gerinnung des Bluts im extrakorporalen Zirkulationssystem verhindern zu können oder um ein Durchspülen bzw. Vorspülen des extrakorporalen Zirkulationssystems (z.B. mit einer physiologischen Salzlösung) zu ermöglichen, umfasst die erfindungsgemäße Apheresevorrichtung zumindest eine Leitung (die so genannte Anschlussleitung (11)), die den Anschluss von zumindest einem Flüssigkeitsbehälter (F) und somit die Einspeisung der in diesem zumindest einen Flüssigkeitsbehälter (F) befindlichen Flüssigkeit (z.B. ein Antikoagulanz oder eine physiologische Salzlösung) in das extrakorporale Zirkulationssystem ermöglicht. In diesem Zusammenhang spricht man auch davon, dass die Anschlussleitung (11) zum Anschluss von zumindest einem Flüssigkeitsbehälter (F) in fluidtechnischer Verbindung mit dem extrakorporalen Zirkulationssystem steht, d.h. eine Flüssigkeit aus einem Flüssigkeitsbehälter über die Anschlussleitung (11) in das extrakorporale Zirkulationssystem eingeleitet werden kann. In bevorzugten Ausführungsformen der vorliegenden Erfindung mündet die zumindest eine Anschlussleitung (11) vor dem Zellseparator (7) in das extrakorporale Zirkulationssystem (2), d.h. in die arterielle Leitung (5), oder direkt in den Zellseparator (7).

Es ist für den Fachmann offensichtlich, dass der oder die Flüssigkeitsbehälter (F) selbst nicht zu der erfindungsgemäßen Apheresevorrichtung gehören müssen, da es sich hierbei in der Regel um Einweg-Artikel, z.B. in Form von gängigen Infusionsbeuteln handelt, die von dem Bedienpersonal (z.B. dem behandelnden Arzt oder einer Krankenschwester) auf die konkrete Anwendung abgestimmt an die Anschlussleitung angeschlossen werden.

Erfindungsgemäß ist das Vorhandensein einer einzelnen Anschlussleitung (11) zum Anschluss eines Flüssigkeitsbehälters möglich. Es ist aber ebenso denkbar, dass eine einzelne Anschlussleitung (11) vorhanden ist, an die zwei oder drei oder bevorzugt mehrere Flüssigkeitsbehälter angeschlossen werden können. Ebenso sind Ausführungsformen der erfindungsgemäßen Apheresevorrichtung mit zwei, bevorzugt drei oder bevorzugt mehreren Anschlussleitungen (11', 11", 11", etc.) zum Anschluss von jeweils zumindest einem Flüssigkeitsbehälter möglich, wobei hierbei bevorzugt ist, dass diese zwei, bevorzugt drei oder bevorzugt mehrere Anschlussleitungen unabhängig voneinander in die arterielle Leitung (5) oder direkt in den Zellseparator (7) münden können. "Unabhängig voneinander" bedeutet in diesem Zusammenhang zum Beispiel, dass bei einer Ausführungsform der erfindungsgemäßen Apheresevorrichtung mit zwei Anschlussleitungen (11', 11") die eine Anschlussleitung (11') in die arterielle Leitung (5) und die andere Anschlussleitung (11") direkt in den Zellseparator (7) münden kann, aber auch dass beide Anschlussleitungen (11', 11") in die arterielle Leitung (5) münden können oder dass beide Anschlussleitungen (11', 11") direkt in den Zellseparator (7) münden können.

Gemäß einer Ausführungsform der vorliegenden Erfindung ist es besonders bevorzugt, wenn die erfindungsgemäße Apheresevorrichtung (1) zwei Anschlussleitungen (11', 11") zum Anschluss von jeweils zumindest einem Flüssigkeitsbehälter aufweist, wobei die Anschlussleitungen (11', 11") unabhängig voneinander in die arterielle Leitung (5) oder direkt in den Zellseparator (7) münden. Folglich münden beide Anschlussleitungen (11', 11") in die arterielle Leitung (5) oder beide Anschlussleitungen (11', 11") münden direkt in den Zellseparator (7) oder besonders bevorzugt mündet die eine Anschlussleitung (11') in die arterielle Leitung (5) und die andere Anschlussleitung (11") direkt in den Zellseparator (7). Hierdurch ist es möglich, dass die zwei Anschlussleitungen (11', 11") an unterschiedliche Flüssigkeitsbehälter angeschlossen werden können. Besonders bevorzugt ist, wenn eine der beiden Anschlussleitungen (z.B. 11') an einen Flüssigkeitsbehälter enthaltend eine physiologische Salzlösung (z.B. NaCl-Lösung) angeschlossen wird, während die zweite der beiden Anschlussleitungen (z.B. 11") an einen Flüssigkeitsbehälter enthaltend z.B. eine Citrat-Lösung angeschlossen wird.

Somit ist besonders bevorzugt, wenn die Apheresevorrichtung (1) eine Anschlussleitung (11') für den Anschluss eines Flüssigkeitsbehälters (F1) und eine Anschlussleitung (11") für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in die Anschlussleitung (11') und damit letztendlich auch in die arterielle Leitung (5) oder in den Zellseparator (7) mündet.

Ein wesentlicher Vorteil der erfindungsgemäßen Apheresevorrichtung besteht darin, dass die in ihrer Reinigungskapazität naturgemäß begrenzte Apheresesäule **im laufenden Betrieb regeneriert** werden kann, d.h. ohne dass die Blutabnahme und -zufuhr oder der Zellseparator gestoppt werden müssen. Hierzu existiert eine Bypass-Leitung (12, auch als "Shunt" bezeichnet), die eine Umleitung des Plasmaflusses unter Umgehung der Apheresesäule (4) ermöglicht. Diese Bypass-Leitung (12) ermöglicht eine temporäre Entkopplung der Apheresesäule (4) von dem Plasmafluss und damit die Regeneration der Apheresesäule (4) ohne dass hierbei der Blut- bzw. Blutplasmafluss in der erfindungsgemäßen Vorrichtung unterbrochen werden muss. Die Bypass-Leitung zweigt von der Plasmaleitung (8A) ab, wobei der Punkt in der Plasmaleitung (8A) von dem die Bypass-Leitung abzweigt als Punkt (P2) bezeichnet wird, und mündet vorzugsweise in die Plasmaleitung (8B), wobei der Punkt in der Plasmaleitung (8B) in den die Bypass-Leitung (12) mündet als Punkt (P6) bezeichnet wird. In einer ebenso möglichen Ausführungsform mündet die Bypass-Leitung (12) nicht in die Plasmaleitung (8B), sondern in die Zellleitung (9), wobei der Punkt in der Zellleitung (9) in den die Bypass-Leitung (12) mündet als Punkt (P3) bezeichnet wird.

Die für die Regeneration der Apheresesäule notwendige Regenerationslösung wird über die Regenerationsleitung (14) in das extrakorporale Zirkulationssystem (2) eingespeist, wobei die Regenerationsleitung (14) entweder direkt in die Apheresesäule (4) mündet oder (in Flussrichtung) vor der Apheresesäule (4) aber (in Flussrichtung) an oder nach der Abzweigung der Bypass-Leitung, also nach dem Punkt (P2) in die Plasmaleitung (8A) mündet.

Damit die Regenerationslösung nach dem Durchfließen der Apheresesäule (4) aus dem System entfernt werden kann (und nicht dem Patienten zugeführt wird), existiert die Abfallleitung (13), die von der Plasmaleitung (8B) abzweigt, wobei der Punkt in der Plasmaleitung (8B) von der die Abfallleitung (13) abzweigt als Punkt (P4) bezeichnet wird. In Ausführungsformen, in denen die Bypass-Leitung (12) in die Zellleitung (9) mündet, liegt der Punkt (P4) vorzugweise in einem Bereich von der Apheresesäule (4) bis zu dem Punkt (P1). In Ausführungsformen, in denen die Bypass-Leitung (12) in die Plasmaleitung (8B) mündet, liegt der Punkt (P4) vorzugweise in einem Bereich von der Apheresesäule (4) bis zu dem Punkt (P6). An besagte Abfallleitung (13) kann natürlich z.B. ein Sammelbehälter angeschlossen werden. Alkalihydroxid-Lösung vorzugsweise Natriumhydroxid-Lösung ist erfindungsgemäß als Regenerationslösung besonders bevorzugt.

Zusätzlich zur Regenerationslösung kann auch noch eine Spüllösung eingesetzt werden. Die Spüllösung kann, muss aber nicht zur Regeneration der Apheresesäule (4) dienen, sondern hat vorrangig die Aufgabe, das Blutplasma aus der Plasmaleitung (8A) in dem Bereich von Punkt P2 bis zur Apheresesäule (4), aus der Apheresesäule (4) sowie aus der Plasmaleitung (8B) von der Apheresesäule (4) bis zum Punkt P4 zu verdrängen, bevor die Regenerationslösung eingesetzt wird, welche nach Durchfluss durch die Apheresesäule (4) dann über die Abfallleitung (13) verworfen wird. Als Spüllösung wird vorzugsweise eine physiologische NaCI-Lösung oder PBS-Lösung verwendet. Noch bevorzugter ist, als Spüllösung eine physiologische NaCI-Lösung zu verwenden, wenn als Regenerationslösung eine Alkalihydroxid-Lösung vorzugsweise eine Natriumhydroxid-Lösung eingesetzt wird.

Bevorzugt umfasst die Apheresevorrichtung (1) zumindest eine Regenerationsleitung (14), die in Flussrichtung an oder nach der Abzweigung der Bypass-Leitung (12) zu der Plasmaleitung (8A) oder direkt in die Apheresesäule (4) führt. Bevorzugt umfasst die Apheresevorrichtung (1) zumindest eine Regenerationsleitung (14) die in einem Bereich von der Abzweigung der Bypass-Leitung (12) an der Plasmaleitung (8A) bis zur Apheresesäule (4) in das extrakorporale Zirkulationssystem (2) mündet. Bevorzugt umfasst die Apheresevorrichtung (1) zumindest eine Regenerationsleitung (14), die in einem Bereich von Punkt (P2) bis zur Apheresesäule (4) in das extrakorporale Zirkulationssystem (2) mündet.

Gemäß einer Ausführungsform der vorliegenden Erfindung ist es bevorzugt, wenn die erfindungsgemäße Apheresevorrichtung (1) zumindest zwei Anschlussleitungen (11) zum Anschluss von jeweils zumindest einem Flüssigkeitsbehälter an die arterielle Leitung (5) oder den Zellseparator (7) aufweist.

Ferner sind Ausführungsformen der Apheresevorrichtung (1) bevorzugt, bei denen die Apheresevorrichtung (1) zumindest zwei Anschlussleitungen (11) zum Anschluss von jeweils zumindest einem Flüssigkeitsbehälter (F) an die arterielle Leitung (5) oder den Zellseparator (7) aufweist, und wobei je Flüssigkeitsbehälter (F) eine Regenerationsleitung (14) existiert, die von dem jeweiligen Flüssigkeitsbehälter (F) oder dessen Anschlussleitung (11) abgehen und die jeweils in die Plasmaleitung (8A) oder direkt in die Apheresesäule (4) führen.

Möglich ist zudem, dass sich die zumindest zwei Anschlussleitungen (11) vor ihrer Einmündung vereinen, d.h. zu einer Leitung zusammenlaufen. Möglich ist auch, dass sich die Regenerationsleitungen (14) vor ihrer Einmündung vereinen, d.h. zu einer Leitung zusammenlaufen.

Wird in der vorliegenden Anmeldung beschrieben, dass ein Vorrichtungsmerkmal in einem Bereich von einer ersten Position in der Vorrichtung bis zu einer zweiten Position in der Vorrichtung liegt oder in diesen Bereich mündet oder von diesem Bereich abzweigt, so ist dies derart zu verstehen, dass sowohl die erste Position als auch die zweite Position und der dazwischen liegende Anschnitt von diesem Bereich eingeschlossen wird. Dies soll an dem folgenden Beispiel verdeutlicht werden: Die Aussage, dass die "Regenerationsleitung (14) in einem Bereich von Punkt (P2) bis zur Apheresesäule (4) in das extrakorporale Zirkulationssystem (2) mündet" bedeutet, dass die Regenerationsleitung (14) in einen Bereich des extrakorporalen Zirkulationssystems (2) mündet, der nicht nur den Abschnitt zwischen Punkt (P2) und der Apheresesäule (4) umfasst, sondern auch den Punkt (P2) selbst auch als die Apheresesäule (4) einschließt. D.h. die Regenerationsleitung (14) kann in Punkt (P2) münden oder in die Apheresesäule (4) oder auch in den Abschnitt der Plasmaleitung (8A), der zwischen dem Punkt (P2) und der Apheresesäule (4) liegt.

Der Punkt (**P1**) ist der Knotenpunkt im extrakorporalen Zirkulationssystem (2), an dem die Plasmaleitung (8B) in die venöse Leitung (6) übergeht. Der Punkt (**P2**) ist der Knotenpunkt im extrakorporalen Zirkulationssystem (2), an dem die Bypass-Leitung (12) von der Plasmaleitung (8A) abzweigt. Der Punkt (**P3**) ist der Knotenpunkt im extrakorporalen Zirkulationssystem (2), an dem die Bypass-Leitung (12) in die Zellleitung (9) mündet. Der Punkt (**P4**) ist der Knotenpunkt im extrakorporalen Zirkulationssystem (2), an dem die Abfallleitung (13) von der Plasmaleitung (8B) abzweigt. Der Punkt (**P5**) ist der Knotenpunkt im extrakorporalen Zirkulationssystem (2), an dem die Regenerationsleitung (14) in die Anschlussleitung (11) mündet. Der Punkt (**P6**) ist der Knotenpunkt im extrakorporalen Zirkulationssystem (2), an dem die Bypass-Leitung (12) in die Plasmaleitung (8B) mündet.

Gemäß einer bevorzugten Ausführungsform der vorliegenden Erfindung mündet die Anschlussleitung (11) in die arterielle Leitung (5). Gemäß einer weiteren bevorzugten Ausführungsform der vorliegenden Erfindung mündet die Anschlussleitung (11) direkt in den Zellseparator (7).

Wie bereits ausgeführt, umfasst die erfindungsgemäße Apheresevorrichtung zumindest eine Leitung (die so genannte Regenerationsleitung (14)), die die Einspeisung einer Regenerationslösung (z.B. eine Alkalihydroxid-Lösung vorzugsweise eine Natriumhydroxid-Lösung) oder einer Spüllösung oder einer Neutralisationslösung in das extrakorporale Zirkulationssystem bevorzugt kurz vor der Apheresesäule (4) oder direkt in die Apheresesäule (4) ermöglicht. In diesem Zusammenhang spricht man auch davon, dass die Regenerationsleitung (14) zum Anschluss von zumindest einem Flüssigkeitsbehälter (F) in fluidtechnischer Verbindung mit dem extrakorporalen Zirkulationssystem steht, d.h. eine Flüssigkeit aus einem Flüssigkeitsbehälter über die Regenerationsleitung in das extrakorporale Zirkulationssystem eingeleitet werden kann.

Gemäß einer bevorzugten Ausführungsform der vorliegenden Erfindung mündet die Regenerationsleitung (14) an oder nach Punkt (P2) in die Plasmaleitung (8A), d.h. zwischen Punkt (P2) und der Apheresesäule (4). Gemäß einer weiteren bevorzugten Ausführungsform der vorliegenden Erfindung mündet die Regenerationsleitung (14) an Punkt (P2) in die Plasmaleitung (8A). Gemäß einer weiteren bevorzugten Ausführungsform der vorliegenden Erfindung mündet die Regenerationsleitung (14) direkt in die Apheresesäule (4).

Es ist für den Fachmann offensichtlich, dass ein Flüssigkeitsbehälter (F) zum Anschluss an die Regenerationsleitung selbst nicht zu der erfindungsgemäßen Apheresevorrichtung gehören muss, da es sich hierbei in der Regel um Einweg-Artikel, z.B. in Form von gängigen Infusionsbeuteln, handelt, die von dem Bedienpersonal (z.B. dem behandelnden Arzt oder einer Krankenschwester) auf die konkrete Anwendung abgestimmt an die Anschlussleitung angeschlossen werden.

Erfindungsgemäß ist das Vorhandensein einer separaten Regenerationsleitung (14) zum Anschluss eines Flüssigkeitsbehälters (F) möglich. Hierbei ist zum Beispiel denkbar, dass an die Regenerationsleitung (14) ein separater Flüssigkeitsbehälter, z.B. ein Infusionsbeutel mit Alkalihydroxid-Lösung vorzugsweise Natriumhydroxid-Lösung angeschlossen werden kann. Es ist aber ebenso denkbar, dass ein Ende einer Regenerationsleitung (14), das den Anschluss eines Flüssigkeitsbehälters ermöglicht, in räumlicher Nähe zu dem Ende einer Anschlussleitung (11), das den Anschluss eines Flüssigkeitsbehälters ermöglicht, liegt, so dass ein Flüssigkeitsbehälter (mit mindestens zwei Anschlussmöglichkeiten bzw. einem entsprechender Adapter) sowohl an die Anschlussleitung (11) als auch an die Regenerationsleitung (14) angeschlossen werden kann z.B. für Infusionsbeutel mit NaCl-Lösung oder Citrat-Lösung.

Erfindungsgemäß ist das Vorhandensein einer einzelnen Regenerationsleitung (14) möglich und besonders bevorzugt sind 1 oder 2 Regenerationsleitungen. Ebenso sind Ausführungsformen der erfindungsgemäßen Apheresevorrichtung mit zwei, drei oder mehreren Regenerationsleitungen (14', 14", 14"', etc.) möglich, wobei hierbei diese zwei, drei oder mehrere Regenerationsleitungen unabhängig voneinander in einem Bereich von der Abzweigung der Bypass-Leitung (12) an der Plasmaleitung (8A) (d.h. vom Punkt P2) bis zur Apheresesäule (4) in das extrakorporale Zirkulationssystem (2) münden können. "Unabhängig voneinander" bedeutet in diesem Zusammenhang zum Beispiel, dass bei einer Ausführungsform der erfindungsgemäßen Apheresevorrichtung mit zwei Regenerationsleitungen (14', 14") die eine Regenerationsleitung (14') in die Plasmaleitung (8A) zwischen Punkt (P2) und der Apheresesäule (4) mündet und die andere Regenerationsleitung (14") direkt in die Apheresesäule (4) mündet, aber auch dass beide Regenerationsleitungen (14', 14") in die Plasmaleitung (8A) zwischen Punkt (P2) und der Apheresesäule (4) münden können. Auch möglich ist, dass eine Regenerationsleitung (14') in die andere Regenerationsleitung (14") mündet. Bei Vorhandensein von zwei oder mehr Regenerationsleitungen (14', 14", 14'", etc.) ist es jedoch besonders bevorzugt, wenn alle Regenerationsleitungen (14', 14", 14'", etc.) an demselben Punkt im Bereich von Punkt (P2) bis zur Apheresesäule (4) in das extrakorporale Zirkulationssystem (2) münden, noch bevorzugter wenn alle Regenerationsleitungen (14', 14", 14'", etc.) an Punkt (P2) in das extrakorporale Zirkulationssystem (2) münden.

Erfindungsgemäß ist es besonders vorteilhaft, wenn eine Anschlussleitung (11) und eine Regenerationsleitung (14) dieselbe Flüssigkeitsquelle nutzen, da so nicht nur Platz gespart wird, sondern auch der Aufwand für Bedienung und Wartung der erfindungsgemäßen Apheresevorrichtung minimiert wird. Auf diese Weise können auch bestehende Apherese-Systeme modifiziert bzw. ergänzt werden, ohne dass ein separates zusätzliches Großgerät angeschlossen werden muss. In bevorzugten Ausführungsformen der vorliegenden Erfindung zweigt daher eine Regenerationsleitung (14) von der Anschlussleitung (11) ab, wobei der Punkt in der Anschlussleitung (11), von dem die Regenerationsleitung (14) abzweigt als Punkt (**P5**) bezeichnet wird.

Es ist daher gemäß einigen Ausführungsformen der vorliegenden Erfindung bevorzugt, dass zumindest eine Regenerationsleitung (14), die in die Plasmaleitung (8A) oder direkt in die Apheresesäule (4) führt, von einem Punkt (P5) in der zumindest einen Anschlussleitung (11) ausgeht.

In Ausführungsformen in denen mehr als eine Anschlussleitung (11', 11", 11'" etc.) vorhanden ist und eine Regenerationsleitung (14) mit mehreren Anschlussleitungen (11', 11", 11'" etc.) verbunden ist, so richtet dich die Nomenklatur der Verzweigungspunkte (P5', P5", P5'" etc.) nach der Nomenklatur der Anschlussleitung (11', 11", 11'" etc.). D.h. beispielhaft, dass bei einer Regenerationsleitung (14), die in zwei vorhandene Anschlussleitungen (11', 11") mündet bzw. eine Verbindung zu diesen herstellt, der Punkt, an dem die Regenerationsleitung (14) in die Anschlussleitung (11') mündet, als Punkt (P5') bezeichnet wird und der Punkt, an dem die Regenerationsleitung (14) in die Anschlussleitung (11") mündet, als Punkt (P5") bezeichnet wird.

Bevorzugt ist eine Apheresevorrichtung (1), wobei die Apheresevorrichtung (1) zwei Anschlussleitungen (11', 11") zum Anschluss von jeweils einem Flüssigkeitsbehälter (F1, F2) an die arterielle Leitung (5) oder den Zellseparator (7) aufweist, und wobei zwei Regenerationsleitungen (14', 14") von den zwei Flüssigkeitsbehältern (F1, F2) oder den zwei Anschlussleitungen (11', 11") abgehen und in die Plasmaleitung (8A) oder direkt in die Apheresesäule (4) führen.

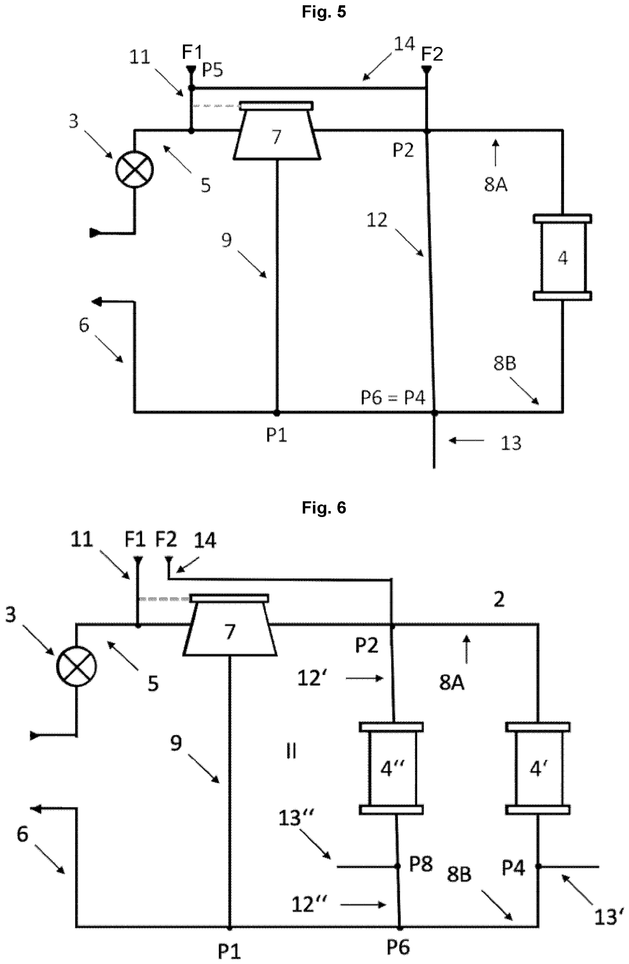

Ebenso sind Ausführungsformen besonders bevorzugt, in denen eine Regenerationsleitung (14), die in die Plasmaleitung (8A) oder direkt in die Apheresesäule (4) führt und die von einem Punkt (P5) in der zumindest einen Anschlussleitung (11) ausgeht, zumindest einen zusätzlichen Anschluss für einen Flüssigkeitsbehälter aufweist (siehe Fig. 5). An diesen zusätzlichen Anschluss kann bevorzugt ein Infusionsbeutel mit Alkalihydroxid-Lösung vorzugsweise Natriumhydroxid-Lösung angeschlossen werden.

In bevorzugten Ausführungsformen umfasst die Apheresevorrichtung (I) weiterhin zumindest eine Regenerationsleitung (14), die von dem zumindest einen Flüssigkeitsbehälter (F1) oder der zumindest einen Anschlussleitung (11) abgeht und in Flussrichtung an oder nach der Abzweigung der Bypass-Leitung (12) zu der Plasmaleitung (8A) führt oder direkt in die Apheresesäule (4) mündet, wobei die zumindest eine Regenerationsleitung (14) zumindest einen zusätzlichen Anschluss für einen Flüssigkeitsbehälter (F2) aufweist. An diesen zusätzlichen Anschluss kann bevorzugt ein Infusionsbeutel mit Alkalihydroxid-Lösung vorzugsweise Natriumhydroxid-Lösung angeschlossen werden.

In bevorzugten Ausführungsformen umfasst die Apheresevorrichtung (I) weiterhin zumindest eine zweite Regenerationsleitung (14), die von zumindest einem Flüssigkeitsbehälter (F2) abgeht und wobei die zweite Regenerationsleitung (14) keinen Anschluss an die arterielle Leitung (5) oder den Zellseparator (7) aufweist und in Flussrichtung an oder nach der Abzweigung der Bypass-Leitung (12) zu der Plasmaleitung (8A) führt oder direkt in die Apheresesäule (4) mündet. An diese zweite Regenerationsleitung (14) kann bevorzugt ein Infusionsbeutel mit Alkalihydroxid-Lösung vorzugsweise mit einer Natriumhydroxid-Lösung angeschlossen werden.

Bei Ausführungsformen mit mehr Anschlussleitungen als Regenerationsleitungen, wobei jede Regenerationsleitung eine Verbindung zu mindestens einer Anschlussleitung herstellt, ist es möglich dass jede Regenerationsleitung mit jeweils einer Anschlussleitung verbunden ist und die überzählige/n Anschlussleitung/en lediglich mit der arteriellen Leitung oder dem Zellseparator verbunden sind, oder dass die zahlreicheren Anschlussleitungen auf die Regenerationsleitungen konvergieren, d.h. mehrere Anschlussleitungen sind mit einer Regenerationsleitung verbunden. Ebenso sind Mischformen möglich.

Es existieren mehrere Möglichkeiten, um die Flussraten in dem Teil der Anschlussleitung (11) nach dem Punkt (P5) und der Regenerationsleitung (14) zu regulieren. Dies könnte z.B. durch separat ansteuerbare Pumpen in dem Teil der Anschlussleitung (11) nach dem Punkt (P5) und in der Regenerationsleitung (14) geschehen. Eine andere Möglichkeit wäre eine Pumpe, die sich in der Anschlussleitung (11) vor dem Punkt (P5) befindet, wobei die Aufteilung der Flussraten nach dem Punkt (P5) entweder durch die Durchmesser von Regenerationsleitung (14) und Anschlussleitung (11) fest determiniert ist oder durch entsprechende Mittel (Klemmen, Ventile) reguliert werden kann (z.B. durch Variation des jeweiligen Leitungsdurchmessers). Die Regulation der Flussraten ist natürlich dann besonders wichtig, wenn eine Lösung (z.B. eine Citrat-Lösung) über die Anschlussleitung (11) in das System eingespeist werden muss (z.B. zur Antikoagulation des Blutes) und gleichzeitig über die Regenerationsleitung (14) in die Apheresesäule gelangen muss (z.B. als Neutralisationslösung). Durch derartige Mechanismen kann z.B. die Einspeisung der Lösung über die Anschlussleitung (11) konstant gehalten werden (z.B. zur gleichbleibenden Antikoagulation), auch wenn phasenweise Lösung zur Neutralisation der Apheresesäule nach Regeneration mit Alkalihydroxid-Lösung vorzugsweise Natriumhydroxid-Lösung über die Regenerationsleitung (14) abgezweigt wird.

Im Vergleich zu anderen Systemen kommt die Apheresevorrichtung (1) mit einer Maximalzahl von 8, vorzugsweise 7, weiter bevorzugt 6 und am meisten bevorzugt 5 Pumpen aus.

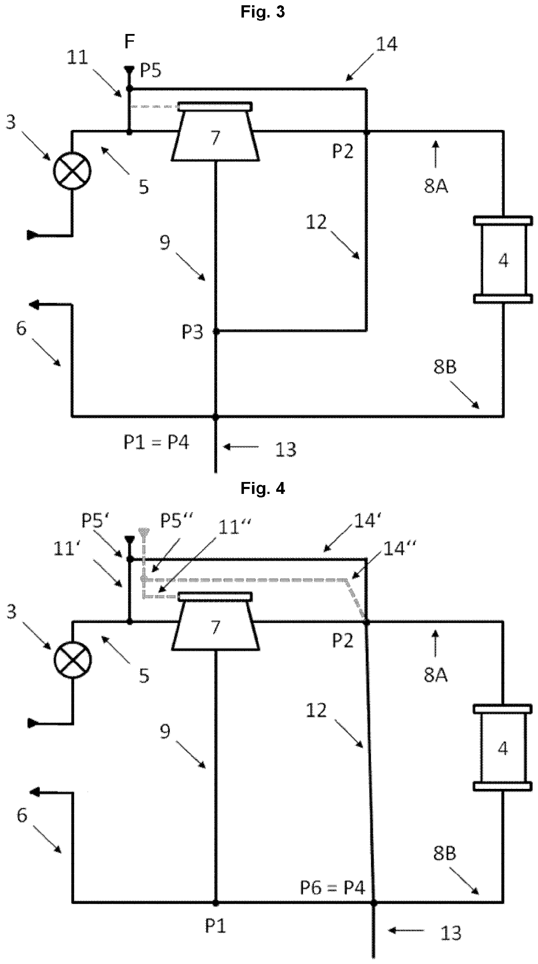

In Ausführungsformen der vorliegenden Erfindung mit mehreren Anschlussleitungen (11', 11", 11", etc.) und mehreren Regenerationsleitungen (14', 14", 14'", etc.) ist es möglich, dass jeweils eine Anschlussleitung mit jeweils einer Regenerationsleitung in Verbindung steht, welche wiederum an oder nach dem Punkt (P2) in die Plasmaleitung (8A) oder direkt in Apheresesäule (4) mündet. Hierbei kann jede Regenerationsleitung unabhängig von anderen Regenerationsleitungen an einer Stelle nach dem Punkt (P2) in die Plasmaleitung (8A) oder direkt in Apheresesäule (4) münden. Bevorzugt ist jedoch, wenn alle Regenerationsleitungen an derselben Stelle an oder nach dem Punkt (P2) in die Plasmaleitung (8A) oder direkt in Apheresesäule (4) münden, noch bevorzugter direkt in die Apheresesäule (4) und am meisten bevorzugt an Punkt (P2). Eine derartige beispielhafte Ausführungsform sei anhand von Fig. 4 erläutert: Hierin besitzt die Apheresevorrichtung (1) eine erste Anschlussleitung (11'), die erstens in die arterielle Leitung (5) führt und von der zweitens am Punkt (P5') eine erste Regenerationsleitung (14') abzweigt. Die Apheresevorrichtung (1) besitzt zudem eine zweite Anschlussleitung (11"), die erstens direkt in den Zellseparator (7) führt und von der zweitens am Punkt (P5") eine zweite Regenerationsleitung (14") abzweigt. Beide Regenerationsleitungen (14', 14") münden in dieser Ausführungsform am Punkt (P2) in das extrakorporale Zirkulationssystem (2).

Bevorzugt ist demnach eine Apheresevorrichtung (1), wobei die Apheresevorrichtung (1) zwei Anschlussleitungen (11', 11") zum Anschluss von jeweils zumindest einem Flüssigkeitsbehälter (F) an die arterielle Leitung (5) oder den Zellseparator (7) aufweist, und wobei zumindest eine Regenerationsleitung (14), die in die Plasmaleitung (8A) oder direkt in die Apheresesäule (4) führt, sich an einem Punkt (P5') mit der Anschlussleitung (11') und an einem Punkt (P5") mit der Anschlussleitung (11") verbindet.

Somit sind insbesondere Ausführungsformen der Apheresevorrichtung (1) bevorzugt, wobei die Apheresevorrichtung (1) zwei Anschlussleitungen (11', 11") zum Anschluss von jeweils zumindest einem Flüssigkeitsbehälter (F1, F2) an die arterielle Leitung (5) oder den Zellseparator (7) aufweist, und wobei zumindest eine Regenerationsleitung (14), die in die Plasmaleitung (8A) oder direkt in die Apheresesäule (4) führt, sich an einem Punkt (P5') mit der Anschlussleitung (11') und an einem Punkt (P5") mit der Anschlussleitung (11") verbindet und wobei eine Regenerationsleitung (14') von dem Flüssigkeitsbehälter (F1) oder von der vom Flüssigkeitsbehälter (F1) abgehenden Anschlussleitung (11') zur Apheresesäule (4) oder zur Plasmaleitung (8A) führt und eine Regenerationsleitung (14") von dem Flüssigkeitsbehälter (F2) oder von der vom Flüssigkeitsbehälter (F2) abgehenden Anschlussleitung (11") zur Apheresesäule (4) oder zur Plasmaleitung (8A) oder in die Regenerationsleitung (14') führt.

Somit ist besonders bevorzugt, wenn die Apheresevorrichtung (1) eine Anschlussleitung (11') für den Anschluss eines Flüssigkeitsbehälters (F1) und eine Anschlussleitung (11") für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in die Anschlussleitung (11') und damit letztendlich auch in die arterielle Leitung (5) oder in den Zellseparator (7) mündet und eine Regenerationsleitung (14') von dem Flüssigkeitsbehälter (F1) oder von der Anschlussleitung (11') zur Apheresesäule (4) oder zur Plasmaleitung (8A) führt und eine Regenerationsleitung (14") von dem Flüssigkeitsbehälter (F2) oder von der Anschlussleitung (11") zur Apheresesäule (4) oder zur Plasmaleitung (8A) oder in die Regenerationsleitung (14') führt.

Ausführungsformen der Apheresevorrichtung (1) sind daher insbesondere bevorzugt, bei denen die Apheresevorrichtung (1) eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7) aufweist, und wobei eine Regenerationsleitung (14') von dem Flüssigkeitsbehälter (F1) oder der Anschlussleitung (11') abgeht und in Flussrichtung nach der Abzweigung der Bypass-Leitung (12) in die Plasmaleitung (8A) oder direkt in die Apheresesäule (4) führt und eine Regenerationsleitung (14") von dem Flüssigkeitsbehälter (F2) oder der Anschlussleitung (11") abgeht und in Flussrichtung nach der Abzweigung der Bypass-Leitung (12) in die Plasmaleitung (8A) oder in die Regenerationsleitung (14') oder direkt in die Apheresesäule (4) führt.

Gemäß einer Ausführungsform ist eine Apheresevorrichtung (1) bevorzugt, wobei die Apheresevorrichtung (1) zwei Anschlussleitungen (11', 11") zum Anschluss von jeweils zumindest einem Flüssigkeitsbehälter an die arterielle Leitung (5) oder den Zellseparator (7) aufweist, und wobei zumindest eine Regenerationsleitung (14), die in die Plasmaleitung (8A) oder direkt in die Apheresesäule (4) führt, eine Verbindung an Punkt (P5') zu der Anschlussleitung (11') und an Punkt (P5") zu der Anschlussleitung (11") herstellt. Dies ist derart zu verstehen, dass eine Regenerationsleitung (14) das Verbindungselement zwischen den Anschlussleitungen (11', 11") auf der einen Seite und der Plasmaleitung (8A) bzw. der Apheresesäule (4) auf der anderen Seite darstellt. Eine Flüssigkeit aus einem der an einer der beiden Anschlussleitungen (11', 11") angeschlossenen Flüssigkeitsbehälter (F) könnte also über die Regenerationsleitung (14) nach Punkt (P2) in die Plasmaleitung (8A) oder direkt in die Apheresesäule (4) fließen.

Ausführungsformen mit zwei (oder sogar mehr) Anschlussleitungen sind ideal dafür geeignet verschiedene Regenerationslösungen zur Regeneration der Apheresesäule (4) zu verwenden und sukzessive in die Apheresesäule (4) einzuleiten. Eine derartige Vorrichtung ist zum Beispiel ideal dafür geeignet, zunächst eine NaCl-Lösung zur Verdrängung des in der Apheresesäule enthaltenen Plasmas einzuleiten, gefolgt von einer Alkalihydroxid-Lösung vorzugsweise einer Natriumhydroxid-Lösung zur effizienten und raschen Regeneration des Adsorbers und abschließend erneut von einer NaCI-Lösung zur Verdrängung der in der Apheresesäule enthaltenen Alkalihydroxid-Lösung vorzugsweise einer Natriumhydroxid-Lösung, bevor dann wieder Plasma in die Apheresesäule eingeleitet wird.

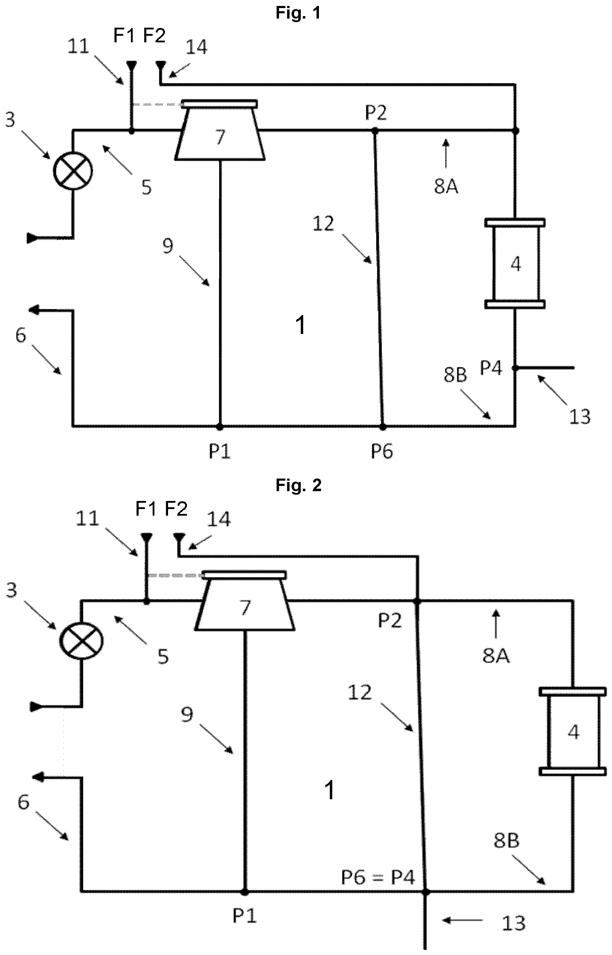

In Ausführungsformen der erfindungsgemäßen Apheresevorrichtung, in denen die Bypass-Leitung (12) zu dem Punkt (P6) in der Plasmaleitung (8B) führt, ist es bevorzugt, wenn der Punkt (P6) (in Flussrichtung) vor dem Punkt (P1) angeordnet ist (siehe **Fig. 1 - 2**).

Gemäß einer bevorzugten Ausführungsform der vorliegenden Erfindung mündet die Anschlussleitung in die arterielle Leitung. Gemäß einer weiteren bevorzugten Ausführungsform der vorliegenden Erfindung mündet die Anschlussleitung direkt in den Zellseparator.

Gemäß einer bevorzugten Ausführungsform der vorliegenden Erfindung mündet die Regenerationsleitung (14) nach Punkt (P2) in die Plasmaleitung (8A), d.h. zwischen Punkt (P2) und der Apheresesäule (4). Gemäß einer weiteren bevorzugten Ausführungsform der vorliegenden Erfindung mündet die Regenerationsleitung (14) direkt in die Apheresesäule (4).

Um das Totvolumen des Systems möglichst gering zu halten, ist es erfindungsgemäß besonders bevorzugt, wenn bei der erfindungsgemäßen Apheresevorrichtung (1) die zumindest eine Regenerationsleitung (14) am Punkt (P2) in das extrakorporale Zirkulationssystem (2) mündet. In Ausführungsformen, in denen mehr als eine Regenerationsleitung (14', 14", 14"', etc.) vorhanden ist, ist es besonders bevorzugt, wenn alle vorhandenen Regenerationsleitungen (14', 14", 14'", etc.) am Punkt (P2) in das extrakorporale Zirkulationssystem (2) münden, bzw. am Punkt (P2) in die Plasmaleitung (8A) münden.

Die vorliegende Erfindung ist daher ebenso gerichtet auf eine erfindungsgemäße Apheresevorrichtung (1), wobei die Bypass-Leitung (12) von einem Punkt (P2) in der Plasmaleitung (8A) zu einem Punkt (P6) in der Plasmaleitung (8B) führt, und die Abfallleitung (13) von einem Punkt (P4) von der Plasmaleitung (8B) abgeht, und die zumindest eine Regenerationsleitung (14) am Punkt (P2) in die Plasmaleitung (8A) mündet.

Um das Totvolumen des Systems noch weiter zu verringern, ist es noch weiter bevorzugt, wenn nicht nur die Regenerationsleitung (14) an dem Punkt (P2) in die Plasmaleitung (8A) mündet, an dem auch die Bypass-Leitung (12) von der Plasmaleitung (8A) abzweigt, sondern wenn auch die Abfallleitung (13) von demselben Punkt in der Plasmaleitung (8B) abzweigt in den auch die Bypass-Leitung (12) mündet. Anders ausgedrückt ist bevorzugt, wenn der Punkt (P6), an dem die Bypass-Leitung (12) in die Plasmaleitung (8B) mündet, und der Punkt (P4), an dem die Abfallleitung (13) von der Plasmaleitung (8B) abzweigt, übereinstimmen, d.h. wenn P4 = P6 ist (siehe hierzu auch **Fig. 2**)

Die vorliegende Erfindung ist daher ebenso gerichtet auf eine erfindungsgemäße Apheresevorrichtung (1), wobei die Bypass-Leitung (12) von einem Punkt (P2) in der Plasmaleitung (8A) zu einem Punkt (P6) in der Plasmaleitung (8B) führt, und die Abfallleitung (13) von einem Punkt (P4) von der Plasmaleitung (8B) abgeht, und die zumindest eine Regenerationsleitung (14) am Punkt (P2) in die Plasmaleitung (8A) mündet und wobei der Punkt (P6) und der Punkt (P4) identisch sind.

In der erfindungsgemäßen Vorrichtung ist ein Zellseparator eingebaut, der das ihm (über die arterielle Leitung) zugeleitete Blut des Patienten in das Blutplasma und die zellulären Bestandteile aufteilt, und diese Fraktionen über die entsprechenden Leitungen, also die Plasmaleitung bzw. die Zellleitung fortleitet. Hierbei muss, wie bereits erwähnt, berücksichtigt werden, dass die Trennung in Blutplasma und zelluläre Bestandteile durch die verwendeten Zellseparatoren nicht vollständig erfolgt, sondern lediglich vorzugsweise 10 bis 90 % des gesamten Blutplasmas von den zellulären Bestandteilen getrennt wird. Bei der Verwendung von zentrifugalen Zellseparatoren wird vorzugsweise 70% bis 90%, weiter bevorzugt 80% bis 87% des gesamten Blutplasmas von den zellulären Bestandteilen getrennt. Bei der Verwendung von Membran-Zellseparatoren wird vorzugsweise 10% bis 30%, weiter bevorzugt 13% bis 25%, noch weiter bevorzugt 15% bis 20% des gesamten Blutplasmas von den zellulären Bestandteilen getrennt.

Mögliche Typen von Zellseparatoren, die im Zusammenhang mit der vorliegenden Erfindung verwendet werden können, umfassen zentrifugale Zellseparatoren, Membran-Zellseparatoren wie z.B. Membran-Zellseparatoren mit semipermeablen Membranen sowie Membran-Zellseparatoren mit rotierenden Membranen.

Die vorliegende Erfindung ist daher ebenso gerichtet auf eine Apheresevorrichtung zur extrakorporalen Entfernung von CRP aus Blut, wobei der Zellseparator (7) entweder ein zentrifugaler Zellseparator oder ein Membran-Zellseparator ist.

Wird in der vorliegenden Anmeldung die Position einer oder mehrerer Komponenten der erfindungsgemäßen Apheresevorrichtung in Relation zu einer anderen Komponente der erfindungsgemäßen Apheresevorrichtung durch die Begriffe **"vor"** oder **"nach"** (bzw. "in Flussrichtung vor" und "in Flussrichtung nach") beschrieben, so nimmt dies Bezug zur generellen Flussrichtung des Bluts bzw. des Blutplasmas in der erfindungsgemäßen Apheresevorrichtung. "Vor" in Bezug zu einer Komponente der erfindungsgemäßen Vorrichtung bedeutet folglich entgegen der allgemeinen Flussrichtung des Bluts bzw. des Blutplasmas, und "nach" in Bezug zu einer Komponente der erfindungsgemäßen Vorrichtung bedeutet folglich mit der allgemeinen Flussrichtung des Bluts bzw. des Blutplasmas. Es ist bevorzugt, dass die Flussrichtung in der Apheresevorrichtung sich nicht umkehrt oder durch das Mittel zur Generierung und Regulation eines Flusses nicht umgekehrt wird.

Gemäß der vorliegenden Erfindung umfasst die erfindungsgemäße Apheresevorrichtung zur extrakorporalen Entfernung von CRP aus Blut eine Apheresesäule (4) zur affinitätschromatographischen Entfernung von CRP aus dem Blut oder Blutplasma, dessen Funktion in der Bindung von CRP besteht, welches sich im Blut bzw. im Blutplasma eines Patienten befindet und das durch die Apheresesäule (4) hindurchgeleitet wird.

Der Begriff **"affinitätschromatographisch"** in Bezug zur Entfernung von CRP, wie in der vorliegenden Anmeldung verwendet, bedeutet, dass die Entfernung von CRP durch eine spezifische Bindung zwischen CRP und Bestandteilen der Apheresesäule (4) zur Entfernung von CRP geschieht. Man kann in diesem Zusammenhang auch von einer "selektiven Entfernung von CRP" oder von einer "selektiven CRP-Apherese" sprechen. Eine derartige spezifische Bindung zwischen CRP und Bestandteilen der Apheresesäule (4) beruhen auf den strukturellen Eigenschaften des CRP-Proteins und umfassen beispielsweise die charakteristische Bindung von CRP an Phosphocholin sowie dessen Derivate oder die Bindung von CRP an Antikörper, die gegen ein Epitop des CRP gerichtet sind. Die selektive oder molekülspezifische Entfernung von CRP beinhaltet, dass CRP mit höherer Affinität an die Matrix in der Apheresesäule (4) bindet als an andere Strukturen/Moleküle. Auch bindet CRP mit höherer Affinität an die Matrix in der Apheresesäule (4) als andere im Blut befindliche Substanzen.

### Pumpen

Gemäß der vorliegenden Erfindung sind in der erfindungsgemäßen Apheresevorrichtung zur extrakorporalen Entfernung von CRP aus Blut Mittel zur Generierung und Regulation eines Flusses des Blutes (oder Blutplasmas) in dem extrakorporalen Zirkulationssystem vorgesehen. Hierzu werden in der Regel eine oder mehrere Pumpen bzw. Pumpsysteme verwendet, die einen regelbaren Fluss des Bluts (bzw. Blutplasmas oder auch der Regenerationslösung oder Antikoagulationslösung) durch das extrakorporale Zirkulationssystem und die fluidtechnisch mit diesem verbundenen Komponenten der erfindungsgemäßen Vorrichtung ermöglichen.

Die erfindungsgemäß bevorzugte Flussrichtung innerhalb des extrakorporalen Zirkulationssystems und der fluidtechnisch mit diesem verbundenen Komponenten der erfindungsgemäßen Vorrichtung verläuft von dem Zugang am Patienten, durch den das Blut in die erfindungsgemäße Vorrichtung gelangt, über die arterielle Leitung des extrakorporalen Zirkulationssystems zu der venösen Leitung des extrakorporalen Zirkulationssystems und dann zu dem Zugang am Patienten, an dem das behandelte Blut wieder in den Patienten zurückgeführt wird.

Bei den erfindungsgemäß verwendeten Mitteln zur Generierung und Regulation eines Flusses in dem extrakorporalen Zirkulationssystem handelt es sich vorzugsweise um Pumpen in Gestalt von Peristaltikpumpen (auch als Schlauchpumpen bezeichnet), Kolbenpumpen, pneumatischen Pumpen, Hydraulikpumpen oder andere dem Fachmann bekannte Pumpentypen. Folglich kann der Begriff "Mittel zur Generierung und Regulation eines Flusses" und der Begriff "Pumpe" hierin gleichbedeutend verwendet werden.

Es ist erfindungsgemäß bevorzugt, wenn die verwendeten Mittel zur Generierung und Regulation eines Flusses von Blut (bzw. Blutplasma oder auch der Regenerationslösung oder Antikoagulationslösung) in dem extrakorporalen Zirkulationssystem keinen direkten physischen Kontakt mit dem Blut (bzw. Blutplasma oder auch der Regenerationslösung oder Antikoagulationslösung) in der erfindungsgemäßen Vorrichtung haben. Dies ist besonders aus hygienischen Gründen vorteilhaft und verhindert die Kontaminierung von komplexen mechanischen Komponenten wie z.B. einer Pumpe durch Blut als natürlich auch des Blutes durch die verwendeten Mittel zur Flussgenerierung.

In einer besonders bevorzugten Ausführungsform der vorliegenden Erfindung handelt es sich bei den Mitteln zur Generierung und Regulation eines Flusses in dem extrakorporalen Zirkulationssystem daher um eine oder mehrere Peristaltikpumpe(n).

Die exakte Position der Mittel zur Generierung und Regulation eines Flusses in dem extrakorporalen Zirkulationssystem, d.h. der einen oder mehreren Pumpe(n), ist nicht wesentlich für die vorliegende Erfindung. Es sind Ausführungsformen der vorliegenden Erfindung unter Verwendung nur einer Pumpe möglich, bei denen sich die Pumpe im Bereich der arteriellen Leitung der erfindungsgemäßen Apheresevorrichtung zur extrakorporalen Entfernung von CRP aus Blut, d.h. vor dem Zellseparator befindet. Sind erfindungsgemäß mehrere Mittel zur Generierung und Regulation eines Flusses in dem extrakorporalen Zirkulationssystem vorgesehen, d.h. mehrere Pumpen, so ist bevorzugt, wenn diese unabhängig voneinander angesteuert und reguliert werden können (z.B. durch die CPU; z.B. durch eine zentrale Recheneinheit). Je nach der konkreten Anwendung können unterschiedliche Flussraten innerhalb des extrakorporalen Zirkulationssystems gewünscht bzw. erforderlich sein. Es ist auch denkbar, dass während einer konkreten Anwendung unterschiedliche Flussraten in verschiedenen Komponenten der erfindungsgemäßen Vorrichtung erwünscht sind.

Erfindungsgemäß können auch mehrere Mittel zur Generierung und Regulation eines Flusses (d.h. Pumpen) in der erfindungsgemäßen Apheresevorrichtung integriert sein. So ist es möglich, dass sich Mittel zur Generierung und Regulation eines Flusses in der arteriellen Leitung (5) und/oder in der Plasmaleitung (8A) und/oder in der Plasmaleitung (8B) und /oder in der venösen Leitung (6) und/oder in der Bypass-Leitung (12) und/oder in der Zellleitung (9) und/oder in der Anschlussleitung (11) bzw. den Anschlussleitungen (11', 11", 11'", etc.) und/oder in der Regenerationsleitung (14) bzw. den Regenerationsleitungen (14', 14", 14'", etc.) befinden. Wie bereits oben angedeutet, ist es gemäß einer Ausführungsform der vorliegenden Erfindung, in der die Regenerationsleitung (14) am Punkt (P5) von der Anschlussleitung (11) abzweigt, bevorzugt, dass ein Mittel zur Generierung und Regulation eines Flusses (von anorganischen Salzlösungen) in der Anschlussleitung (11) vor dem Punkt (P5) angebracht ist.

Gemäß einer weiteren Ausführungsform der vorliegenden Erfindung, in der die Regenerationsleitung (14) am Punkt (P5) von der Anschlussleitung (11) abzweigt, ist es bevorzugt, dass ein Mittel zur Generierung und Regulation eines Flusses in der Anschlussleitung (11) nach dem Punkt (P5) und ein Mittel zur Generierung und Regulation eines Flusses in der Regenerationsleitung (14) angebracht ist.

Des Weiteren weist die Apheresevorrichtung (1) vorzugsweise zumindest einen Partikelfilter auf, der in Flussrichtung hinter der Apheresesäule (4) in der Plasmaleitung (8B) oder der venösen Leitung (6) vorgesehen ist.
Des Weiteren weist die Apheresevorrichtung (1) vorzugsweise zumindest einen Blasenfänger (Bubblecatcher) auf, der in Flussrichtung hinter der Apheresesäule (4) in der Plasmaleitung (8B) oder der venösen Leitung (6) vorgesehen ist.
Im Falle einer Zentrifuge als Zellseparator (7) weist die Apheresevorrichtung (1) vorzugsweise zumindest ein Plasmareservoir auf, das in Flussrichtung nach der Zentrifuge (7) und vor der Apheresesäule (4) in der Plasmaleitung (8A) vorgesehen ist.

In weiteren Ausführungsformen kann die erfindungsgemäße Apheresevorrichtung zur extrakorporalen Entfernung von CRP aus Blut oder Blutplasma eine oder mehrere Drucksensoren umfassen, die dazu dienen, den Druck in einem bestimmten Abschnitt der erfindungsgemäßen Vorrichtung zu messen bzw. zu überwachen. Dies dient nicht nur zur Überwachung und Einstellung der Betriebsparameter der erfindungsgemäßen Apheresevorrichtung, sondern ist auch dahingehend von Vorteil, dass im Falle einer Fehlfunktion (z.B. einer Verstopfung eines Schlauches oder Filters der Vorrichtung) der Betrieb gestoppt werden kann, um für den Patienten schädliche Folgen zu vermeiden. Die genaue Funktionsweise und Einbauposition in der erfindungsgemäßen Vorrichtung gehört zum Stand der Technik und ist dem Fachmann bekannt. In einer bevorzugten Ausführungsform der vorliegenden Erfindung ist sowohl mindestens ein Drucksensor in der arteriellen Leitung der erfindungsgemäßen Apheresevorrichtung als auch mindestens ein Drucksensor in der venösen Leitung der erfindungsgemäßen Apheresevorrichtung, angeordnet. In einer weiteren bevorzugten Ausführungsform der vorliegenden Erfindung sind derartige Drucksensoren in den verwendeten Mitteln zur Generierung und Regulation eines Flusses in dem extrakorporalen Zirkulationssystem der erfindungsgemäßen Apheresevorrichtung integriert.

Bevorzugte Drücke bzw. Druckbereiche für NaOH-Lösung sind -200 bis 200 mbar; Differenzdruck; vor und nach Adsorber: max. 300 mbar.

Um an den Knotenpunkten des extrakorporalen Zirkulationssystems, also an den Punkten an denen mehrere Leitungen zusammenführen bzw. voneinander abgehen, die Flussrichtung im System kontrollieren zu können sind vorzugsweise Mittel vorgesehen, die den Fluss der Lösung (also z.B. des Bluts, des Plasmas oder der Regenerationslösung) bestimmen. Hierbei kann es sich um Ventile, Mehrwegeventile, Klemmen, oder Ventile in Gestalt von Sperrventilen, Rückschlagventilen, Druckventilen, Wegeventilen oder andere dem Fachmann bekannte Ventiltypen handeln, die den Fluss in einer bestimmten Richtung freigeben und in einer anderen Richtung blockieren. Bevorzugt befinden sich derartige Mittel zur Flussregulation (z.B. Ventile) am Punkt (P1) und/oder am Punkt (P2) und/oder am Punkt (P3) und/oder am Punkt (P4) und/oder am Punkt (P5) und/oder am Punkt (P6). Zudem ist es möglich, dass z.B. an einem Punkt zwei oder mehr Ventile in Reihe geschaltet sind, um eine komplexere Flussregulation zu ermöglichen.

Es ist zudem besonders bevorzugt, wenn die Mittel zur Flussregulation (z.B. Ventile) elektronisch steuerbar sind, d.h. deren Stellung durch die zentrale Recheneinheit erfolgen kann.

Die vorliegende Erfindung ist daher ebenso gerichtet auf eine Apheresevorrichtung zur extrakorporalen Entfernung von CRP aus Blut, wobei elektronisch gesteuerte Ventile an den Punkten (P1), (P2), (P4), (P5), (P6), (P7) und (P8) angebracht sind.

Ebenso ist es denkbar und erfindungsgemäß, wenn sich Ventile nicht direkt an den Verzweigungspunkten (P1, P2, P4, P5, P6, P7 und P8) befinden, sondern in den vorgeschalteten und/oder den nachgeschalteten Leitungen befinden, und so den Fluss der Lösungen in dem extrakorporalen Zirkulationsystem steuern. Zu diesem Zweck können auch Schlauchklemmen verwendet werden. Besonders bevorzugt ist, wenn diese Ventile oder Schlauchklemmen elektronisch gesteuert sind.

Ein weiterer Vorteil der vorliegenden Erfindung, der damit zusammenhängt, dass die Apherese und die Regeneration der Apherese-Säule in einer einzelnen Vorrichtung realisiert ist, besteht darin, dass die gesamte Vorrichtung über nur eine einzelne zentrale Recheneinheit (CPU) kontrolliert werden kann. So können die unterschiedlichen Programme während einer Apherese-Sitzung, also zum Beispiel der Normalbetrieb, in dem das Blutplasma durch die Apheresesäule geleitet wird, und der Regenerationsbetrieb, in dem das Blutplasma durch die Bypass-Leitung an der Apheresesäule vorbeigeleitet wird und die Apheresesäule mit Regenerationslösung gespült wird, durch eine einzelne Recheneinheit bzw. eine auf ihr befindliche Software gesteuert werden. Dies erleichtert die Automatisierung vieler Prozesse und verringert somit den Spielraum für Bedienungsfehler seitens des Personals. Bei Geräten aus dem Stand der Technik müssen hingegen unterschiedliche komplexe Systeme (Primärsystem zur Bluttrennung in Plasma und zelluläre Bestandteile; und Sekundärsystem zur Apherese und Regeneration) kombiniert werden, wobei jedes System separat gesteuert werden muss.

Die vorliegende Erfindung ist daher ebenso gerichtet auf eine Apheresevorrichtung zur extrakorporalen Entfernung von CRP aus Blut, wobei die gesamte Vorrichtung nur mittels der einen zentralen Recheneinheit kontrolliert wird.

Ein weiterer Aspekt der vorliegenden Erfindung betrifft eine Apheresevorrichtung (II) zur extrakorporalen Entfernung von CRP aus Blut eines Patienten, wobei die Apheresevorrichtung mindestens zwei Apheresesäulen enthält, und wobei die Apheresevorrichtung (II) derart ausgestaltet ist, dass diese gegenüber einer Alkalihydroxid-Lösung vorzugsweise einer Natriumhydroxid-Lösung beständig ist.

Mit anderen Worten ist ein weiterer Aspekt der vorliegenden Erfindung gerichtet auf eine Apheresevorrichtung (II) zur extrakorporalen Entfernung von CRP aus Blut eines Patienten, wobei die Apheresevorrichtung mindestens zwei Apheresesäulen enthält, wobei die Apheresevorrichtung an den Blutkreislauf des Patienten anschließbar ist, und wobei Apheresevorrichtung (II) derart ausgestaltet ist, dass diese gegenüber einer Alkalihydroxid-Lösung vorzugsweise einer Natriumhydroxid-Lösung beständig ist.

Ein weiterer Aspekt der vorliegenden Erfindung betrifft eine Apheresevorrichtung, wobei mindestens eine weitere Apheressäule (4") bevorzugt eine zweite Apheresesäule (4") mit der Bypass-Leitung verbunden ist oder die Bypassleitung mindestens eine weitere Apheresesäule (4") bevorzugt eine zweite Apheresesäule umfasst. Bevorzugt ist die mindestens eine weitere Apheresesäule (4") bevorzugt die zweite Apheresesäule (4") in der Bypass-Leitung enthalten. Daher können die hierin beschriebenen erfindungsgemäßen Apheresevorrichtungen zur extrakorporalen Entfernung von CRP aus Blut mindestens eine weitere Apheresesäule (4") vorzugsweise eine zweite Apheresesäule (4") enthalten, wobei die mindestens eine weitere Apheresesäule vorzugsweise zweite Aphersesäule (4") in der Bypassleitung enthalten ist. Eine Apheresesäule (4") ist in der Bypass-Leitung enthalten, wenn ein Abschnitt der Bypass-Leitung (12') der Bypass-Leitung (12) in die zweite Apheresesäule (4") mündet und ein weiterer Abschnitt der Bypass-Leitung (12") der Bypass-Leitung (12) am Ausgang der Apheresesäule (4") von dieser wegführt.

Daher ist ein weiterer Aspekt der der vorliegenden Erfindung eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nur abwechselnd betreibbar sind, d.h. nicht zeitgleich zur CRP-Entfernung nutzbar sind, wobei die Apheresevorrichtung (II) derart ausgestaltet ist, dass diese gegenüber einer Alkalihydroxid-Lösung vorzugsweise einer Natriumhydroxid-Lösung beständig ist.
Bevorzugt umfasst die Apheresevorrichtung (II) weiter eine zentrale Recheneinheit zur Steuerung der Apheresevorrichtung (II).

Daher ist ein weiterer Aspekt der der vorliegenden Erfindung eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F1) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F1) oder der zumindest einen Anschlussleitung (11) abgeht und in Flussrichtung an oder nach der Abzweigung der Bypass-Leitung (12) zu der Plasmaleitung (8A) führt oder direkt in die Apheresesäule (4') mündet, und
zumindest eine zweite Regenerationsleitung (14), die von zumindest einem Flüssigkeitsbehälter (F2) abgeht und wobei die zweite Regenerationsleitung (14) keinen Anschluss an die arterielle Leitung (5) oder den Zellseparator (7) aufweist und in Flussrichtung an oder nach der Abzweigung der Bypass-Leitung (12) zu der Plasmaleitung (8A) führt oder direkt in die Apheresesäule (4') mündet,
wobei eine zweite Apheresesäule (4") parallel zur ersten Apheresesäule (4') geschaltet ist und beide Apheresesäulen (4', 4") nur abwechselnd betreibbar sind, d.h. nicht zeitgleich zur CRP-Entfernung nutzbar sind, wobei die Apheresevorrichtung (II) derart ausgestaltet ist, dass diese gegenüber einer Alkalihydroxid-Lösung vorzugsweise einer Natriumhydroxid-Lösung beständig ist.
Bevorzugt umfasst die Apheresevorrichtung (II) weiter eine zentrale Recheneinheit zur Steuerung der Apheresevorrichtung (II).

Bevorzugt umfasst die Apheresevorrichtung (II) daher zumindest zwei Regenerationsleitungen (14', 14"), die unabhängig voneinander in Flussrichtung an oder nach der Abzweigung der Bypass-Leitung (12) zu der Plasmaleitung (8A) führen oder direkt in die Apheresesäule (4') münden, wobei zumindest eine der Regenerationsleitungen (14', 14") von dem zumindest einen Flüssigkeitsbehälter (F1) oder der zumindest einen Anschlussleitung (11) abgeht.

Daher ist ein weiterer Aspekt der der vorliegenden Erfindung eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F1) an die arterielle Leitung (5) oder den Zellseparator (7),
dadurch gekennzeichnet, dass
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die von dem zumindest einen Flüssigkeitsbehälter (F1) oder der zumindest einen Anschlussleitung (11) abgeht und in Flussrichtung an oder nach der Abzweigung der Bypass-Leitung (12) zu der Plasmaleitung (8A) führt oder direkt in die Apheresesäule (4') mündet, wobei die zumindest eine Regenerationsleitung (14) zumindest einen zusätzlichen Anschluss für einen Flüssigkeitsbehälter (F2)aufweist,
wobei eine zweite Apheresesäule (4") parallel zur ersten Apheresesäule (4') geschaltet ist und beide Apheresesäulen (4', 4") nur abwechselnd betreibbar sind, d.h. nicht zeitgleich zur CRP-Entfernung nutzbar sind, wobei die Apheresevorrichtung (II) derart ausgestaltet ist, dass diese gegenüber einer Alkalihydroxid-Lösung vorzugsweise einer Natriumhydroxid-Lösung beständig ist.
Bevorzugt umfasst die Apheresevorrichtung (II) weiter eine zentrale Recheneinheit (10) zur Steuerung der Apheresevorrichtung (II).

Die oben genannten Ausgestaltungen der erfindungsgemäßen Apheresevorrichtung (1) sind auf die erfindungsgemäße Apheresevorrichtung (II) zu übertragen. Die Bypass-Leitung (12) der Apheresevorrichtung (1) wird in der Apheresevorrichtung (II) als Plasmaleitung verwendet.

Mithilfe dieser erfindungsgemäßen Apheresevorrichtung (II) ist es möglich CRP bei gleicher Behandlungsdauer effizienter aus Blut zu entfernen als mit Vorrichtungen aus dem Stand der Technik. Durch die Verwendung von zwei parallel geschalteten Apheresesäulen, die nur abwechselnd zur CRP-Entfernung einsetzbar sind, kann mittels der erfindungsgemäßen Apheresevorrichtung eine Apheresesäule für die Entfernung von CRP aus dem Blut genutzt werden, während die zweite Apheresesäule entweder durch eine andere Apherese ausgetauscht oder die zweite Apheresesäule während der laufenden Apheresesitzung regeneriert werden kann. Dadurch kann auch ein hoher Klinikdurchsatz mittels einer Apheresevorrichtung erzielt werden. Weiterhin ist die Verwendung der erfindungsgemäßen Apheresevorrichtung nicht durch das Totvolumen limitiert. Üblicherweise werden überdimensionierte Apheresesäulen aber auch in Reihe geschaltete Apheresesäulen durch ihr großes Totvolumen in ihrer Anwendung für die Apherese stark beschränkt. Zudem wird das Volumen einer Apheresevorrichtung und damit das Volumen oder die Anzahl an in Reihe geschalteten Apheresesäulen durch die Blutflussrate des Menschen vorgegeben. Auch Apheresevorrichtungen mit parallel geschalteten Apheresesäulen, die zeitgleich verwendet werden, können aufgrund des großen Totvolumens nicht effizient und ohne Risiko für den Patienten für die Entfernung von CRP aus Blut genutzt werden.

Eine hierin beschriebene erfindungsgemäße Apheresevorrichtung (II) zeichnet sich dadurch aus, dass eine zweite Apheresesäule zu einer ersten Apheresesäule (4') parallel geschaltet ist. "Parallel" bedeutet in diesem Zusammenhang, dass mehrere Kreisläufe innerhalb des extrakorporalen Zirkulationssystems (2) nebeneinander vorhanden sind, d.h., dass z.B. eine erste Apheresesäule (4') mit der Plasmaleitung (8A) für das abgetrennte Plasma und mit der Plasmaleitung (8B) für das CRP-abgereicherte Plasma ein erstes Kreislaufsystem des extrakorporalen Zirkulationssystems (2) darstellt und eine zweite Apheresesäule (4") mit dem Bypass-Leitungsabschnitt (12') der Bypass-Leitung (12) und dem Bypass-Leitungsabschnitt (12") der Bypass-Leitung (12) ein zweites Kreislaufsystem des extrakorporalen Zirkulationssystems (2) darstellt. "Parallel" bedeutet auch, dass die zwei Apheresesäulen nicht in Reihe geschaltet sind, d.h. nicht hintereinander, dass der Ausfluss der ersten Apheresesäule in die zweite Apheresesäule eingeleitet wird. Durch die parallele Anordnung der Apheresesäulen addieren sich deren Kapazitäten auch nicht.
Hiervon zu unterscheiden ist die serielle Schaltung der Apheresesäulen, die nicht erfindungsgemäß ist. "Seriell" bedeutet, dass mehrere Apheresesäulen sich nur in einem Kreislauf des extrakorporalen Zirkulationssystems (2) befinden, d.h., dass z.B. die erste Apheresesäule (4') und die zweite Apheresesäule (4") zusammen mit der Plasmaleitung (8A) und der Plasmaleitung (8B) nur einen Kreislauf des extrakorporalen Zirkulationssystems (2) bilden, d.h. in Reihe geschaltet oder angeordnet wären.

Erfindungsgemäß können die beiden zueinander parallel geschalteten oder parallel angeordneten Apheresesäulen (4', 4") nur abwechselnd betrieben werden. "Abwechselnd" bedeutet, dass das abgetrennte Blutplasma entweder durch die Apheresesäule (4') oder durch die Apheresesäule (4") aber nicht zeitgleich durch beide Apheresesäulen (4', 4") geleitet wird. "Abwechselnd" betrieben meint dabei die therapeutische CRP-Entfernung. Beide Apheresesäulen (4' und 4") sind nicht zeitgleich zur CRP-Entfernung nutzbar. Natürlich kann eine der beiden Apheresesäulen regeneriert werden, während die andere zeitgleich zur CRP-Entfernung eingesetzt wird. Ausgeschlossen ist nur der zeitgleiche therapeutische Betrieb zur CRP-Entfernung beider Apheresesäulen.

Folgende Zustände sind daher möglich. Durch eine Apheresesäule wird das Blutplasma geleitet, um das CRP zu entfernen. Zeitgleich ist die zweite Apheresesäule gebrauchsfertig und es kann der Plasmafluss auf diese zweite Apheresesäule umgeleitet werden sobald die Kapazität der ersten Apheresesäule erschöpft ist oder andere Probleme mit der ersten Apheresesäule auftreten sollten oder die zweite Apheresesäule wurde bereits zur CRP-Entfernung genutzt und muss ausgetauscht oder regeneriert werden oder die zweite Apheresesäule wird regeneriert während die erste CRP entfernt.

In Ausführungsformen der vorliegenden Erfindung ist die Apheresevorrichtung (II) mit zwei Apheresesäulen daher derart ausgestaltet, dass die Apheresesäulen nur abwechselnd betreibbar sind.

Somit ist gemäß einer Ausführungsform der erfindungsgemäßen Apheresevorrichtung (II) das Blutplasma zeitgleich entweder nur durch die erste Apheresesäule (4') oder nur durch die zweite Apheresesäule (4") leitbar. In weiteren Ausführungsformen der erfindungsgemäßen Vorrichtung ist somit die Apheresevorrichtung derart ausgestaltet, dass das Blutplasma zeitgleich entweder nur durch die erste Apheresesäule (4') oder nur durch die zweite Apheresesäule (4") leitbar ist.

Während des abwechselnden Betriebs der beiden Apheresesäulen (4', 4") wird entweder durch die Apheresesäule (4') oder durch die Apheresesäule (4") kein Blutplasma geleitet. Dadurch ergibt sich die Möglichkeit, eine der beiden Apheresesäule aus der Apheresevorrichtung während des Betriebs der Apheresevorrichtung auszutauschen. "Austauschen" bedeutet hierbei, dass eine der beiden Apheresesäulen durch eine neue Apheresesäule ersetzt oder eine der beiden Apheresesäulen regeneriert wird. Die Regeneration einer der beiden Apheresesäulen kann z.B. durch Spülen mit einer Alkalihydroxid-Lösung vorzugsweise einer Natriumhydroxid-Lösung erfolgen. Die Verwendung einer Alkalihydroxid-Lösung vorzugsweise einer Natriumhydroxid-Lösung ist für die Regeneration der Apheresesäulen bevorzugt. "Während des Betriebs" bedeutet in diesem Zusammenhang, dass die Entfernung des CRP aus dem Blut weiterhin abläuft.

Eine Ausführungsform der hierin beschriebenen erfindungsgemäßen Apheresevorrichtungen (II) betrifft daher eine Apheresevorrichtung, in dem eine erste Apheresesäule (4') während des Betriebs einer zweiten Apheresesäule (4") austauschbar ist und die zweite Apheresesäule (4") während des Betriebs der ersten Apheresesäule (4') austauschbar ist.

Ebenso sind Ausführungsformen denkbar, wobei eine erste Apheresesäule (4') während des Betriebs einer zweiten Apheresesäule (4") regenerierbar ist und die zweite Apheresesäule (4") während des Betriebs der ersten Apheresesäule (4') regenerierbar ist.

Somit ist in einer Ausführungsform der vorliegenden Erfindung die Apheresevorrichtung (II) derart ausgestaltet, dass eine erste Apheresesäule (4') während des Betriebs einer zweiten Apheresesäule (4") austauschbar oder regenerierbar ist und die zweite Apheresesäule (4") während des Betriebs der ersten Apheresesäule (4') austauschbar oder regenerierbar ist.

Bevorzugt ist auch eine Ausführungsform der Apheresevorrichtung (II) zur extrakorporalen Entfernung von CRP aus Blut,
wobei eine zweite Apheresesäule (4") parallel zur ersten Apheresesäule (4') geschaltet ist und beide Apheresesäulen (4', 4") zeitgleich nur abwechselnd betreibbar sind, und wobei die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist, wobei die Apheresevorrichtung (II) derart ausgestaltet ist, dass diese gegenüber einer Alkalihydroxid-Lösung vorzugsweise einer Natriumhydroxid-Lösung beständig ist.

Bevorzugt ist auch eine Ausführungsform der Apheresevorrichtung (II) zur extrakorporalen Entfernung von CRP aus Blut, wobei eine zweite Apheresesäule (4") parallel zur ersten Apheresesäule (4') geschaltet ist und beide Apheresesäulen (4', 4") nicht zeitgleich zur CRP-Entfernung nutzbar sind, und wobei eine der Apheresesäulen (4', 4") zeitgleich zur CRP-Entfernung durch die andere Apheresesäule regenerierbar ist, wobei die Apheresevorrichtung (II) derart ausgestaltet ist, dass diese gegenüber einer Alkalihydroxid-Lösung vorzugsweise einer Natriumhydroxid-Lösung beständig ist.

Daher ist gemäß einer Ausführungsform der vorliegenden Erfindung die Apheresevorrichtung (II) derart ausgestaltet, dass die erste Apheresesäule (4') während des Betriebs der zweiten Apheresesäule (4") austauschbar und die Apheresesäule derart ausgestaltet ist, dass sie regenerierbar ist und die zweite Apheresesäule (4") während des Betriebs der ersten Apheresesäule (4') austauschbar ist und die Apheresesäule (4") derart ausgestaltet ist, dass die Apheresesäule (4") regenerierbar ist.

Die zur ersten Apheresesäule (4') parallel geschaltete zweite Apheresesäule (4") kann in die Bypass-Leitung eingebunden sein, d.h. die Bypass-Leitung (12) setzt sich zusammen aus einen Bypass-Leitungs-Abschnitt (12') und einen Bypass-Leitungs-Abschnitt (12"), wobei zwischen den besagten Bypass-Leitungsabschnitten die zweite Apheresesäule (4") befindet. Bevorzugt ist daher eine Apheresevorrichtung (II) die dadurch gekennzeichnet ist, dass ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet.
Bevorzugt ist auch eine Ausführungsform der Apheresevorrichtung (II) zur extrakorporalen Entfernung von CRP aus Blut,
wobei eine zweite Apheresesäule (4") parallel zur ersten Apheresesäule (4') geschaltet ist und beide Apheresesäulen (4', 4") nicht zeitgleich zur CRP-Entfernung nutzbar sind (d.h. nur abwechselnd betreibbar sind), wobei die Apheresevorrichtung (1) derart ausgestaltet ist, dass diese gegenüber einer Alkalihydroxid-Lösung vorzugsweise einer Natriumhydroxid-Lösung beständig ist.

Gemäß einer Ausführungsform der vorliegenden Erfindung ist es besonders bevorzugt, wenn die erfindungsgemäße Apheresevorrichtung (II) zwei Anschlussleitungen (11',11") zum Anschluss von jeweils zumindest einen Flüssigkeitsbehälter (F) aufweist, wobei die Anschlussleitungen (11', 11") unabhängig voneinander in die arterielle Leitung (5) oder direkt in den Zellseparator (7) münden. Folglich münden beide Anschlussleitungen (11', 11") in die arterielle Leitung (5) oder beide Anschlussleitungen (11', 11") münden direkt in den Zellseparator (7) oder besonders bevorzugt mündet eine Anschlussleitung (11') in die arterielle Leitung (5) und die andere Anschlussleitung (11") direkt in den Zellseparator (7). Hierdurch wird ermöglich, dass die zwei Anschlussleitungen (11', 11") an unterschiedliche Flüssigkeitsbehälter angeschlossen werden können.

Gemäß einer weiteren Ausführungsform der vorliegenden Erfindung enthält die Apheresevorrichtung (II) eine Abfallleitung (13'), die direkt von Apheresesäule (4') abgeht oder von der Plasmaleitung (8B) in Flussrichtung vor der Einmündung des Bypass-Leitungsabschnitts (12") der Bypass-Leitung in die Plasmaleitung (8B) abgeht und eine Abfallleitung (13"), die direkt von der Apheresesäule (4") abgeht oder von dem Bypass-Leitungsabschnitt (12") in Flussrichtung vor der Einmündung in die Plasmaleitung (8B).

Die vorliegende Erfindung betrifft somit auch eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13') direkt von der Apheresesäule (4') abgeht oder von der Plasmaleitung (8B) in Flussrichtung vor der Einmündung des Bypass-Leitungsabschnitts (12") der Bypass-Leitung (12) abgeht, eine Abfallleitung (13"), die direkt von Apheresesäule (4") abgeht oder von der Plasmaleitung (8B) in Flussrichtung vor der Einmündung des Bypass-Leitungsabschnitts (12") der Bypass-Leitung in die Plasmaleitung (8B) abgeht und eine Abfallleitung (13"), die direkt von der Apheresesäule (4") abgeht oder von dem Bypass-Leitungsabschnitt (12") in Flussrichtung vor der Einmündung in die Plasmaleitung (8B),
   und
zumindest eine Regenerationsleitung (14), die in Flussrichtung nach der Abzweigung des Bypass-Leitungsabschnitts (12') der Bypass-Leitung (12) zu der Plasmaleitung (8A) oder zu dem Bypass-Leitungsabschnitt (12') führt oder direkt in die Apheresesäule (4') oder Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd betreibbar sind, wobei die Apheresevorrichtung (II) derart ausgestaltet ist, dass diese gegenüber einer Alkalihydroxid-Lösung vorzugsweise einer Natriumhydroxid-Lösung beständig ist.

Gemäß einer weiteren Ausführungsform der vorliegenden Erfindung enthält die Apheresevorrichtung (II) weiterhin zumindest eine Regenerationsleitung (14), die von dem zumindest einen Flüssigkeitsbehälter (F) oder der zumindest einen Anschlussleitung (11) abgeht und in die Plasmaleitung (8A) oder in den Bypass-Leitungsabschnitt (12' der Bypass-Leitung (12) führt oder direkt in die Apheresesäule (4') oder direkt in die Apheresesäule (4") mündet. Gemäß einer weiteren Ausführungsform der vorliegenden Erfindung enthält die Apheresevorrichtung (II) weiterhin zumindest eine Regenerationsleitung (14), die in einem Bereich von dem Punkt (P2) und der Apheresesäule (4') in den Bypass-Leitungsabschnitt (12') oder in einem Bereich von dem Punkt (P2) und der Apheresesäule (4") in die Plasmaleitung (8A) mündet oder direkt in die Apheresesäule (4') oder direkt in die Apheresesäule (4") mündet.

In einer besonders bevorzugten Ausführungsform der vorliegenden Erfindung umfasst die Apheresevorrichtung (II) eine Abfallleitung (13'), die direkt von der Apheresesäule (4') abgeht oder von der Plasmaleitung (8B) in Flussrichtung vor der Einmündung des Bypass-Leitungsabschnitts (12") der Bypass-Leitung (12) abgeht und zumindest eine Regenerationsleitung (14), die von dem zumindest einen Flüssigkeitsbehälter (F) oder der zumindest einen Anschlussleitung (11) abgeht und in den Bypass-Leitungsabschnitt (12') oder in die Plasmaleitung (8A) führt oder direkt in die Apheresesäule (4') oder direkt in die Apheresesäule (4") mündet.

In einer insbesondere bevorzugte Ausführungsform der vorliegenden Erfindung umfasst die Apheresevorrichtung (II) eine Abfallleitung (13'), die direkt von der Apheresesäule (4') abgeht oder von der Plasmaleitung (8B) in Flussrichtung vor der Einmündung des Bypass-Leitungsabschnitts (12") der Bypass-Leitung abgeht, eine Abfallleitung (13"), die direkt von der Apheresesäule (4") abgeht oder von dem Bypass-Leitungsabschnitt (12") der Bypass-Leitung (12) in Flussrichtung vor der Einmündung des Bypass-Leitungsabschnitts (12') der Bypass-Leitung abgeht und zumindest eine Regenerationsleitung (14), die von dem zumindest einen Flüssigkeitsbehälter (F) oder der zumindest einen Anschlussleitung (11) abgeht und in die Plasmaleitung (8A) oder in den Bypass-Leitungsabschnitt (12') der Bypass-Leitung (12) führt oder direkt in die Apheresesäule (4') oder direkt in die Apheresesäule (4") mündet.

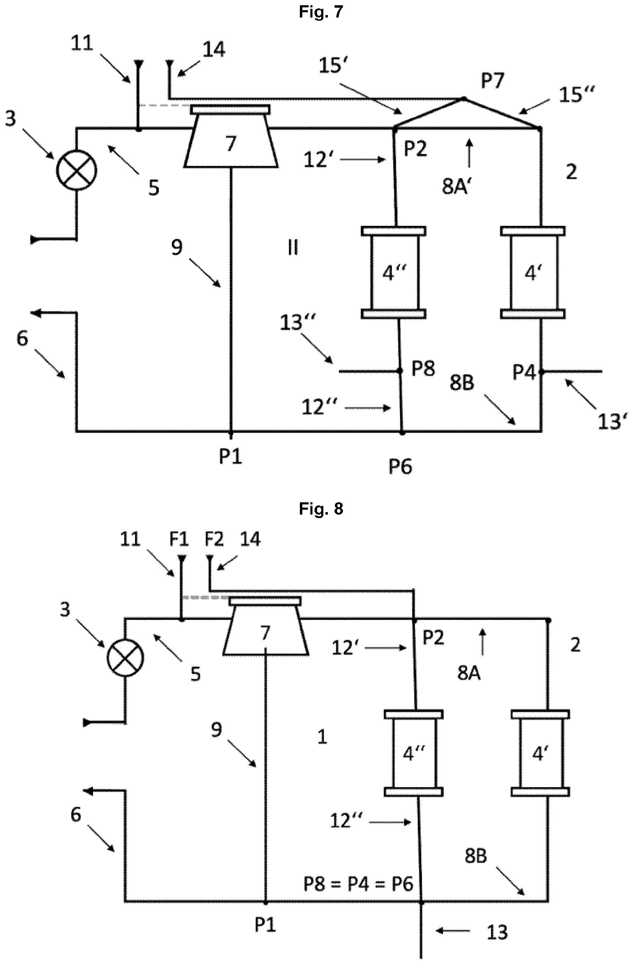

Weiterhin sind Ausführungsformen der erfindungsgemäßen Apheresevorrichtung (II) möglich, wobei die zumindest eine Regenerationsleitung (14) zu einem Punkt (P7) läuft und von dem Punkt (P7) eine Leitung (15') bis zum Punkt (P2) führt oder in die Plasmaleitung (8A) mündet und von dem Punkt (P7) eine Leitung (15") in Plasmaleitung (8A) (siehe Fig. 7).

Für den Fall, dass die zumindest eine Regenerationsleitung (14) für die Spüllösung in die Plasmaleitung (8A) zwischen den Punkt (P2) und der Apheresesäule (4') mündet oder für den Fall, dass die zumindest eine Regenerationsleitung (14) in den Bypass-Leitungsabschnitt (12') der Bypass-Leitung (12) zwischen den Punkt (P2) und der Apheresesäule (4") mündet, kann die Spüllösung entweder nur für die Apheresesäule (4') oder für die Apheresesäule (4") verwendet werden. Die Regenerationsleitung (14) ist somit entweder selektiv zur Apheresesäule (4') oder selektiv zur Apheresesäule (4").

Ebenso sind Ausführungsformen der erfindungsgemäßen Apheresevorrichtung (II) mit zwei, drei oder mehrere Regenerationsleitungen (14'. 14", 14''', etc.) möglich, wobei hierbei diese zwei, drei oder mehrere Regenerationsleitungen unabhängig voneinander in die Plasmaleitung (8A) [d.h. vom Punkt (P2) bis zur Apheresesäule (4')] oder in den Bypass-Leitungsabschnitt (12') [d.h. vom Punkt (P2) bis zur Apheresesäule (4")] oder in die Apheresesäule (4') oder in die Apheresesäule (4") münden können. "Unabhängig voneinander" bedeutet in diesem Zusammenhang zum Einen, dass bei einer Ausführungsform der erfindungsgemäßen Apheresevorrichtungen mit zwei Regenerationsleitungen (14', 14") die eine Regenerationsleitung (14') in die Plasmaleitung (8A) zwischen den Punkt (P2) und der Apheresesäule (4') mündet und die andere Regenerationsleitung (14") direkt in die Apheresesäule (4") mündet, aber auch dass beide Regenerationsleitungen (14', 14") in die Plasmaleitung (8A) zwischen den Punkt (P2) und der Apheresesäule (4') münden können. Ein weitere Möglichkeit ist, dass eine Regenerationsleitung (14') an dem Punkt (P2) in das extrakorporale Zirkulationssystem (2) mündet und die andere Regenerationsleitung (14") in den Bypass-Leitungsabschnitt (12') der Bypass-Leitung (12) zwischen den Punkt (P2) und der Apheresesäule (4") mündet. Auch denkbar ist, dass eine Regenerationsleitung (14') an dem Punkt (P2) in das extrakorporale Zirkulationssystem mündet und die andere Regenerationsleitung (14") in die Apheresesäule (4") mündet. Auch möglich ist, dass eine Regenerationsleitung (14') in die andere Regenerationsleitung (14") mündet. Bei Vorhandensein von zwei oder mehr Regenerationsleitungen (14', 14", 14''', etc.) ist es jedoch, dass bevorzugt alle Regenerationsleitungen (14', 14", 14''', etc.) am Punkt (P2) in das extrakorporale Zirkulationssystem (2) münden, wobei die Apheresevorrichtung (II) derart ausgestaltet ist, dass diese gegenüber einer Alkalihydroxid-Lösung vorzugsweise einer Natriumhydroxid-Lösung beständig ist.

Weiterhin bevorzugt ist, wenn eine Regenerationsleitung (14') in den Bypass-Leitungsabschnitt (12') der Bypass-Leitung (12) zwischen den Punkt (P2) und der Apheresesäule (4') mündet und die andere Regenerationsleitung (14") in den Bypass-Leitungsabschnitt (12') der Bypass-Leitung (12) zwischen dem Punkt P2 und der Apheresesäule (4") mündet. Weiterhin bevorzugt ist, wenn die Regenerationsleitung (14') in die Apheresesäule (4') und die andere Regenerationsleitung (14") in die Apheresesäule (4") mündet. Hierbei ist die Regenerationsleitung (14') selektiv zur ersten Apheresesäule (4') und die Regenerationsleitung selektiv zur zweiten Apheresesäule (4").

Gemäß einer besonders bevorzugten Ausführungsform der vorliegenden Erfindung umfasst die Apheresevorrichtung (II) daher des Weiteren eine Regenerationsleitung (14') für eine Spüllösung selektiv zur ersten Apheresesäule (4') und/oder des Weiteren eine Regenerationsleitung (14") für eine Spüllösung selektiv zur zweiten Apheresesäule (4").

Wie bereits oben erwähnt, können die für die Regeneration der Apheresesäulen notwendige Regenerationslösung über die Regenerationsleitung (14) in das extrakorporale Zirkulationssystem (2) eingespeist werden, und somit zusätzlich zur Spüllösung auch noch eine Regenerationslösung (z.B. Alkalihydroxid-Lösung vorzugsweise Natriumhydroxid-Lösung) eingesetzt werden. Die Spüllösung kann, muss aber nicht, zur Regeneration der ersten Apheresesäule (4') und/oder Apheresesäule (4") dienen, sondern hat neben der oben genannten Funktion die Aufgabe, das Blutplasma aus der Plasmaleitung (8A) in dem Bereich von Punkt (P2) bis zu der Apheresesäule (4') sowie aus der Plasmaleitung (8B) von der Apheresesäule (4') bis zu dem Punkt (P8) oder das Blutplasma aus dem Bypass-Leitungsabschnitt (12') der Bypass-Leitung (12) in dem Bereich von Punkt (P2) bis zu der Apheresesäule (4") sowie aus dem Bypass-Leitungsabschnitt (12") der Bypass-Leitung (12) von der Apheresesäule (4") bis zu dem Punkt (P8) zu verdrängen, bevor die Regenerationslösung eingesetzt wird, welche nach Durchfluss durch eine der beiden Apheresesäulen (4', 4") dann über die Ablaufleitung (13', 13") verworfen wird.

Somit ist es denkbar, dass die parallel geschaltet Apheresesäulen (4', 4") nicht nur abwechselnd betrieben werden können, sondern auch abwechselnd regeneriert werden.

In den hierin beschriebenen erfindungsgemäßen Apheresevorrichtungen (II) kann die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist.
Mit anderen Worten sind Apheresevorrichtungen (II) bevorzugt, wobei eine zweite Apheresesäule (4") parallel zur ersten Apheresesäule (4') geschaltet ist und beide Apheresesäulen (4', 4") nur abwechselnd betreibbar sind, und wobei die erste Apheresesäule (4') während des Betriebs der zweiten Apheresesäule (4") austauschbar oder regenerierbar ist und die zweite Apheresesäule (4") während des Betriebs der ersten Apheresesäule (4') austauschbar oder regenerierbar ist, wobei die Apheresevorrichtung (II) derart ausgestaltet ist, dass diese gegenüber einer Alkalihydroxid-Lösung vorzugsweise einer Natriumhydroxid-Lösung beständig ist.

Weiterhin sind Ausführungsformen der vorliegenden Erfindung denkbar, in dem die Apheresevorrichtung je Flüssigkeitsbehälter (F) eine Regenerationsleitung (14) aufweist, die von dem jeweiligen Flüssigkeitsbehälter (F) oder dessen Anschlussleitung (11) abgehen und die jeweils in die Plasmaleitung (8A) oder in die Bypass-Leitungsabschnitt (12') der Bypass-Leitung (12) oder direkt in die Apheresesäule (4') oder direkt in die Apheresesäule (4") führt.

Eine besonders bevorzugte Ausführungsform der zugrundeliegenden Erfindung betrifft eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (4") abgeht oder von dem Bypass-Leitungsabschnitt (12") der Bypass-Leitung (12) in Flussrichtung vor der Einmündung der Bypass-Leitungsabschnitt (12') der Bypass-Leitung (12), und je Flüssigkeitsbehälter (F) eine Regenerationsleitung (14) enthalten ist, die von dem jeweiligen Flüssigkeitsbehälter (F) oder dessen Anschlussleitung (11) abgehen und in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, wobei die Apheresevorrichtung (II) derart ausgestaltet ist, dass diese gegenüber einer Alkalihydroxid-Lösung vorzugsweise einer Natriumhydroxid-Lösung beständig ist.

Ferner sind Ausführungsformen der Apheresevorrichtung (II) bevorzugt, bei denen die Apheresevorrichtung (II) zumindest zwei Anschlussleitungen (11) zum Anschluss von jeweils zumindest einem Flüssigkeitsbehälter (F) an die arterielle Leitung (5) oder den Zellseparator (7) aufweist, und wobei je Flüssigkeitsbehälter (F) eine Regenerationsleitung (14) existiert, die von dem jeweiligen Flüssigkeitsbehälter (F) oder dessen Anschlussleitung (11) abgehen und die jeweils in die Plasmaleitung (8A) oder in den Bypass-Leitungsabschnitt (12') der Bypass-Leitung (12) oder direkt in die Apheresesäule (4') oder direkt in die Apheresesäule (4") führen.

Gemäß einigen Ausführungsformen der vorliegenden Erfindung bevorzugt, dass zumindest eine Regenerationsleitung (14), die in die Plasmaleitung (8A) oder in den Bypass-Leitungsabschnitt (12') der Bypass-Leitung (12) führt oder direkt in die Apheresesäule (4') oder direkt in die Apheresesäule (4") mündet, von einem Punkt (P5) in der zumindest einen Anschlussleitung (11) ausgeht.

Bevorzugt ist eine Apheresevorrichtung (II), wobei die Apheresevorrichtung (II) zwei Anschlussleitungen (11', 11') zum Anschluss von jeweils einem Flüssigkeitsbehälter (F1, F2) an die arterielle Leitung (5) oder den Zellseparator (7) aufweist, und zwei Regenerationsleitungen (14', 14") von zwei Flüssigkeitsbehältern (F1, F2) oder den zwei Anschlussleitungen (11', 11") abgehen und in die Plasmaleitung (8A) oder in den Bypass-Leitungsabschnitt (12') der Bypass-Leitung (12) oder direkt in Apheresesäule (4') oder direkt die Apheresesäule (4") führen.

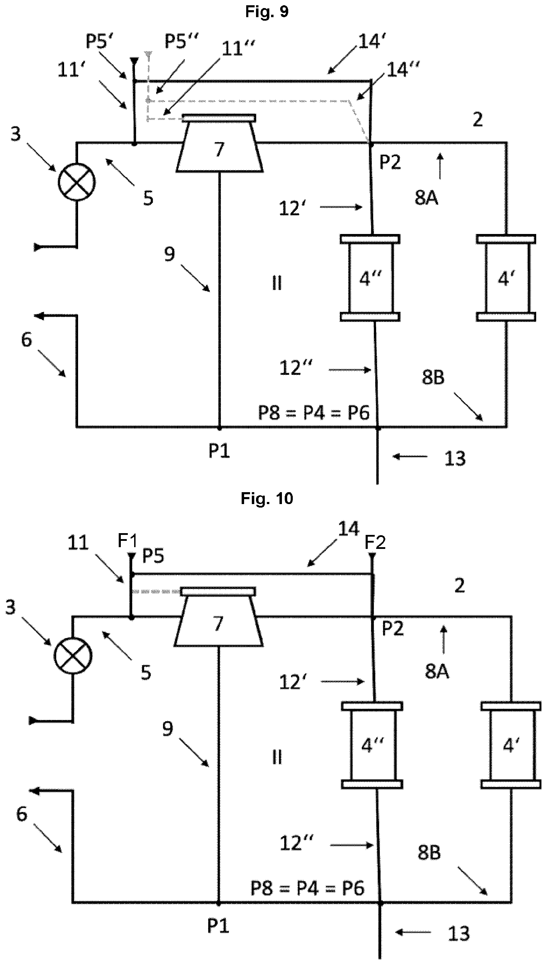

Besonders bevorzugt sind Ausführungsformen, in denen eine Regenerationsleitung (14), die in die Plasmaleitung (8A) oder in den Bypass-Leitungsabschnitt (12') der Bypass-Leitung (12) oder direkt in die Apheresesäule (4') oder direkt in die Apheresesäule (4") führt und die von einem Punkt (P5) in der zumindest einen Anschlussleitung (11) ausgeht, zumindest einen zusätzlichen Anschluss für einen Flüssigkeitsbehälter aufweist (Fig. 10). An diesen zusätzlichen Anschluss kann beispielweise ein Infusionsbeutel mit Alkalihydroxid-Lösung vorzugsweise Natriumhydroxid-Lösung angeschlossen werden.

In Ausführungsformen der vorliegenden Erfindung mit mehreren Anschlussleitungen (11', 11", 11''', etc.) und mehreren Regenerationsleitungen (14', 14", 14''', etc.) ist es möglich, dass jeweils eine Anschlussleitung mit jeweils einer Regenerationsleitung in Verbindung steht, welche wiederum nach dem Punkt (P2) in die Plasmaleitung (8A) oder in den Bypass-Leitungsabschnitt (12') der Bypass-Leitung (12) oder direkt in Apheresesäule (4') oder direkt in die Apheresesäule (4") mündet. Hierbei kann jede Regenerationsleitung unabhängig von den anderen Regenerationsleitungen in die Plasmaleitung (8A) oder in den Bypass-Leitungsabschnitt (12') der Bypass-Leitung (12) oder direkt in Apheresesäule (4') oder direkt in die Apheresesäule (4") münden. Bevorzugt ist jedoch, wenn alle Regenerationsleitungen direkt in die Apheresesäulen (4'; 4"), bevorzugt am Punkt (P2) in das extrakorporale Zirkulationssystem (2) münden. Eine derartige beispielhafte Ausführungsform sei anhand von Fig. 10 erläutert. Hierin besitzt die Apheresevorrichtung (II) eine erste Anschlussleitung (11'), die erstens in die arterielle Leitung (5) führt und von der zweitens am Punkt (P5') eine erste Regenerationsleitung (14') abzweigt. Die Apheresevorrichtung (II) besitzt zudem eine zweite Anschlussleitung (11"), die erstens direkt in den Zellseparator (7) führt und von der zweitens am Punkt (P5") eine zweite Regenerationsleitung (14") abzweigt. Beide Regenerationsleitungen münden in dieser Ausführungsform am Punkt (P2) in das extrakorporale Zirkulationssystem (2).

Bevorzugt ist demnach eine Apheresevorrichtung (II), wobei die Apheresevorrichtung (II) zwei Anschlussleitungen (11', 11") zum Anschluss von jeweils zumindest einem Flüssigkeitsbehälter (F) an die arterielle Leitung (5) oder den Zellseparator (7) aufweist, und wobei die zumindest eine Regenerationsleitung (14), die in die Plasmaleitung (8A) oder in den Bypass-Leitungsabschnitt (12') der Bypass-Leitung (12) oder direkt in die Apheresesäule (4') oder direkt in die Apheresesäule (4") führt, sich an einem Punkt (P5') mit der Anschlussleitung (11') und an einem Punkt (P5") mit der Anschlussleitung (11") verbindet.

Somit sind insbesondere Ausführungsformen der Apheresevorrichtung bevorzugt, wobei die Apheresevorrichtung (II) zwei Anschlussleitungen (11', 11") zum Anschluss von jeweils einem Flüssigkeitsbehälter (F1, F2) an die arterielle Leitung (5) oder den Zellseparator (7) aufweist, und wobei zumindest eine Regenerationsleitung (14), die in die Plasmaleitung (8A) oder in den Bypass-Leitungsabschnitt (12') der Bypass-Leitung (12) oder direkt die Apheresesäule (4') oder direkt in die Apheresesäule (4") führt, sich an einem Punkt (P5') mit der Anschlussleitung (11') und an einem Punkt (P5") mit der Anschlussleitung (11") verbindet und wobei eine Regenerationsleitung (14') von dem Flüssigkeitsbehälter (F1) oder von der vom Flüssigkeitsbehälter (F1) abgehenden Anschlussleitung (11') zur Apheresesäule (4') oder zur Apheresesäule (4") oder zur Plasmaleitung (8A') oder zur Plasmaleitung (8A") und eine Regenerationsleitung (14") von dem Flüssigkeitsbehälter (F2) oder der vom Flüssigkeitsbehälter (F2) abgehenden Anschlussleitung (11") zur Apheresesäule (4') oder zur Apheresesäule (4") oder zur Plasmaleitung (8A) oder zur Bypass-Leitungsabschnitt (12') der Bypass-Leitung (12) oder in die Regenerationsleitung (14') führt.

Somit ist besonders bevorzugt, wenn die Apheresevorrichtung (II) eine Anschlussleitung (11') für den Anschluss eines Flüssigkeitsbehälters (F1) und eine Anschlussleitung (11") für den Anschluss eines Flüssigkeitsbehälters (F2) aufweist und die Anschlussleitung (11') in die arterielle Leitung (5) oder in den Zellseparator (7) mündet und die Anschlussleitung (11") in die arterielle Leitung (5) oder in den Zellseparator (7) oder in Anschlussleitung (11') und damit letztendlich auch in die arterielle Leitung (5) oder in den Zellseparator (7) mündet und eine Regenerationsleitung (14') von dem Flüssigkeitsbehälter (F1) oder von der Anschlussleitung (11') zur Apheresesäule (4') oder zur Apheresesäule (4") oder zur Plasmaleitung (8A) oder zur Plasmaleitung (8A") führt und eine Regenerationsleitung (14") von dem Flüssigkeitsbehälter (F2) oder von der Anschlussleitung (11") zur Apheresesäule (4') oder zur Apheresesäule (4") oder zur Plasmaleitung (8A') oder zum Bypass-Leitungsabschnitt (12') der Bypass-Leitung (12) oder in die Regenerationsleitung (14') führt.

Ausführungsformen der Apheresevorrichtung (II) sind daher insbesondere bevorzugt, bei denen die Apheresevorrichtungen (II) eine Anschlussleitung (11') zum Anschluss von einem Flüssigkeitsbehälter (F1) an die arterielle Leitung (5) oder den Zellseparator (7) und eine Anschlussleitung (11") zum Anschluss von einem Flüssigkeitsbehälter (F2) an die arterielle Leitung (5) oder den Zellseparator (7) aufweist, und wobei eine Regenerationsleitung (14') von dem Flüssigkeitsbehälter (F1) oder der Anschlussleitung (11') abgeht und in die Plasmaleitung (8A) oder in den Bypass-Leitungsabschnitt (12') der Bypass-Leitung (12) oder direkt in die Apheresesäule (4') oder direkt in die Apheresesäule (4") führt und eine Regenerationsleitung (14") von einem Flüssigkeitsbehälter (F2) oder der Anschlussleitung (11") abgeht und in die Plasmaleitung (8A) oder in den Bypass-Leitungsabschnitt (12') der Bypass-Leitung (12) oder in die Regenerationsleitung (14') oder direkt in die Apheresesäule (4') oder direkt in die Apheresesäule (4") führt.

Die vorliegende Erfindung ist daher ebenso gerichtet auf eine erfindungsgemäße Apheresevorrichtung (II), wobei die Plasmaleitung (8A) und der Bypass-Leitungsabschnitt (12') der Bypass-Leitung (12) von einem Punkt (P2) auseinanderlaufen, und die Plasmaleitung (8B) und der Bypass-Leitungsabschnitt (12") der Bypass-Leitung (12) Plasmaleitung (8B) am Punkt (P6) zusammenlaufen, und die Abfallleitung (13') von einem Punkt (P4) von der Plasmaleitung (8B) abgeht und die Abfallleitung (13") von einem Punkt (P8) von dem Bypass-Leitungsabschnitt (12") der Bypass-Leitung (12) abgeht, und die zumindest eine Regenerationsleitung (14) am Punkt (P2) in das extrakorporale Zirkulationssystem (2) mündet.

Eine bevorzugte Ausführungsform der zugrundeliegenden Erfindung betrifft eine Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile, zwei Apheresesäulen (4', 4") zur affinitätschromatographischen Entfernung von CRP aus Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1),
eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
dadurch gekennzeichnet, dass
ein Bypass-Leitungsabschnitt (12') der Bypass-Leitung (12) von der Plasmaleitung (8A) abzweigt und in die zweite Apheresesäule (4') mündet und der Bypass-Leitungsabschnitt (12") der Bypass-Leitung (12") ausgehend von der Apheresesäule (4") in die Plasmaleitung (8B) mündet,
eine Abfallleitung (13'), die direkt von der Apheresesäule (4') abgeht oder von der Plasmaleitung (8B) in Flussrichtung vor der Einmündung des Bypass-Leitungsabschnitt (12') der Bypass-Leitung (12) abgeht, eine Abfallleitung (13"), die direkt von der Apheresesäule (14") abgeht oder von dem Bypass-Leitungsabschnitt (12") der Bypass-Leitung (12) in Flussrichtung vor der Einmündung der Bypass-Leitungsabschnitt (12') der Bypass-Leitung (12), wobei die Plasmaleitung (8A) und der Bypass-Leitungsabschnitt (12') der Bypass-Leitung (12) von einem Punkt (P2) auseinanderlaufen, und die Plasmaleitung (8B) und der Bypass-Leitungsabschnitt (12") der Bypass-Leitung (12) an einem Punkt (P6) zusammenlaufen, und
zumindest eine Regenerationsleitung (14), die in Flussrichtung nach der Abzweigung des Bypass-Leitungsabschnitts (12') der Bypass-Leitung (12) zu der Plasmaleitung (8A) oder zu dem Bypass-Leitungsabschnitts (12') der Bypass-Leitung (12) führt oder direkt in die Apheresesäule (4') oder direkt in die Apheresesäule (4") mündet, und
die Abfallleitung (13') von einem Punkt (P4) von der Plasmaleitung (8B) abgeht und die Abfallleitung (13") von einem Punkt (P8) von dem Bypass-Leitungsabschnitt (12") der Bypass-Leitung (12) abgeht, und
die zumindest eine Regenerationsleitung (14) am Punkt (P2) in das extrakorporale Zirkulationssystem (2) mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, d.h. nur abwechselnd nutzbar sind, wobei die Apheresevorrichtung (II) derart ausgestaltet ist, dass diese gegenüber einer Alkalihydroxid-Lösung vorzugsweise einer Natriumhydroxid-Lösung beständig ist.

Um das Totvolumen des Systems noch weiter zu verringern, ist es noch weiter bevorzugt, wenn nicht nur die Regenerationsleitung (14) an dem Punkt (P2), an dem die Plasmaleitung (8A) und der Bypass-Leitungsabschnitt (12') der Bypass-Leitung (12) auseinanderlaufen, in das extrakorporale Zirkulationssystem mündet, sondern wenn auch die Ablaufleitungen (13', 13") von demselben Punkt (P6), an dem die Plasmaleitung (8B) und der Bypass-Leitungsabschnitt (12") der Bypass-Leitung (12) zusammenlaufen, abzweigen. Anders ausgedrückt ist bevorzugt, wenn der Punkt (P6), an dem die Plasmaleitungen (8B) und der Bypass-Leitungsabschnitt (12") der Bypass-Leitung (12) zusammenlaufen, der Punkt (P8), an dem die Abfallleitung (13") abzweigt und der Punkt (P4), an dem Abfallleitung (13') abzweigt übereinstimmen, d.h. wenn P8 = P4 = P6 ist (siehe hierzu Fig. 8).

Die vorliegende Erfindung ist daher ebenso gerichtet auf eine erfindungsgemäße Apheresevorrichtung (II), wobei die Plasmaleitung (8B) und der Bypass-Leitungsabschnitt (12") der Bypass-Leitung (12) an einem Punkt (P6) zusammenlaufen, und die Abfallleitung (13") von einem Punkt (P8) von dem Bypass-Leitungsabschnitt (12") der Bypass-Leitung (12) abgeht und die Abfallleitung (13') von einem Punkt (P4) von der Plasmaleitung (8B) abgeht, und die zumindest eine Regenerationsleitung (14) am Punkt (P2) in das extrakorporale Zirkulationssystem (2) mündet und wobei der Punkt (P6), der Punkt (P4) und der Punkt (P8) identisch sind.

Gemäß der vorliegenden Erfindung umfasst eine erfindungsgemäße Ausführungsform der Apheresevorrichtung (II) zur extrakorporalen Entfernung von CRP aus Blut zwei Apheresesäulen (4', 4") zur affinitätschromatographischen Entfernung von CRP aus dem Blut oder Blutplasma, dessen Funktion in der Bindung von CRP besteht, welches sich im Blut bzw. im Blutplasma eines Patienten befindet und das durch die Apheresesäule (4') oder (4") hindurch geleitet wird.

### Verfahren

Ein weiterer Aspekt der vorliegenden Erfindung betrifft auch ein Verfahren zur Regeneration einer Apheresesäule (4) zur affinitätschromatographischen Entfernung von CRP in einer Apheresevorrichtung (1), wobei das Verfahren die Regeneration im laufenden Betrieb ermöglicht und durch die folgenden Schritte gekennzeichnet ist:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4),
(G) Schließung der Abfallleitung (13) und Weiterleiten des aus der Apheresesäule (4) austretenden Flüssigkeitsstromes in die venöse Leitung (6).

Ein weiterer Aspekt der vorliegenden Erfindung betrifft auch ein Verfahren zur Regeneration einer Apheresesäule (4) zur affinitätschromatographischen Entfernung von CRP in einer Apheresevorrichtung (1), wobei das Verfahren die Regeneration im laufenden Betrieb ermöglicht und die folgenden Schritte umfasst:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung.

Ein weiterer Aspekt der vorliegenden Erfindung betrifft auch ein Verfahren zur Regeneration einer Apheresesäule (4) zur affinitätschromatographischen Entfernung von CRP in einer Apheresevorrichtung (1), wobei das Verfahren die Regeneration im laufenden Betrieb ermöglicht und die folgenden Schritte umfasst:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4),
(G) Schließung der Abfallleitung (13).

Ein weiterer Aspekt der vorliegenden Erfindung betrifft auch ein Verfahren zur Regeneration einer Apheresesäule (4) zur affinitätschromatographischen Entfernung von CRP in einer Apheresevorrichtung (1), wobei das Verfahren die Regeneration im laufenden Betrieb ermöglicht und die folgenden Schritte umfasst:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4).

Unter dem Begriff "Stoppen der Einleitung des abgetrennten Plasmas" gemäß Schritt (A) kann je nach Ausführungsform der vorliegenden Erfindung die Verwendung von Schlauchklemmen, Steuerungselementen, Ventilen und/oder Schlauchpumpen zum Verhindern des weiteren Flusses von Blutplasma in die Plasmaleitung (8A) oder in den Bypass-Leitungsabschnitt (12') der Bypass-Leitung (12) oder in die in die Apheresesäule (4') oder (4") verstanden werden.

Unter dem Begriff "Stoppen der Einleitung von Regenerationslösung" gemäß Schritt (D) kann je nach Ausführungsform der vorliegenden Erfindung die Verwendung von Schlauchklemmen, Steuerungselementen, Ventilen und/oder Schlauchpumpen zum Verhindern des weiteren Flusses von Regenerationslösung in die Plasmaleitung (8A) bzw. in die Apheresesäule (4) verstanden werden. Hierbei soll verstanden werden, dass in Ausführungsformen, bei denen nur eine Regenerationslösung verwendet wird, die Einleitung ebendieser gestoppt wird. In Ausführungsformen, in denen mehrere Regenerationslösungen sukzessive eingeleitet werden, bedeutet dies, dass die Einleitung der zuletzt verwendeten Regenerationslösung gestoppt wird und damit auch die Einleitung jeglicher Regenerationslösung beendet wird.

Unter dem Begriff "Schließen der Abfallleitung (13)" gemäß Schritt (E) kann je nach Ausführungsform der vorliegenden Erfindung die Verwendung von Schlauchklemmen, Steuerungselementen, Ventilen und/oder Schlauchpumpen zum Verhindern des weiteren Flusses des aus der Apheresesäule (4) austretenden Flüssigkeitsstromes verstanden werden. Hierbei soll verstanden werden, dass in Ausführungsformen, bei denen nur eine Regenerationslösung verwendet wird, die Einleitung eben dieser gestoppt wird. In Ausführungsformen, in denen mehrere Regenerationslösungen sukzessive eingeleitet werden, bedeutet dies, dass die Einleitung der zuletzt verwendeten Regenerationslösung gestoppt wird und damit auch die Einleitung jeglicher Regenerationslösung beendet wird.

Durch "Weiterleiten des aus der Apheresesäule (4) austretenden Flüssigkeitsstromes" gemäß Schritt (E) fließt das abgetrennte Plasma fortan nach Durchlaufen der Apheresesäule (4) wieder in die Plasmaleitung (8B) und von dort weiter durch die venöse Leitung (6). Je nach Ausführungsform der vorliegenden Erfindung können zur Änderung der Flussrichtung des aus der Apheresesäule (4) austretenden Flüssigkeitsstromes Schlauchklemmen, Steuerungselemente, Ventile und/oder Schlauchpumpen verwendet werden.

Ferner betrifft die vorliegende Erfindung ein Verfahren zur Regeneration einer Apheresesäule (4) zur affinitätschromatographischen Entfernung von CRP in einer Apheresevorrichtung (1) wie hierin beschrieben, wobei das Verfahren die Regeneration im laufenden Betrieb durch Umschalten von einem Apherese-Modus in einen Regenerations-Modus ermöglicht, wobei im Apherese-Modus Plasma, das mittels des Zellseparators (7) aus Blut abgetrennt wird, über die Plasmaleitung (8A) in die Apheresesäule (4) geleitet wird und der aus der Apheresesäule (4) austretende Flüssigkeitsstrom über die Plasmaleitung (8B) in die venöse Leitung (6) geleitet wird;
und wobei der Regenerations-Modus durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4),
(E) Schließung der Abfallleitung (13).

Bevorzugt ist ein Schritt (E) Schließung der Abfallleitung (13) und Umschalten in den Apherese-Modus. Weiter bevorzugt ist ein Schritt (E) Schließung der Abfallleitung (13) und Weiterleiten des aus der Apheresesäule (4) austretenden Flüssigkeitsstromes in die venöse Leitung (6) und damit wieder ein Umschalten in den Apherese-Modus.

Hinsichtlich der beiden vorgenannten Verfahren handelt es sich bei der Regenerationslösung vorzugsweise um eine Alkalihydroxid-Lösung vorzugsweise eine Natrium hydroxid-Lösung.

Ferner sind Verfahren bevorzugt, bei denen Schritt (C) initiiert wird, nachdem ein Gesamtvolumen X an Regenerationslösung/-en in die Plasmaleitung (8A) bzw. direkt in die Apheresesäule (4) eingeleitet wurde, wobei X mindestens 75 % des Volumens der Vorrichtung zwischen dem Punkt, an dem die Regenerationsleitung (14) in Flussrichtung nach der Abzweigung der Bypass-Leitung (12) in das extrakorporale Zirkulationssystem (2) mündet, und dem Punkt, an dem die Abfallleitung (13) dem extrakorporalen Zirkulationssystem (2) entspringt, entspricht. Hier handelt es sich bei der Regenerationslösung beispielsweise um eine Alkalihydroxid-Lösung vorzugsweise eine Natriumhydroxid-Lösung.

Zudem sind Verfahren bevorzugt, bei denen Schritt (E) initiiert wird, nachdem ein Volumen Y an Plasma in die Plasmaleitung (8A) oder direkt in die Apheresesäule (4) eingeleitet wurde, wobei Y mindestens 90 % des Volumens der Vorrichtung zwischen dem Punkt, an dem die Regenerationsleitung (14) in Flussrichtung nach der Abzweigung der Bypass-Leitung (12) in das extrakorporale Zirkulationssystem (2) mündet, und dem Punkt, an dem die Abfallleitung (13) dem extrakorporalen Zirkulationssystem (2) entspringt, entspricht.

"**Im laufenden Betrieb",** wie hierin verwendet, bedeutet, dass zur Durchführung des erfindungsgemäßen Verfahrens zur Regeneration einer Apheresesäule (4) die Blutabnahme und -zufuhr sowie der Betrieb des Zellseparators nicht gestoppt werden müssen. Anders ausgedrückt wird während des erfindungsgemäßen Verfahrens zur Regeneration einer Apheresesäule (4) das kontinuierlich gewonnene Plasma über die Bypass-Leitung (12) unter Umgehung der Apheresesäule (4) mit den Zellbestandteilen zusammengeführt und dem Patienten zugeführt. Während der Regeneration ist die Apheresesäule somit von der Blutabnahme und -zufuhr bzw. Blutkreislauf entkoppelt. Während der Zeit, in der die Umleitung des Plasmas über die Bypass-Leitung (12) stattfindet, wird die in der Regel in ihrer Kapazität geminderte Apheresesäule (4) regeneriert. Somit wird der Kreislauf des Patienten nicht belastet, weil das kontinuierlich entnommene Blut ohne Verzögerung dem Patienten wieder zurückgeführt wird.

"Im laufenden Betrieb" wie hierin verwendet bedeutet demnach nicht, dass zur Durchführung des erfindungsgemäßen Verfahrens zur Regeneration einer Apheresesäule (4) die kontinuierliche Plasmagewinnung unterbrochen werden muss. Ferner bedeutet es auch nicht, dass während der Regeneration der Apheresesäule die CRP-Abreicherung stattfindet.

Somit ist bei den beiden vorgenannten und den allgemein hierin offenbarten Verfahren bevorzugt, dass die Einleitung von Regenerationslösung die Einleitung einer einzelnen Regenerationslösung oder die sukzessive Einleitung mehrerer Regenerationslösungen umfasst.

Für den Fachmann ist völlig klar, dass ein initialer Spülvorgang des Adsorbers bzw. des gesamten Systems vor der Ausführung des erfindungsgemäßen Verfahrens stattgefunden haben muss. Damit verbunden ist ein Vorfüllen des gesamten Schlauchsystems. Hierzu können unter Umständen weitere Anschlüsse an dem System vorhanden sein, die ein Durchspülen des gesamten Systems ermöglichen. Nachdem der Patient vom Schlauchsystem getrennt wurde besteht die Möglichkeit der Konservierung des Adsorbers, sodass dieser für eine weitere Behandlung an demselben Patienten wieder verwendet werden kann.

Anders ausgedrückt betrifft die vorliegende Erfindung auch ein erfindungsgemäßes Verfahren zur Regeneration einer Apheresesäule (4) zur affinitätschromatographischen Entfernung von CRP in der Apheresevorrichtung (1), wobei das Verfahren die Regeneration im laufenden Betrieb ermöglicht und durch die folgenden Schritte gekennzeichnet ist:
(A) Umleitung des abgetrennten Plasmas aus der Plasmaleitung (8A) in die Bypass-Leitung (12),
(B) Einleitung zumindest einer Regenerationslösung aus einem Flüssigkeitsbehälter über die Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(C) Umleitung des aus der Apheresesäule (4) austretenden Flüssigkeitsstromes von der Plasmaleitung (8B) in die Abfallleitung (13),
(D) Stoppen der Einleitung der Regenerationslösung,
(E) Beginn der Einleitung einer Neutralisationslösung,
(F) Umleitung des abgetrennten Plasmas aus der Plasmaleitung (8A) in die Apheresesäule (4) und Stoppen der Einleitung von Neutralisationslösung,
(G) Schließung der Abfallleitung (13).

Bevorzugt ist ein Schritt (G) Schließung der Abfallleitung (13) und Weiterleiten des aus der Apheresesäule (4) austretenden Flüssigkeitsstromes in die venöse Leitung (6).

Der Begriff "Umleitung" wie hierin verwendet betrifft eine Änderung der Fließrichtung der jeweiligen Flüssigkeit. Während des Behandlungsmodus strömt das abgetrennte Plasma durch die Plasmaleitung (8A) in die Apheresesäule (4). Nach Verlassen der Apheresesäule (4) strömt das abgereicherte Plasma durch die Plasmaleitung (8B) in die venöse Leitung (6).

Durch die "Umleitung" der Fließrichtung des abgetrennten Plasmas gemäß Schritt (A) fließt das abgetrennte Plasma fortan nicht mehr durch die Apheresesäule (4), sondern umgeht diese, indem es in die Bypass-Leitung (12) umgeleitet wird.

Unter dem Begriff "Einleitung" wie hierin verwendet gemäß Schritt (B) kann je nach Ausführungsform der vorliegenden Erfindung die Einspeisung zumindest einer Regenerationslösung (unter Verwendung bzw. Betätigung von Schlauchklemmen, Steuerungselementen, Ventilen und/oder Schlauchpumpen) in die Plasmaleitung (8A) bzw. in die Apheresesäule (4) verstanden werden.

Durch die "Umleitung" der Fließrichtung des aus der Apheresesäule (4) austretenden Flüssigkeitsstromes gemäß Schritt (C) fließt die austretende Flüssigkeit fortan nicht mehr in die Plasmaleitung (8B) sondern direkt in die Abfallleitung (13). Erfindungsgemäß ist es bevorzugt, dass die Abfallleitung (13) direkt bzw. unmittelbar von bzw. nach der Apheresesäule (4) abzweigt, um somit das Volumen an Regenerationslösung, das nötig ist um die Apheresesäule (4) zu regenerieren, minimiert wird. Im Sinne der Erfindung kann die Abfallleitung (13) auch aus der Plasmaleitung (8B) abzweigen und muss somit nicht direkt aus der Apheresesäule abzweigen.

Durch die "Umleitung" der Fließrichtung des abgetrennten Plasmas gemäß Schritt (D) fließt das abgetrennte Plasma fortan wieder durch die Apheresesäule (4) und nicht mehr in die Bypass-Leitung (12). In bestimmten Ausführungsformen ist eine Pumpe in der Bypass-Leitung (12) vorhanden, wodurch das in der Bypass-Leitung (12) vorhandene Plasma nach Umleitung gemäß Schritt (D) in die Plasmaleitung (8B) und über die venöse Leitung (6) gepumpt wird. Hierbei wird bevorzugt das in der Bypass-Leitung befindliche Plasma durch eine NaCl-Lösung aus der Regenerationsleitung (14) verdrängt. Bevorzugt handelt es sich dabei um eine 0,9%ige NaCl Lösung. Ebenso wäre es denkbar, dass ein separater Flüssigkeitsbehälter an die Bypass-Leitung (12) angeschlossen werden kann, durch den besagte NaCl-Lösung zur Verdrängung bereitgestellt wird.

Besonders bevorzugt ist daher ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(E) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4),
(F) Stoppen der Einleitung von Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13).

Bevorzugt ist ein Schritt (G) Schließung der Abfallleitung (13) und Weiterleiten des aus der Apheresesäule (4) austretenden Flüssigkeitsstromes in die venöse Leitung (6).

Alternativ ist auch besonders bevorzugt ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(D) Beginn der Umleitung des aus der Apheresesäule (4) austretenden Flüssigkeitsstromes von der Plasmaleitung (8B) in die Abfallleitung (13),
(E) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4),
(F) Schließung der Abfallleitung (13);
(G) Stoppen der Einleitung von Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (F) Schließung der Abfallleitung (13) und Weiterleiten des aus der Apheresesäule (4) austretenden Flüssigkeitsstromes in die venöse Leitung (6).

Bei den beiden vorgenannten Verfahren wird zusätzlich zur Regenerationslösung noch eine Spüllösung eingesetzt. Die Spüllösung ist vorzugsweise physiologisch verträglich und dient vorrangig zur Verdrängung des Blutplasmas aus der Plasmaleitung (8A) ab dem Punkt P2, aus der Apheresesäule (4) sowie aus der Plasmaleitung (8B) bis zum Punkt P4. Die Spüllösung dient weniger oder nicht zur Regeneration der Apheresesäule (4). Erst wenn das Blutplasma aus dem mit Regenerationslösung zu durchspülenden Abschnitt der Apheresevorrichtung (1) weitestgehend bis vollständig verdrängt ist, wird die Regenerationslösung eingeleitet, um die Apheresesäule (4) zu regenerieren. Nach erfolgter Regeneration wird dann erst wieder Spüllösung in den mit Regenerationslösung durchspülten Abschnitt der Apheresevorrichtung (1) (also in Flussrichtung von Punkt P2 durch die Apheresesäule (4) bis zum Punkt P4) geleitet, bis die Regenerationslösung vollständig durch die Abfallleitung (13) entsorgt worden ist. Erst dann wird die Bypass-Leitung (12) geschlossen und wieder Blutplasma durch die Apheresesäule (4) geleitet. Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden. Die Ausführung des Schrittes (D) vor dem Schritt (C) ist jedoch bevorzugt.

Bei diesem Verfahren handelt es sich bei der Spüllösung vorzugsweise um eine physiologische NaCI-Lösung oder PBS-Lösung und bei der Regenerationslösung um eine Alkalihydroxid-Lösung vorzugsweise eine Natriumhydroxid-Lösung.

Besonders bevorzugt ist daher ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(E) Stoppen der Einleitung der Regenerationslösung und Übergang zur Einleitung der Neutralisationslösung über die zumindest eine Regenerationsleitung (14) in die Plasmaleitung (8A) oder direkt in die Apheresesäule (4),
(F) Stoppen der Einleitung der Neutralisationslösung und Übergang zur Einleitung der Spüllösung über die zumindest eine Regenerationsleitung (14) in die Plasmaleitung (8A) oder direkt in die Apheresesäule (4),
(G) Stoppen der Einleitung der Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(H) Schließung der Abfallleitung (13).

Bevorzugt ist ein Schritt (H) Schließung der Abfallleitung (13) und Weiterleiten des aus der Apheresesäule (4) austretenden Flüssigkeitsstromes in die venöse Leitung (6).

Alternativ ist auch besonders bevorzugt ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4), wobei die Regenerationslösung eine Alkalihydroxid-Lösung vorzugsweise eine Natriumhydroxid-Lösung ist,
(D) Beginn der Umleitung des aus der Apheresesäule (4) austretenden Flüssigkeitsstromes von der Plasmaleitung (8B) in die Abfallleitung (13),
(E) Stoppen der Einleitung der Regenerationslösung und Übergang zur Einleitung der Neutralisationslösung über die zumindest eine Regenerationsleitung (14) in die Plasmaleitung (8A) oder direkt in die Apheresesäule (4),
(F) Stoppen der Einleitung der Neutralisationslösung und Übergang zur Einleitung der Spüllösung über die zumindest eine Regenerationsleitung (14) in die Plasmaleitung (8A) oder direkt in die Apheresesäule (4),
(G) Schließung der Abfallleitung (13);
(H) Stoppen der Einleitung der Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (G) Schließung der Abfallleitung (13) und Weiterleiten des aus der Apheresesäule (4) austretenden Flüssigkeitsstromes in die venöse Leitung (6).

Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden. Die Ausführung des Schrittes (D) vor dem Schritt (C) ist jedoch bevorzugt.

Die bevorzugte Ausführungsform des erfindungsgemäßen Verfahrens dient zur effizienteren Durchführung des Verfahrens ohne Verlust von Blutplasma. Durch die zeitgleiche Umleitung des abgetrennten Plasmas und der parallelen Einleitung der Spüllösung in die Apheresesäule (4) entsteht kein Verlust oder kein nennenswerter Verlust von Blutplasma. Ferner ist an der bevorzugten Ausführungsform von Vorteil, dass ein Durchmischen von Regenerationslösung und Blutplasma vollständig vermieden wird. Dadurch wird sichergestellt, dass keine Regenerationslösung in den Patienten gelangt und andererseits kein Verlust von Blutplasma für den Patienten auftritt.

Dies wird durch die sequenzielle Abfolge der Schritte (B) bis (E) sichergestellt. Eine Verdünnung des Blutplasmas findet wenn überhaupt nur durch Spüllösung statt. Hingegen wird eine Vermischung von Blutplasma mit Regenerationslösung vollständig vermieden.
Das Volumen an Spüllösung gemäß Schritt (B) entspricht vorzugsweise dem 3- bis 4-fachen Volumen der Matrix der Apheresesäule (4).
Minimal entspricht das Volumen an Spüllösung gemäß Schritt (B) dem Volumen der Plasmaleitung (8A) ab Punkt P2 bis zur Apheresesäule (4) zuzüglich dem Volumen der Matrix der Apheresesäule (4) und zuzüglich dem Volumen der Plasmaleitung (8B) ab der Apheresesäule (4) bis zum Punkt P4.

Das Volumen an Regenerationslösung gemäß Schritt (C) entspricht vorzugsweise dem 2-bis 100-fachen Volumen der Matrix der Apheresesäule (4).

Das Volumen an Spüllösung gemäß Schritt (E) entspricht vorzugsweise dem 2- bis 4-fachen Volumen der Matrix der Apheresesäule (4).
Zumindest entspricht das Volumen an Spüllösung gemäß Schritt (E) dem Volumen der Plasmaleitung (8A) ab Punkt P2 bis zur Apheresesäule (4) zuzüglich dem Volumen der Matrix der Apheresesäule (4) und zuzüglich dem Volumen der Plasmaleitung (8B) ab der Apheresesäule (4) bis zum Punkt P4.

Gemäß dieser noch bevorzugteren Ausführungsform wird eine Verdünnung des Plasmas weitgehend vermieden und eine Durchmischung mit Regenerationslösung vollständig verhindert. Der Benutzer oder die Benutzerin ist mit keiner zu großen Komplexität hinsichtlich der Benutzung der Apheresevorrichtung (1) konfrontiert. In einer alternativen Ausführungsform können somit die Verfahrensschritte auch manuell betätigt werden, ohne zu komplex für den Benutzer oder die Benutzerin zu erscheinen oder zu sein.

Das "Volumen der Matrix der Apheresesäule", wie hierin verwendet, bedeutet das Volumen der festen Phase innerhalb der Säule, welche wiederum ein Matrixsubstratmaterial und daran gebundene Verbindungen umfasst, die die Eigenschaft haben, CRP spezifisch zu binden. Abzugrenzen hiervon ist das "Totvolumen der Apheresesäule", d.h. der Raum innerhalb der Säule, der der mobilen Phase (z.B. des Plasmas) zur Verfügung steht. Das "Totvolumen der Apheresesäule" ergibt sich aus der Differenz aus dem durch das Gehäuse der Apheresesäule umschlossenen Volumen und dem Volumen, das durch die gequollene Matrix beansprucht wird (d.h. dem "Volumen der Matrix der Apheresesäule").

Ein weiterer Aspekt der vorliegenden Erfindung ist gerichtet auf ein Verfahren zur Regeneration einer Apheresesäule (4') zur affinitätschromatographischen Entfernung von CRP während des laufenden Betriebes einer zweiten Apheresesäule (4") in einer Apheresevorrichtung (II) umfassend die folgenden Schritte:
(A) Ausgehend vom Fluss des Blutplasmas durch die Apheresesäule (4"), Beginn der Einleitung des abgetrennten Blutplasmas über die Plasmaleitung (8A) in die Apheresesäule (4') und Stoppen der Einleitung des abgetrennten Plasmas über den Bypass-Leitungsabschnitt (12') der Bypass-Leitung (12) in die Apheresesäule (4"),
(B) Beginn der Einleitung von Regenerationslösung über die zumindest eine Regenerationsleitung (14) in den Bypass-Leitungsabschnitt (12') der Bypass-Leitung (12) oder direkt in die Apheresesäule (4"), wobei zumindest eine Regenerationslösung eine Alkalihydroxid-Lösung vorzugsweise eine Natriumhydroxid-Lösung ist,
(C) Beginn der Umleitung des aus der Apheresesäule (4") austretenden Flüssigkeitsstromes von dem Bypass-Leitungsabschnitt (12") der Bypass-Leitung (12) in die Abfallleitung (13"),
(D) Beginn der Einleitung einer Neutralisationslösung über die zumindest eine Regenerationsleitung (14) in den Bypass-Leitungsabschnitt (12') der Bypass-Leitung (12) oder direkt in die Apheresesäule (4"),
(E) Stoppen der Einleitung der Neutralisationslösung,
(F) Beginn der Einleitung des abgetrennten Blutplasmas über den Bypass-Leitungsabschnitt (12') der Bypass-Leitung (12) in die Apheresesäule (4")und Stoppen der Einleitung des abgetrennten Blutplasmas über die Plasmaleitung (8A) in Apheresesäule (4'),
(G) Schließung der Abfallleitung (13") und Beginn der Umleitung des aus der Apheresesäule (4') austretenden Flüssigkeitsstromes von der Plasmaleitung (8B) in die Abfallleitung (13').
Bevorzugt ist ein Schritt (A) Ausgehend vom Fluss des Blutplasmas durch die Apheresesäule (4"), Beginn der Einleitung des abgetrennten Blutplasmas über die Plasmaleitung (8A) in die Apheresesäule (4') und Leiten des CRP-abgereicherten Blutplasmas in die venöse Leitung (6) und damit Stoppen der Einleitung des abgetrennten Plasmas über den Bypass-Leitungsabschnitt (12') der Bypass-Leitung (12) in die Apheresesäule (4").
Bevorzugt ist ein Schritt (F) Beginn der Einleitung des abgetrennten Blutplasmas über den Bypass-Leitungsabschnitt (12') der Bypass-Leitung (12) in die Apheresesäule (4") und Leiten des CRP-abgereicherten Plasmas in die venöse Leitung (6) und damit Stoppen der Einleitung des abgetrennten Blutplasmas über die Plasmaleitung (8A) in Apheresesäule (4').

**"Im laufenden Betrieb",** wie in diesem Zusammenhang verwendet, bedeutet, dass zur Durchführung des erfindungsgemäßen Verfahrens zur Regeneration einer Apheresesäule (4') oder Regeneration einer Apheresesäule (4") die Blutabnahme und -zufuhr sowie der Betrieb des Zellseparators nicht gestoppt werden müssen. Somit wird der Kreislauf des Patienten nicht belastet, weil das kontinuierlich entnommene Blut ohne Verzögerung dem Patienten wieder zurückgeführt wird.

Anders ausgedrückt betrifft die vorliegende Erfindung in einer Ausführungsform ein Verfahren zur Regeneration einer Apheresesäule (4') zur affinitätschromatographischen Entfernung von CRP während des laufenden Betriebes einer zweiten Apheresesäule (4") in einer Apheresevorrichtung (II), umfassend die folgenden Schritte:
(A) Ausgehend vom Fluss des Blutplasmas durch die Apheresesäule (4"), Einleitung des abgetrennten Plasmas über die Plasmaleitung (8A) in die Apheresesäule (4')und Stoppen der Einleitung des abgetrennten Plasmas über den Bypass-Leitungsabschnitt (12') der Bypass-Leitung (12) in die Apheresesäule (4"),
(B) Einleitung zumindest einer Regenerationslösung über die zumindest eine Regenerationsleitung (14) in den Bypass-Leitungsabschnitt (12') der Bypass-Leitung (12) oder direkt in die Apheresesäule (4"), wobei zumindest eine Regenerationslösung eine Alkalihydroxid-Lösung vorzugsweise eine Natriumhydroxid-Lösung ist,
(C) Umleitung des aus der Apheresesäule (4") austretenden Flüssigkeitsstromes von dem Bypass-Leitungsabschnitt (12") der Bypass-Leitung (12) in die Abfallleitung (13"),
(D) Beginn der Einleitung einer Neutralisationslösung über die zumindest eine Regenerationsleitung (14) in den Bypass-Leitungsabschnitt (12') der Bypass-Leitung (12) oder direkt in die Apheresesäule (4"),
(E) Stoppen der Einleitung der Neutralisationslösung,
(F) Einleitung des abgetrennten Plasmas über den Bypass-Leitungsabschnitt (12') der Bypass-Leitung (12) in die Apheresesäule (4")und Stoppen der Einleitung des abgetrennten Plasmas über die Plasmaleitung (8A) in Apheresesäule (4')
(G) Schließung der Abfallleitung (13") und Umleitung des aus der Apheresesäule (4') austretenden Flüssigkeitsstromes von der Plasmaleitung (8B) in die Abfallleitung (13').

Bevorzugt ist ein Schritt (A) Ausgehend vom Fluss des Blutplasmas durch die Apheresesäule (4"), Einleitung des abgetrennten Plasmas über die Plasmaleitung (8A) in die Apheresesäule (4') und Leiten des CRP-abgereicherten Plasmas in die venöse Leitung (6) und damit Stoppen der Einleitung des abgetrennten Plasmas über den Bypass-Leitungsabschnitt (12') der Bypass-Leitung (12) in die Apheresesäule (4").

Bevorzugt ist ein Schritt (F) Einleitung des abgetrennten Plasmas über den Bypass-Leitungsabschnitt (12') der Bypass-Leitung (12) in die Apheresesäule (4") und Leiten des CRP-abgereicherten Plasmas in die venöse Leitung (6) und damit Stoppen der Einleitung des abgetrennten Plasmas über die Plasmaleitung (8A) in Apheresesäule (4').

Ferner betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4')und Stoppen der Einleitung des abgetrennten Plasmas über den Bypass-Leitungsabschnitt (12') der Bypass-Leitung (12) in die Apheresesäule (4"),
(B) Beginn der Einleitung einer Spüllösung über die zumindest eine Regenerationsleitung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der Spüllösung und Übergang zur Einleitung der Regenerationslösung über die zumindest eine Regenerationsleitung (14) in den Bypass-Leitungsabschnitt (12') der Bypass-Leitung (12) oder direkt in die Apheresesäule (4"), wobei die Regenerationslösung eine Alkalihydroxid-Lösung vorzugsweise eine Natriumhydroxid-Lösung ist,
(E) Stoppen der Einleitung der Regenerationslösung und Übergang zur Einleitung der Spüllösung über die zumindest eine Regenerationsleitung (14) in den Bypass-Leitungsabschnitt (12') der Bypass-Leitung (12) oder direkt in Apheresesäule (4"),
(F) Beginn der Einleitung der Spüllösung in die Plasmaleitung (8A) über die Apheresesäule (4') und damit Einleitung des abgetrennten Plasmas über den Bypass-Leitungsabschnitt (12') der Bypass-Leitung (12) in Apheresesäule (4"),
(G) Schließung der Abfallleitung (13"),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4').
(J) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in die venöse Leitung (6) und damit Stoppen der Einleitung des abgetrennten Plasmas über den Bypass-Leitungsabschnitt (12') der Bypass-Leitung (12) in die Apheresesäule (4").
Bevorzugt ist ein Schritt (G) Schließung der Abfallleitung (13") und Weiterleiten des aus der Apheresesäule (4") austretenden Flüssigkeitsstromes in die venöse Leitung (6).

Bei den beiden vorgenannten Verfahren wird zusätzlich zur Regenerationslösung noch eine Spüllösung eingesetzt. Die Spüllösung ist vorzugsweise physiologisch verträglich und dient vorrangig zur Verdrängung des Blutplasmas aus der Plasmaleitung (8A) oder dem Bypass-Leitungsabschnitt (12') der Bypass-Leitung (12) ab dem Punkt P2, aus der Apheresesäule (4') oder Apheresesäule (4") sowie aus der Bypass-Leitungsabschnitt (12") der Bypass-Leitung (12) bis zum Punkt P3 und der Plasmaleitung (8B) bis zum Punkt P4. Die Spüllösung dient weniger oder nicht zur Regeneration der Apheresesäule (4') oder der Apheresesäule (4"). Durch die Spüllösung wird daher der Plasmaverlust minimiert oder sogar vollständig vermieden. Erst wenn das Blutplasma aus dem mit Regenerationslösung zu durchspülenden Abschnitt der Apheresevorrichtung (II) weitestgehend bis vollständig verdrängt ist, wird die Regenerationslösung eingeleitet, um die Apheresesäule (4') oder die Apheresesäule (4") zu regenerieren. Nach erfolgter Regeneration wird dann erst wieder Spüllösung in den mit Regenerationslösung durchspülten Abschnitt der Apheresevorrichtung (II) (also in Flussrichtung von Punkt P2 durch die Apheresesäule (4") bis zum Punkt P8) oder durch die Apheresesäule (4') bis zum Punkt P4) geleitet, bis die Regenerationslösung vollständig durch die Abfallleitungen (13', 13") entsorgt worden ist.

Bei diesem Verfahren handelt es sich bei der Spüllösung vorzugsweise um eine Kochsalzlösung bzw. eine physiologische Kochsalzlösung oder um eine PBS-Lösung (phosphate buffered saline) oder um eine Kombination von Kochsalzlösung und PBS-Lösung nacheinander oder zeitgleich und bei der Regenerationslösung um eine Alkalihydroxid-Lösung vorzugsweise eine Natriumhydroxid-Lösung.
Ferner betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4')und Stoppen der Einleitung des abgetrennten Plasmas über den Bypass-Leitungsabschnitt (12') der Bypass-Leitung (12) in die Apheresesäule (4"),
(B) Beginn der Einleitung einer Spüllösung über die zumindest eine Regenerationsleitung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der Spüllösung und Übergang zur Einleitung der Regenerationslösung über die zumindest eine Regenerationsleitung (14) in den Bypass-Leitungsabschnitt (12') der Bypass-Leitung (12) oder direkt in die Apheresesäule (4"), wobei die Regenerationslösung eine Alkalihydroxid-Lösung vorzugsweise eine Natriumhydroxid-Lösung ist,
(E) Stoppen der Einleitung der Regenerationslösung und Übergang zur Einleitung der Neutralisationslösung über die zumindest eine Regenerationsleitung (14) in den Bypass-Leitungsabschnitt (12') der Bypass-Leitung (12) oder direkt in Apheresesäule (4"),
(F) Stoppen der Einleitung der Neutralisationslösung und Übergang zur Einleitung der Neutralisationslösung über die zumindest eine Regenerationsleitung (14) in den Bypass-Leitungsabschnitt (12') der Bypass-Leitung (12) oder direkt in Apheresesäule (4"),
(G) Beginn der Einleitung der Spüllösung in die Plasmaleitung (8A) über die Apheresesäule (4') und damit Einleitung des abgetrennten Plasmas über den Bypass-Leitungsabschnitt (12') der Bypass-Leitung (12) in Apheresesäule (4"),
(H) Schließung der Abfallleitung (13"),
(I) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4').
(J) Stoppen der Einleitung der Regenerationslösung und Übergang zur Einleitung der Spüllösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in die venöse Leitung (6) und damit Stoppen der Einleitung des abgetrennten Plasmas über den Bypass-Leitungsabschnitt (12') der Bypass-Leitung (12) in die Apheresesäule (4").
Bevorzugt ist ein Schritt (H) Schließung der Abfallleitung (13") und Weiterleiten des aus der Apheresesäule (4") austretenden Flüssigkeitsstromes in die venöse Leitung (6).

Bei den beiden vorgenannten Verfahren sind die Schritte (C) und (D) vertauschbar, d.h. können in beliebiger Reihenfolge und auch zeitgleich erfolgen und können auch in einem Schritt zusammengefasst werden. Die Ausführung des Schrittes (D) vor dem Schritt (C) ist jedoch bevorzugt.

Die bevorzugte Ausführungsform des erfindungsgemäßen Verfahrens dient zur effizienteren Durchführung des Verfahrens ohne Verlust von Blutplasma. Durch die zeitgleiche Umleitung des abgetrennten Plasmas und der parallelen Einleitung der Spüllösung in die Apheresesäule (4") entsteht kein Verlust oder kein nennenswerter Verlust von Blutplasma. Ferner ist an der bevorzugten Ausführungsform von Vorteil, dass ein Durchmischen von Regenerationslösung und Blutplasma vollständig vermieden wird. Dadurch wird sichergestellt, dass keine Regenerationslösung in den Patienten gelangt und andererseits kein Verlust von Blutplasma für den Patienten auftritt.

Dies wird durch die sequenzielle Abfolge der Schritte (B) bis (E) sichergestellt. Eine Verdünnung des Blutplasmas findet wenn überhaupt nur durch Spüllösung statt. Hingegen wird eine Vermischung von Blutplasma mit Regenerationslösung vollständig vermieden.

Das Volumen an Spüllösung gemäß Schritt (B) entspricht vorzugsweise dem 3- bis 4-fachen Volumen der Matrix der Apheresesäule (4"). Minimal entspricht das Volumen an Spüllösung gemäß Schritt (B) dem Volumen dem Bypass-Leitungsabschnitt (12') der Bypass-Leitung (12) ab Punkt P2 bis zur Apheresesäule (4") zuzüglich dem Volumen der Matrix der Apheresesäule (4") und zuzüglich dem Volumen dem Bypass-Leitungsabschnitt (12') der Bypass-Leitung (12) ab der Apheresesäule (4") bis zum Punkt (P3).

Das Volumen an Spüllösung gemäß Schritt (F) entspricht vorzugsweise dem 3- bis 4-fachen Volumen der Matrix der Apheresesäule (4'). Minimal entspricht das Volumen an Spüllösung gemäß Schritt (B) dem Volumen der Plasmaleitung (8A') ab dem Punkt (P2) bis zur Apheresesäule (4') zuzüglich dem Volumen der Matrix der Apheresesäule (4') und zuzüglich dem Volumen der Plasmaleitung (8B) ab der Apheresesäule bis zum Punkt (P4).

Das Volumen an Regenerationslösung gemäß Schritt (D) entspricht vorzugsweise dem 2- bis 100-fachen Volumen der Matrix der Apheresesäule (4").

Das Volumen an Regenerationslösung gemäß Schritt (I) entspricht vorzugsweise dem 2- bis 100-fachen Volumen der Matrix der Apheresesäule (4').

Das Volumen an Spüllösung gemäß Schritt (E) entspricht vorzugsweise dem 2- bis 4-fachen Volumen der Matrix der Apheresesäule (4").
Zumindest entspricht das Volumen an Spüllösung gemäß Schritt (E) dem Volumen des Bypass-Leitungsabschnitt (12') der Bypass-Leitung (12) ab Punkt P2 bis zur Apheresesäule (4") zuzüglich dem Volumen der Matrix der Apheresesäule (4") und zuzüglich dem Volumen des Bypass-Leitungsabschnitt (12') der Bypass-Leitung (12) ab der Apheresesäule (4') bis zum Punkt P3.

Das Volumen an Spüllösung gemäß Schritt (J) entspricht vorzugsweise dem 2- bis 4-fachen Volumen der Matrix der Apheresesäule (4').
Zumindest entspricht das Volumen an Spüllösung gemäß Schritt (E) dem Volumen der Plasmaleitung (8A) ab Punkt P2 bis zur Apheresesäule (4') zuzüglich dem Volumen der Matrix der Apheresesäule (4') und zuzüglich dem Volumen der Plasmaleitung (8B) ab der Apheresesäule (4') bis zum Punkt P4.

Bei einer bevorzugten konkreten Ausführungsform handelt es sich bei der Spüllösung um eine Kochsalzlösung bzw. eine physiologische Kochsalzlösung oder um eine PBS-Lösung (phosphate buffered saline) oder um eine Kombination von Kochsalzlösung und PBS-Lösung nacheinander oder zeitgleich und bei der Regenerationslösung um eine Alkalihydroxid-Lösung vorzugsweise eine Natriumhydroxid-Lösung.

Weiterhin betrifft eine bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(E) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4),
(F) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13).
Bevorzugt ist ein Schritt (G) Schließung der Abfallleitung (13) und Weiterleiten des aus der Apheresesäule (4) austretenden Flüssigkeitsstromes in die venöse Leitung (6).

Alternativ betrifft die bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4),
(F) Schließung der Abfallleitung (13);
(G) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (F) Schließung der Abfallleitung (13) und Weiterleiten des aus der Apheresesäule (4) austretenden Flüssigkeitsstromes in die venöse Leitung (6).

Weiterhin betrifft eine bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(E) Stoppen der Einleitung der Alkalihydroxid-Lösung vorzugsweise der Natriumhydroxid-Lösung und Übergang zur Einleitung der Citrat-Lösung über die zumindest eine Regenerationsleitung (14) in die Plasmaleitung (8A) oder direkt in die Apheresesäule (4),
(F) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(G) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(H) Schließung der Abfallleitung (13).

Bevorzugt ist ein Schritt (H) Schließung der Abfallleitung (13) und Weiterleiten des aus der Apheresesäule (4) austretenden Flüssigkeitsstromes in die venöse Leitung (6).

Alternativ betrifft die bevorzugte konkrete Ausführungsform ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E) Stoppen der Einleitung der Alkalihydroxid-Lösung vorzugsweise der Natriumhydroxid-Lösung und Übergang zur Einleitung der Citrat-Lösung über die zumindest eine Regenerationsleitung (14) in die Plasmaleitung (8A) oder direkt in die Apheresesäule (4),
(F) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4),
(G) Schließung der Abfallleitung (13);
(H) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (G) Schließung der Abfallleitung (13) und Weiterleiten des aus der Apheresesäule (4) austretenden Flüssigkeitsstromes in die venöse Leitung (6).

Bei den vier vorgenannten Verfahren sind die Schritte (C) und (D) vertauschbar, d.h. können in beliebiger Reihenfolge und auch zeitgleich erfolgen und können auch in einem Schritt zusammengefasst werden.

Bevorzugt werden die erfindungsgemäßen Regenerationsverfahren so durchgeführt, dass zuerst das Plasma mit einer Spüllösung, wie z.B. einer Kochsalzlösung bzw. physiologischen Kochsalzlösung, aus der Apheresesäule (4) verdrängt und zurück in den Patienten geführt wird bis hin zu dem Punkt, dass fast nur noch Kochsalzlösung zurückgeführt wird. Erst dann wird die Kochsalzlösung in die Abfallleitung (13) geleitet und Regenerationslösung, wie z.B. eine Alkalihydroxid-Lösung vorzugsweise eine Natriumhydroxid-Lösung, wird in Flussrichtung nach der Bypass-Leitung (12) in die Plasmaleitung (8A) eingeleitet, welche die Kochsalzlösung verdrängt, die Apheresesäule (4) regeneriert, vollständig in die Abfallleitung (13) eingeleitet und verworfen wird. Nach erfolgter Regeneration der Apheresesäule (4) mit mehreren Apheresesäulevolumina an Regenerationslösung wird wieder eine Spüllösung, wie z.B. eine Kochsalzlösung bzw. physiologische Kochsalzlösung, so lange eingeleitet, bis die Regenerationslösung vollständig aus der Apheresevorrichtung (1) verdrängt und verworfen ist. Erst danach erfolgt die Schließung der Abfallleitung (13), die Rückführung der Spüllösung in den Patienten, die Schließung der Bypass-Leitung (12) und die erneute Einleitung von Plasma durch die Plasmaleitung (8A) in die Apheresesäule (4) zeitgleich oder unmittelbar nacheinander, wobei die Reihenfolge der Schritte vertauschbar ist.

Eine weitere bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(E1) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4),
(E2) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E3) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13).

Bevorzugt ist ein Schritt (G) Schließung der Abfallleitung (13) und Weiterleiten des aus der Apheresesäule (4) austretenden Flüssigkeitsstromes in die venöse Leitung (6).

Eine alternative bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E1) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4),
(E2) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E3) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Schließung der Abfallleitung (13);
(G) Stoppen der Einleitung der Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (F) Schließung der Abfallleitung (13) und Weiterleiten des aus der Apheresesäule (4) austretenden Flüssigkeitsstromes in die venöse Leitung (6).

Eine weitere bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(E1) Stoppen der Einleitung der Alkalihydroxid-Lösung vorzugsweise der Natriumhydroxid-Lösung und Übergang zur Einleitung einer Citrat-Lösung über die zumindest eine Regenerationsleitung (14) in die Plasmaleitung (8A) oder direkt in die Apheresesäule (4),
(E2) Stoppen der Einleitung der Citrat-Lösung und Übergang zur Einleitung einer Kochsalzlösung über die zumindest eine Regenerationsleitung (14) in die Plasmaleitung (8A) oder direkt in die Apheresesäule (4),
(E3) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E4) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(G) Schließung der Abfallleitung (13).

Bevorzugt ist ein Schritt (G) Schließung der Abfallleitung (13) und Weiterleiten des aus der Apheresesäule (4) austretenden Flüssigkeitsstromes in die venöse Leitung (6).

Eine alternative bevorzugte konkrete Ausführungsform betrifft ein Verfahren zur Regeneration einer Apheresesäule (4) zur affinitätschromatographischen Entfernung von CRP in einer Apheresevorrichtung (1), wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Kochsalzlösung über die zumindest eine Regenerationsleitung (14) in die Plasmaleitung (8A) oder direkt in die Apheresesäule (4),
(C) Stoppen der Einleitung der Kochsalzlösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4),
(D) Beginn der Umleitung des aus der Apheresesäule (4) austretenden Flüssigkeitsstromes von der Plasmaleitung (8B) in die Abfallleitung (13),
(E1) Stoppen der Einleitung der Alkalihydroxid-Lösung vorzugsweise der Natriumhydroxid-Lösung und Übergang zur Einleitung einer Citrat-Lösung über die zumindest eine Regenerationsleitung (14) in die Plasmaleitung (8A) oder direkt in die Apheresesäule (4),
(E2) Stoppen der Einleitung der Citrat-Lösung und Übergang zur Einleitung einer Kochsalzlösung über die zumindest eine Regenerationsleitung (14) in die Plasmaleitung (8A) oder direkt in die Apheresesäule (4),
(E3) Stoppen der Einleitung der Kochsalzlösung und Übergang zur Einleitung einer PBS-Lösung über die zumindest eine Regenerationsleitung (14) in die Plasmaleitung (8A) oder direkt in die Apheresesäule (4),
(E4) Stoppen der Einleitung der PBS-Lösung und Übergang zur Einleitung einer Kochsalzlösung über die zumindest eine Regenerationsleitung (14) in die Plasmaleitung (8A) oder direkt in die Apheresesäule (4),
(F) Schließung der Abfallleitung (13);
(G) Stoppen der Einleitung von Kochsalzlösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4).

Bevorzugt ist ein Schritt (F) Schließung der Abfallleitung (13) und Weiterleiten des aus der Apheresesäule (4) austretenden Flüssigkeitsstromes in die venöse Leitung (6).

Bei den vier vorgenannten Verfahren sind die Schritte (C) und (D) vertauschbar, d.h. können in beliebiger Reihenfolge und auch zeitgleich erfolgen und können auch in einem Schritt zusammengefasst werden.

Somit betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Stoppen der Einleitung des abgetrennten Plasmas über den Bypass-Leitungsabschnitt (12') der Bypass-Leitung (12) in die Apheresesäule (4"),
(B) Beginn der Einleitung einer Kochsalzlösung über die zumindest eine Regenerationslösung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der Kochsalzlösung und Übergang zur Einleitung der Alkalihydroxid-Lösung vorzugsweise der Natriumhydroxid-Lösung über die zumindest eine Regenerationsleitung (14) in den Bypass-Leitungsabschnitt (12') der Bypass-Leitung (12) oder direkt in die Apheresesäule (4"),
(E) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in den Bypass-Leitungsabschnitt (12') der Bypass-Leitung (12) oder direkt in Apheresesäule (4"),
(F) Beginn der Einleitung der Kochsalzlösung in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13"),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4'),
(J) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"). Bevorzugt ist ein Schritt (G) Schließung der Abfallleitung (13") und Weiterleiten des aus der Apheresesäule (4") austretenden Flüssigkeitsstromes in die venöse Leitung (6)

Somit betrifft die vorliegende Erfindung ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4')und Stoppen der Einleitung des abgetrennten Plasmas über den Bypass-Leitungsabschnitt (12') der Bypass-Leitung (12) in die Apheresesäule (4"),
(B) Beginn der Einleitung einer Kochsalzlösung über die zumindest eine Regenerationslösung (14) in den Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12") der Bypass-Leitung (12) in die Abfallleitung (13"),
(D) Stoppen der Einleitung der Kochsalzlösung und Übergang zur Einleitung der Alkalihydroxid-Lösung vorzugsweise der Natriumhydroxid-Lösung über die zumindest eine Regenerationsleitung (14) in den Bypass-Leitungsabschnitt (12') der Bypass-Leitung (12) oder direkt in die Apheresesäule (4"),
(E) Stoppen der Einleitung der Alkalihydroxid-Lösung vorzugsweise derNatriumhydroxid-Lösung und Übergang zur Einleitung der Kochsalzlösung über die zumindest eine Regenerationsleitung (14) in den Bypass-Leitungsabschnitt (12') der Bypass-Leitung (12) oder direkt in Apheresesäule (4"),
(F) Beginn der Einleitung der Kochsalzlösung in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13"),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Alkalihydroxid-Lösung vorzugsweise einer Natriumhydroxid-Lösung über die zumindest eine Regenerationsleitung (13) in die Plasmaleitung (8A) oder direkt in die Apheresesäule (4'),
(J) Stoppen der Einleitung der Alkalihydroxid-Lösung vorzugsweise der Natriumhydroxid-Lösung und Übergang zur Einleitung einer Citrat-Lösung über die zumindest eine Regenerationsleitung (14) in die Plasmaleitung (8A) oder direkt in die Apheresesäule (4'),
(K) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"). Bevorzugt ist ein Schritt (G) Schließung der Abfallleitung (13") und Weiterleiten des aus der Apheresesäule (4") austretenden Flüssigkeitsstromes in die venöse Leitung (6).

Bevorzugt werden die erfindungsgemäßen Regenerationsverfahren so durchgeführt, dass zuerst das Plasma mit einer Spüllösung, wie z.B. einer Kochsalzlösung bzw. physiologischen Kochsalzlösung, aus der Apheresesäule (4") verdrängt wird bis hin zu dem Punkt, dass fast nur noch Kochsalzlösung durchgeführt wird. Erst dann wird bevorzugt die Kochsalzlösung in die Abfallleitung (13") geleitet und Regenerationslösung, wie z.B. eine Alkalihydroxid-Lösung vorzugsweise eine Natriumhydroxid-Lösung, wird in Flussrichtung an dem Punkt P2 in den Bypass-Leitungsabschnitt (12') der Bypass-Leitung (12) eingeleitet, welche die Kochsalzlösung verdrängt, die Apheresesäule (4") regeneriert, vollständig in die Abfallleitung (13") eingeleitet und verworfen wird. Nach erfolgter Regeneration der Apheresesäule (4") mit mehreren Apheresesäulevolumina an Regenerationslösung wird wieder eine Spüllösung, wie z.B. eine Kochsalzlösung bzw. physiologische Kochsalzlösung, so lange eingeleitet, bis die Regenerationslösung vollständig aus der Apheresevorrichtung (II) verdrängt und verworfen ist. Erst danach erfolgt die Schließung der Plasmaleitung (8A), die Rückführung der Spüllösung in den Patienten, und die erneute Einleitung von Plasma durch den Bypass-Leitungsabschnitt (12') der Bypass-Leitung (12) in die Apheresesäule (4") zeitgleich oder direkt nacheinander.

Eine weitere bevorzugte konkrete Ausführungsform betrifft ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Stoppen der Einleitung des abgetrennten Plasmas über den Bypass-Leitungsabschnitt (12') der Bypass-Leitung (12) in die Apheresesäule (4"),
(B) Beginn der Einleitung einer Kochsalzlösung über die zumindest eine Regenerationsleitung (14) in die Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12') der Bypass-Leitung (12) in die Abfallleitung (13"),
(D) Stoppen der Einleitung der Kochsalzlösung und Übergang zur Einleitung der Alkalihydroxid-Lösung vorzugsweise der Natriumhydroxid-Lösung über die zumindest eine Regenerationsleitung (14) in den Bypass-Leitungsabschnitt (12') der Bypass-Leitung (12) oder direkt in die Apheresesäule (4"),
(E1) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in den Bypass-Leitungsabschnitt (12') der Bypass-Leitung (12) oder direkt in Apheresesäule (4"),
(E2) Stoppen der Einleitung der Kochsalzlösung und Übergang zur Einleitung einer PBS-Lösung über die zumindest eine Regenerationsleitung (14) in den Bypass-Leitungsabschnitt (12') der Bypass-Leitung (12) oder direkt in die Apheresesäule (4"),
(E3) Stoppen der Einleitung der PBS-Lösung und Übergang zur Einleitung einer Kochsalzlösung über die zumindest eine Regenerationsleitung (14) in den Bypass-Leitungsabschnitt (12') der Bypass-Leitung (12) oder direkt in die Apheresesäule (4"),
(F) Beginn der Einleitung der Kochsalzlösung in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13"),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4'),
(J) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"). Bevorzugt ist ein Schritt (G) Schließung der Abfallleitung (13") und Weiterleiten des aus der Apheresesäule (4") austretenden Flüssigkeitsstromes in die venöse Leitung (6).

Eine weitere bevorzugte konkrete Ausführungsform betrifft ein Verfahren zur Regeneration von zwei Apheresesäulen (4',4") zur affinitätschromatographischen Entfernung von CRP in einer Apheresevorrichtung (II), wobei das Verfahren die Regeneration im laufenden Betrieb ermöglicht und durch die folgenden Schritte gekennzeichnet ist:
(A) Ausgehend vom Fluss des Blutplasmas durch die Apheresesäule (4"), Beginn der Einleitung des abgetrennten Plasmas über die Plasmaleitung (8A) in die Apheresesäule (4') und Stoppen der Einleitung des abgetrennten Plasmas über den Bypass-Leitungsabschnitt (12') der Bypass-Leitung (12) in die Apheresesäule (4"),
(B) Beginn der Einleitung einer Kochsalzlösung über die zumindest eine Regenerationsleitung (14) in die Bypass-Leitungsabschnitt (12') der Bypass-Leitung (12) oder direkt in die Apheresesäule (4"),
(C) Beginn der Einleitung des aus der Apheresesäule (4") austretenden Flüssigkeitsstromes von dem Bypass-Leitungsabschnitt (12') der Bypass-Leitung (12) in die Abfallleitung (13"),
(D) Stoppen der Einleitung der Kochsalzlösung und Übergang zur Einleitung der Alkalihydroxid-Lösung vorzugsweise der Natriumhydroxid-Lösung über die zumindest eine Regenerationsleitung (14) in den Bypass-Leitungsabschnitt (12') der Bypass-Leitung (12) oder direkt in die Apheresesäule (4"),
(E1) Stoppen der Einleitung der Alkalihydroxid-Lösung vorzugsweise der Natriumhydroxid-Lösung und Übergang zur Einleitung der Kochsalzlösung über die zumindest eine Regenerationsleitung (14) in den Bypass-Leitungsabschnitt (12') der Bypass-Leitung (12) oder direkt in Apheresesäule (4"),
(E2) Stoppen der Einleitung der Kochsalzlösung und Übergang zur Einleitung einer PBS-Lösung über die zumindest eine Regenerationsleitung (14) in den Bypass-Leitungsabschnitt (12') der Bypass-Leitung (12) oder direkt in die Apheresesäule (4"),
(E3) Stoppen der Einleitung der PBS-Lösung und Übergang zur Einleitung einer Kochsalzlösung über die zumindest eine Regenerationsleitung (14) in den Bypass-Leitungsabschnitt (12') der Bypass-Leitung (12) oder direkt in die Apheresesäule (4"),
(F) Beginn der Einleitung der Kochsalzlösung in die Plasmaleitung (8A) über die Apheresesäule (4') und damit Einleitung des abgetrennten Plasmas über den Bypass-Leitungsabschnitt (12') der Bypass-Leitung (12) in die Apheresesäule (4"),
(G) Schließung der Abfallleitung (13"),
(H) Beginn der Umleitung des aus der Apheresesäule (4') austretenden Flüssigkeitsstromes von der Plasmaleitung (8B) in die Abfallleitung (13'),
(I) Stoppen der Einleitung der Spüllösung und Übergang zur Einleitung einer Alkalihydroxid-Lösung vorzugsweise einer Natriumhydroxid-Lösung über die zumindest eine Regenerationsleitung (14) in die Plasmaleitung (8A) oder direkt in die Apheresesäule (4'),
(J) Stoppen der Einleitung der Alkalihydroxid-Lösung vorzugsweise der Natriumhydroxid-Lösung und Übergang zur Einleitung einer Citrat-Lösung über die zumindest eine Regenerationsleitung (14) in die Plasmaleitung (8A) oder direkt in die Apheresesäule (4'),
(K) Stoppen der Einleitung der Citrat-Lösung und Übergang zur Einleitung der Kochsalzlösung über die zumindest eine Regenerationsleitung (14) in die Plasmaleitung (8A) oder direkt in die Apheresesäule (4').

Bevorzugt ist ein Schritt (A) Ausgehend vom Fluss des Blutplasmas durch die Apheresesäule (4"), Beginn der Einleitung des abgetrennten Plasmas über die Plasmaleitung (8A) in die Apheresesäule (4') und Leiten des CRP-abgereicherten Plasmas in venöse Leitung (6) und damit Stoppen der Einleitung des abgetrennten Plasmas über den Bypass-Leitungsabschnitt (12') der Bypass-Leitung (12) in die Apheresesäule (4"). Bevorzugt ist ein Schritt (G) Schließung der Abfallleitung (13") und Weiterleiten des aus der Apheresesäule (4") austretenden Flüssigkeitsstromes in die venöse Leitung (6).

Bei allen vorgenannten Verfahren sind die Teile der Apheresevorrichtung, in welche die Alkalihydroxid-Lösung eingeleitet wird, beständig gegen die verwendete Alkalihydroxid-Lösung, insbesondere die verwendete Natriumhydroxid-Lösung.

### Beispiele

### Anwendungsbeispiel:

Der Begriff "Matrixvolumen" (auch als MV abgekürzt), wie hierin verwendet, bezieht sich auf das Volumen der Matrix, welche im Adsorber enthalten ist.

Der Begriff "Adsorbervolumen" (auch als AV abgekürzt), wie hierin verwendet, bezieht sich auf das Volumen des Adsorbergehäuses.

### Beispiel 1: Apherese mit einer Bypass-Leitung und einer Apheresesäule

### Vorbereitung:

In die Apheresevorrichtung (1) zur extrakorporalen Entfernung von CRP aus Blut eines Patienten gemäß Fig. 7, mit einer Plasmazentrifuge als Zellseparator (7), wird ein passendes Schlauchsystem eingelegt. An die Anschlussleitung bzw. Regenerationsleitung wird ein 5 L Beutel mit 0,9% NaCI-Lösung sowie ein 500 ml Beutel mit ACD-A Lösung (Acid-Citrate-Dextrose Lösung) und/oder ein 2000 ml Beutel mit 0,08 M Natriumhydroxid-Lösung und/oder ein 2000 ml Beutel mit Glycin/HCl und/oder ein 2000 ml Beutel PBS-Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden die Plasmaleitungen (8A und 8B) vor und nach dem Adsorber mit einem Adapter (ohne Adsorber dazwischen) verbunden, so dass ein geschlossenes System entsteht.

Durch Vorspülen mit 1 L 0,9 % NaCl Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte NaCl wird über P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, die Bypassleitung (12) und P4/P6 in den zweiten Abfallbeutel geleitet. Beginnt die Plasmaseparation, so wird nach einem Volumen, das der Schlauchleitung von der Plasmazentrifuge bis zum Punkt P4/6 entspricht, so umgeschaltet, dass das Plasma in die venöse Leitung (6), und damit zum Patienten zurück, fließt. Nachdem für 3 Minuten ein konstanter Plasmafluss von ca. 30 ml/min erzielt wurde, kann der erste Zyklus beginnen.
2. Die Bypassleitung (12) wird geschlossen und das Plasma wird über den Adsorber geleitet (Beladung). Dabei wird das in der Plasmaleitung (8A und 8B) und dem Adsorber befindliche NaCl bis zu einem Volumen, bestehend aus dem Volumen der Plasmaleitung (8A und 8B) plus dem AV, über P4/P6 in den zweiten Abfallbeutel geleitet. Der Adsorber wird anschließend mit 50 - 100 MV (1000 bis 2000 ml) Plasma beladen. Anschließend beginnt die Regeneration.

### 3. Regeneration

### Variante A - Glycin/HCl und PBS-Lösung

Dazu wird das Plasma wieder über die Bypassleitung (12) zum Patienten zurückgeführt. Der Adsorber wird nun über die Regenerationsleitung (14) und die Plasmaleitung (8A und 8B) mit 0,9% NaCl gespült (30 ml/min). Das dafür benötigte Volumen berechnet sich aus dem AV und dem Volumen der Plasmaleitung (8A und 8B). Das in der Plasmaleitung (8A und 8B) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 3 MV (60 ml) 0,9% NaCl und danach mit 4 MV (80 ml) Glycin/HCl regeneriert (100 ml/min). Anschließend wird it 4 MV (80 ml) PBS neutralisiert. Danach wird mit 0,9% NaCl nachgespült (100 ml/min). Das dafür benötigte Volumen berechnet sich aus dem AV, dem Volumen der Regenerationsleitung (14) und der Plasmaleitung (8A und 8B).

Anschließend kann wieder Schritt 2 (Beladung), danach Schritt 3 erfolgen.

### Variante B - Natriumhydroxid-Lösung und PBS-Lösung

Dazu wird das Plasma wieder über die Bypassleitung (12) zum Patienten zurückgeführt. Der Adsorber wird nun über die Regenerationsleitung (14) und die Plasmaleitung (8A und 8B) mit 0,9% NaCl gespült (30 ml/min-40ml/min). Das dafür benötigte Volumen berechnet sich aus dem AV und dem Volumen der Plasmaleitung (8A und 8B). Das in der Plasmaleitung (8A und 8B) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 3 MV (60 ml) 0,9% NaCl vorgespült und danach mit 5 MV 0,08 M NaOH (pH 12,6; Flussrate von 80 ml/min) regeneriert. Danach wird mit 6 MV PBS-Lösung (pH 12,6; Flussrate 80ml/min) neutralisiert. Anschließend wird das PBS mit 4 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung bzw. PBS-Lösung ersetzt werden.

### Variante C - Natriumhydroxid-Lösung und Citrat-Lösung

Dazu wird das Plasma wieder über die Bypassleitung (12) zum Patienten zurückgeführt. Der Adsorber wird nun über die Regenerationsleitung (14) und die Plasmaleitung (8A und 8B) mit 0,9% NaCl gespült (30 ml/min-40ml/min). Das dafür benötigte Volumen berechnet sich aus dem AV und dem Volumen der Plasmaleitung (8A und 8B). Das in der Plasmaleitung (8A und 8B) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 3 MV (60 ml) 0,9% NaCl vorgespült und danach mit 5 MV 0,1 M NaOH (pH 12,9; Flussrate von 80 ml/min) regeneriert. Danach wird mit 4 MV 4% Citrat-Lösung (pH 7; Flussrate 80ml/min) neutralisiert. Anschließend wird das Citrat mit 3 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung bzw. Citrat-Lösung ersetzt werden.

### Variante D - Natriumhydroxid-Lösung und Natriumchlorid-Lösung

Dazu wird das Plasma wieder über die Bypassleitung (12) zum Patienten zurückgeführt. Der Adsorber wird nun über die Regenerationsleitung (14) und die Plasmaleitung (8A und 8B) mit 0,9% NaCl gespült (30 ml/min-40ml/min). Das dafür benötigte Volumen berechnet sich aus dem AV und dem Volumen der Plasmaleitung (8A und 8B). Das in der Plasmaleitung (8A und 8B) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 2 MV (60 ml) 0,9% NaCl vorgespült und danach mit 5 MV 0,1 M NaOH (pH 12,9; Flussrate von 80 ml/min) regeneriert. Anschließend wird das Natriumhydroxid mit 6 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung ersetzt werden.

4. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) sowie das restliche Plasma aus der Plasmaleitung bis P2 und der Bypassleitung (12) verdrängt und dem Patienten zurückgeführt. Das dazu benötigte Volumen setzt sich zusammen aus dem Volumen der Plasmazentrifuge (7), dem Volumen der Plasmaleitung bis P2, Bypassleitung (12), der Zellleitung (9) und der arteriellen Leitung (6). Der Patient kann anschließend von der Apheresevorrichtung getrennt werden.

### Variante E - Kaliumhydroxid-Lösung und PBS-Lösung

Versuchsdurchführung wie Variante B. Nach der Spülung mit NaCI-Lösung wird mit 5 MV 0,08 M KOH (pH 13,2; Flussrate von 80 ml/min) regeneriert.

### Variante F - Kaliumhydroxid-Lösung und Citrat-Lösung

Versuchsdurchführung wie Variante C. Nach der Spülung mit NaCI-Lösung wird mit 5 MV 0,1 M KOH (pH 13,4; Flussrate von 80 ml/min) regeneriert.

### Variante G - Kaliumhydroxid-Lösung und Natriumchlorid-Lösung

Versuchsdurchführung wie Variante D. Nach der Spülung mit NaCI-Lösung wird mit 5 MV 0,1 M KOH (pH 13,3; Flussrate von 80 ml/min) regeneriert.

### Variante H - Lithiumhydroxid-Lösung und PBS-Lösung

Versuchsdurchführung wie Variante B. Nach der Spülung mit NaCI-Lösung wird mit 5 MV 0,08 M LiOH (pH 13,3; Flussrate von 80 ml/min) regeneriert.

### Variante I - Lithiumhydroxid-Lösung und Citrat-Lösung

Versuchsdurchführung wie Variante C. Nach der Spülung mit NaCI-Lösung wird mit 5 MV 0,1 M LiOH (pH 13,5; Flussrate von 80 ml/min) regeneriert.

### Variante J - Lithiumhydroxid-Lösung und Natriumchlorid-Lösung

Versuchsdurchführung wie Variante D. Nach der Spülung mit NaCI-Lösung wird mit 5 MV 0,1 M LiOH (pH 13,5; Flussrate von 80 ml/min) regeneriert.

### 5. Konservierung

### Variante A

Falls gewünscht, kann nun der NaCI-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Variante B

Über die Regenerationsleitung wird der Adsorber mit 10 MV Natriumhydroxid-Lösung als Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Ergebnisse:

Bei der Regeneration nach Variante A mit Glycin/HCl-Lösung wurde die Bildung einer Proteinschicht um die Matrixpartikel (Agarosepartikel) beobachtet. Dies beruht vermutlich aufgrund des sehr niedrigen pH-Wertes von pH 2 - 3 auf einer sauren Proteinfällung. Wenn das zu reinigende Blut des Patienten eine hohe Konzentration von zellfreier DNA/RNA enthielt, führte dies zu einer Verstärkung des Effekts. Es hat sich gezeigt, dass durch die Ausbildung der Proteinschicht in der Apheresesäule Bindungsstellen maskiert werden und die Leistung des Apheresematerials verringert wird. Der ursprüngliche Zustand konnte mit bekannten Maßnahmen wie weiteren Regenerationsversuchen mit einer Glycin/HCl-Lösung nicht wiederhergestellt werden. Mit fortschreitender Schädigung der Apheresesäule erhöht sich die Behandlungszeit für den Patienten und somit die Leidenszeit des Patienten. Zudem waren die beschädigten Apheresesäulen für einen weiteren Einsatz oft nicht mehr brauchbar, sodass die Behandlungskosten erheblich steigen. Weiterhin kann es durch die Proteinschicht bzw. Protein-DNA sowie Protein-RNA Schicht zu einer Verstopfung der feinen Poren kommen, wodurch der Systemdruck bei gleichbleibender Flussrate steigt. Eine weitere Erhöhung der Flussrate geht mit einer weiteren Druckerhöhung einher. Dies kann zu einem Abbruch der Behandlung führen. Diese Apheresesäulen waren ebenfalls für einen weiteren Einsatz nicht mehr brauchbar.

Bei der Regeneration nach Variante B, C, D, E, F, G, H, I und J mit Natriumhydroxid-Lösung, Kaliumhydroxid-Lösung bzw. Lithiumhydroxid-Lösung hat sich gezeigt, dass einerseits eine basische Regeneration mit der Alkalihydroxid-Lösung auch eine bereits geschädigte Adsorbermatrix regenerieren kann. Überraschenderweise hat sich gezeigt, dass bei der Verwendung von nur einer Alkalihydroxid-Lösung zur Regeneration und vorzugsweise von nur einer Natriumhydroxid-Lösung als Regenerationsmittel keine saure Proteinfällung stattfindet und damit die oben beschriebenen Nachteile der Regeneration mit Glycin/HCl-Lösung nicht auftreten. Als Neutralisationslösung wurde PBS-Lösung (Variante B), Citrat-Lösung (Variante C) bzw. Natriumchlorid-Lösung (Variante D) verwendet. Die Vorteile der Verwendung einer Citrat-Lösung gegenüber einer PBS-Lösung liegen in der verringerten Neutralisationszeit und in dem verringerten benötigten Spülvolumen.

### Beispiel 2: Abwechselnde Verwendung der parallel geschalteten Apheresesäule

### Vorbereitung:

In die Apheresevorrichtung (II) zur extrakorporalen Entfernung von CRP aus Blut eines Patienten gemäß Fig. 13, mit einer Plasmazentrifuge als Zellseparator (7), wird ein passendes Schlauchsystem eingelegt. An die Anschlussleitung wird ein 5 L Beutel mit 0,9% NaCI-Lösung sowie ein 500 ml Beutel mit ACD-A Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden vor die Plasmaleitungen (8A und 8B) und nach dem Adsorber mit einem Adapter (ohne Adsorber dazwischen) verbunden sowie die Bypass-Leitungsabschnitte (12' und 12") der Bypass-Leitung (12) vor und nach dem Adsorber mit einem Adapter (ohne Adsorber dazwischen) verbunden, so dass ein geschlossenes System entsteht.

Durch Vorspülen mit 1 L 0,9 % NaCl Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in den die Bypass-Leitungsabschnitten (12' und 12") und in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte Natriumchlorid wird über P8/P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, den Bypass-Leitungsabschnitt 12' und P8/P4/P6 in den zweiten Abfallbeutel geleitet. Beginnt die Plasmaseparation, so wird nach einem Volumen, das der Schlauchleitung von der Plasmazentrifuge bis zum Punkt P8/P4/P6 entspricht, so umgeschaltet, dass das Plasma in die venöse Leitung (6), und damit zum Patienten zurück fließt. Nachdem für 3 Minuten ein konstanter Plasmafluss von ca. 30 ml/min erzielt wurde, kann der erste Zyklus beginnen.
2. Die Plasmaleitung (8A) in dem Bereich zwischen dem Knotenpunkt (P2) und dem Adsorber (4') wird geschlossen und das Plasma wird über den Adsorber (4") geleitet (Beladung). Dabei wird das in den Bypass-Leitungsabschnitten (12' und 12") und dem Adsorber (4") befindliche NaCl bis zu einem Volumen, bestehend aus dem Volumen der die Bypass-Leitungsabschnitte (12' und 12") plus dem AV, über P3/P4/P6 in den zweiten Abfallbeutel geleitet. Der Adsorber (4") wird anschließend mit 50 - 100 MV (1000 bis 2000 ml) Plasma beladen. Anschließend wird das Blutplasma mit der Natriumchlorid-Lösung aus dem Adsorber (4") verdrängt.
3. Es wird auf den zweiten Adsorber umgeschaltet und der Bypass-Leitungsabschnitt (12') in den Bereich zwischen dem Knotenpunkt (P2) und dem Adsorber (4") geschlossen. Das Plasma wird über den Adsorber (4') geleitet (Beladung). Dabei wird die in den Bypass-Leitungsabschnitten (12' und 12") und dem Adsorber (4') befindliche Natriumchlorid-Lösung bis zu einem Volumen, bestehend aus dem Volumen der Plasmaleitung (8A und 8B) plus dem AV, über P8/P4/P6 in den zweiten Abfallbeutel geleitet. Der Adsorber (4') wird anschließend mit 50 - 100 MV (1000 bis 2000 ml) Plasma beladen. Anschließend wird das Blutplasma mit der Natriumchlorid-Lösung aus dem Adsorber (4') verdrängt und dem Patienten zugeführt.
4. Während Adsorber (4') mit Plasma beladen wird, wird gleichzeitig Adsorber (4") regeneriert gemäß einem Verfahren entsprechend Beispiel 1 (Variante A, B, C, D). Wird Adsorber (4") beladen kann Adsorber (4') regeneriert werden.
5. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) verdrängt und dem Patienten zurückgeführt. Das dazu benötigte Volumen setzt sich zusammen aus dem Volumen der Plasmazentrifuge (7) und dem Volumen der Zellleitung (9) und der arteriellen Leitung (6). Der Patient kann anschließend von der Apheresevorrichtung getrennt werden.

Bei der Regeneration nach Variante A mit Glycin/HCl-Lösung wurden Proteinablagerungen auf der Adsorbermatrix beobachtet, welche auch nach einer längeren Regenerationsphase mit Glycin/HCl-Lösung nicht aufzulösen bzw. von der Adsorbermatrix abzulösen waren.

Derartige Proteinablagerungen waren bei den Regenerationen gemäß der Varianten B, C und D nicht zu beobachten und die regenerierten Adsorber gemäß Varianten B, C und D wiesen nach der Regeneration eine höhere CRP-Beladungskapazität auf, als die Adsorber nach Variante A.

### Regeneration eines mit Glycin/HCl-Lösung regenerierten Adsorbers mittels NaOH-Lösung

Der gemäß Variante A mittels Glycin/HCl-Lösung regenerierte Adsorber, welcher deutliche Proteinablagerungen auf dem Adsorber zeigte, wurde gemäß Variante B mittels einer Natriumhydroxid-Lösung regeneriert. Überraschend wurde gefunden, dass die vorhandenen Proteinablagerungen durch die Spülung mittels Natriumhydroxid-Lösung einer Konzentration von 0,1 mol/l wieder entfernt werden konnten.

Somit lassen sich mit einer Alkalihydroxid-Lösung auch Adsorber regenerieren, auf denen sich bereits Proteinablagerungen befinden.

### 6. Konservierung

### Variante A

Falls gewünscht, kann nun der Natriumchlorid-Lösungs-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Variante B

Über die Regenerationsleitung wird der Adsorber mit 10 MV Natriumhydroxid-Lösung als Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Beispiel 3: Abwechselnde Verwendung der parallel geschalteten Apheresesäulen (4', 4") und Regeneration im laufenden Betrieb

### Vorbereitung:

In die Apheresevorrichtung (II) zur extrakorporalen Entfernung von CRP aus Blut eines Patienten gemäß Fig. 16, mit einer Plasmazentrifuge als Zellseparator (7), wird ein passendes Schlauchsystem eingelegt. An die Anschlussleitung bzw. Regenerationsleitung wird ein 5 L Beutel mit 0,9% NaCI-Lösung sowie ein 500 ml Beutel mit ACD-A Lösung (Acid-Citrate-Dextrose Lösung) und/oder ein 2000 ml Beutel mit 0,08 M Natriumhydroxid-Lösung und/oder ein 2000 ml Beutel Glycin/HCl und/oder ein 2000ml Beutel PBS-Lösung angeschlossen. An die Abfallleitung (13) werden zwei 3 L Abfallbeutel angeschlossen (z. B. über einen 3 Wege-Hahn).

Die arterielle (5) und die venöse (6) Leitung werden mit einem Adapter miteinander verbunden. Ebenso werden die Bypass-Leitungsabschnitte (12' und 12") vor und nach dem Adsorber mit einem Adapter (ohne Adsorber dazwischen) verbunden sowie die Plasmaleitungen (8A und 8B) vor und nach dem Adsorber mit einem Adapter (ohne Adsorber dazwischen) verbunden, so dass ein geschlossenes System entsteht.

Durch Vorspülen mit 1 L 0,9 % NaCl Lösung (200 ml/Min) wird das gesamte System mit NaCl Lösung gefüllt; die vorhandene Luft wird in den ersten Abfallbeutel verdrängt. Anschließend wird anstelle des Adapters ein aufgeschüttelter CRP-Adsorber (MV 20 ml, AV 30 ml) in den Bypass-Leitungsabschnitten (12' und 12")und in die Plasmaleitung (8A und 8B) eingesetzt. Der Adsorber wird mit 1 L NaCl Lösung (100 ml/min) vorgespült. Das NaCl wird ebenfalls in den ersten Abfallbeutel geleitet.

Als letzter Schritt der Vorbereitung wird die Plasmazentrifuge mit 0,9% NaCl Lösung und 1:15 verdünnter ACD-A Lösung vorgefüllt. Das benötigte Volumen setzt sich zusammen aus dem Volumen des Schlauchsystems in der Plasmazentrifuge (7), der Anschlussleitung (11) bis zur Plasmazentrifuge und der Plasmaleitung zwischen der Plasmazentrifuge und P2. Das verdrängte Natriumchlorid wird über P8/P4/P6 in den ersten Abfallbeutel geleitet.

### Apherese:

1. Nach Ende der Vorbereitung wird auf den zweiten Abfallbeutel umgeschaltet. Der Patient wird an die arterielle (5) und die venöse (6) Leitung angeschlossen. Bei Beginn der Apherese wird das Blut in die Zentrifuge geleitet (60 - 80 ml/min). Während der gesamten Behandlung wird ACD-A im Verhältnis 1:15 (1 ml ACD-A auf 15 ml Blut) über die Anschlussleitung (11) dem Blut zugemischt.
   Das dadurch verdrängte NaCl wird über P2, der Bypass-Leitungsabschnitten (12') und P8/P4/P6 in den zweiten Abfallbeutel geleitet. Beginnt die Plasmaseparation, so wird nach einem Volumen, das der Schlauchleitung von der Plasmazentrifuge bis zum Punkt P8/P4/6 entspricht, so umgeschaltet, dass das Plasma in die venöse Leitung (6), und damit zum Patienten zurück, fließt. Nachdem für 3 Minuten ein konstanter Plasmafluß von ca. 30 ml/min erzielt wurde, kann der erste Zyklus beginnen.
2. Die Plasmaleitung (8A) wird geschlossen und das Plasma wird über den Adsorber (4") geleitet (Beladung). Dabei wird das in den Bypass-Leitungsabschnitten (12' und 12") und dem Adsorber (4") befindliche NaCl bis zu einem Volumen, bestehend aus dem Volumen der Bypass-Leitungsabschnitten (12' und 12") plus dem AV, über P8/P4/P6 in den zweiten Abfallbeutel geleitet. Der Adsorber wird anschließend mit 50 - 100 MV (1000 bis 2000 ml) Plasma beladen. Anschließend wird das Blutplasma mit der Natriumchlorid-Lösung aus dem Adsorber (4") verdrängt.
3. Es wird auf den zweiten Adsorber umgeschaltet und der Bypass-Leitungsabschnitt (12') in den Bereich zwischen dem Knotenpunkt (P2) und dem Adsorber (4") geschlossen. Das Plasma wird über den Adsorber (4') geleitet (Beladung). Dabei wird die in den Bypass-Leitungsabschnitten (12' und 12") und dem Adsorber (4") befindliche Natriumchlorid-Lösung bis zu einem Volumen, bestehend aus dem Volumen der Plasmaleitung (8A und 8B) plus dem AV, über P8/P4/P6 in den zweiten Abfallbeutel geleitet. Der Adsorber (4') wird anschließend mit 50 - 100 MV (1000 bis 2000 ml) Plasma beladen. Anschließend wird das Blutplasma mit der Natriumchlorid-Lösung aus dem Adsorber (4') verdrängt und dem Patienten zugeführt.

### Regeneration

### Variante A - Glycin/HCl und PBS-Lösung

Zeitgleich wird der Adsorber (4") nun über die Regenerationsleitung (14) und die Bypass-Leitungsabschnitte (12' und 12") mit 0,9% NaCl gespült (30 ml/min). Das dafür benötigte Volumen berechnet sich aus dem AV und dem Volumen der Bypass-Leitungsabschnitte (12' und 12"). Das in den Bypass-Leitungsabschnitten (12' und 12") und dem Adsorber (4") befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.
Im nächsten Schritt wird mit 3 MV (60 ml) 0,9% NaCl und danach mit 4 MV (80ml) Glycin/HCl regeneriert (100 ml/min). Anschließend wird mit 5 MV PBS neutralisiert. Danach wird mit 0,9% NaCl nachgespült (100 ml/min). Das dafür benötigte Volumen berechnet sich aus dem AV, dem Volumen der Regenerationsleitung (14) und der Plasmaleitung (8A und 8B).

Anschließend kann wieder Schritt 2 (Beladung), danach Schritt 3 erfolgen.

### Variante B - Natriumhydroxid-Lösung und PBS-Lösung

Zeitgleich wird der Adsorber (4") nun über die Regenerationsleitung (14) und die Bypass-Leitungsabschnitte (12' und 12") mit 0,9% NaCl gespült (30 ml/min-40ml/min). Das dafür benötigte Volumen berechnet sich aus dem AV und dem Volumen der Bypass-Leitungsabschnitte (12' und 12"). Das in den Bypass-Leitungsabschnitten (12' und 12").) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 3 MV (60 ml) 0,9% NaCl vorgespült und danach mit 5 MV 0,08M NaOH (pH 12,6; Flussrate von 80 ml/min) regeneriert. Danach wird mit 6 MV PBS-Lösung (pH 12,6; Flussrate 80ml/min) neutralisiert. Anschließend wird das PBS mit 4 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung bzw. PBS-Lösung ersetzt werden.

### Variante C - Natriumhydroxid-Lösung und Citrat-Lösung

Zeitgleich wird der Adsorber (4") nun über die Regenerationsleitung (14) und die Bypass-Leitungsabschnitte (12' und 12") mit 0,9% NaCl gespült (30 ml/min-40ml/min). Das dafür benötigte Volumen berechnet sich aus dem AV und dem Volumen der Bypass-Leitungsabschnitte (12' und 12"). Das in den Bypass-Leitungsabschnitten (12' und 12").) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 3 MV (60 ml) 0,9% NaCl vorgespült und danach mit 5 MV 0,1 M NaOH (pH 12,9; Flussrate von 80 ml/min) regeneriert. Danach wird mit 4 MV 4% Citrat-Lösung (pH 7; Flussrate 80ml/min) neutralisiert. Anschließend wird das Citrat mit 3 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung bzw. Citrat-Lösung ersetzt werden.

### Variante D - Natriumhydroxid-Lösung und NaCl-Lösung

Zeitgleich wird der Adsorber (4") nun über die Regenerationsleitung (14) und die Bypass-Leitungsabschnitte (12' und 12") mit 0,9% NaCl gespült (30 ml/min-40ml/min). Das dafür benötigte Volumen berechnet sich aus dem AV und dem Volumen der Bypass-Leitungsabschnitte (12' und 12"). Das in den Bypass-Leitungsabschnitten (12' und 12").) und dem Adsorber befindliche Plasma wird bis zu einem Volumen, bestehend aus dem AV und 75% des Volumens der Plasmaleitung (8A und 8B), ebenfalls zum Patienten zurückgeleitet. Anschließend wird P4/P6 so umgeschaltet, dass die Lösungen in den zweiten Abfallbeutel geleitet werden.

Im nächsten Schritt wird mit 2 MV (60 ml) 0,9% NaCl vorgespült und danach mit 5 MV 0,1 M NaOH (pH 12,9; Flussrate von 80 ml/min) regeneriert. Anschließend wird das Natriumhydroxid mit 6 MV 0,9% NaCl (Flussrate 80ml/min) verdrängt.

Anschließend kann wieder Schritt 2 (Beladung), danach Schritt 3 erfolgen. Bei Bedarf muss der Beutel mit Natriumhydroxid-Lösung ersetzt werden.
4. Nachdem die letzte Beladung erfolgte, wird eine abschließende Regeneration durchgeführt. Gleichzeitig wird die arterielle Leitung (5) geschlossen. Über die Anschlussleitung (11) wird mit 0,9% NaCl (30 ml/min) das Blut aus der Plasmazentrifuge (7) über die Zellleitung (9) verdrängt und dem Patienten zurückgeführt. Das dazu benötigte Volumen setzt sich zusammen aus dem Volumen der Plasmazentrifuge (7) und dem Volumen der Zellleitung (9) und der arteriellen Leitung (6). Der Patient kann anschließend von der Apheresevorrichtung getrennt werden.
5. Konservierung

### Variante A

Falls gewünscht, kann nun der Natriumchlorid-Lösungs-Beutel durch einen Beutel mit Konservierungslösung (z. B. PBS mit Na-Azid) ersetzt werden. Über die Regenerationsleitung wird der Adsorber mit 10 MV Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Variante B

Über die Regenerationsleitung wird der Adsorber mit 10 MV Natriumhydroxid-Lösung als Konservierungslösung gespült (in den zweiten Abfallbeutel). Anschließend wird der Adsorber entnommen, verschlossen und gelagert. Das Schlauchsystem wird aus der Apheresevorrichtung entfernt und entsorgt.

### Ergebnisse:

Bei der Regeneration nach Variante A mit Glycin/HCl-Lösung wurde die Bildung einer Proteinschicht um die Matrixpartikel (Agarosepartikel) beobachtet. Dies beruht vermutlich aufgrund des sehr niedrigen pH-Wertes von pH 2-3 auf einer sauren Proteinfällung. Wenn das zu reinigende Blut des Patienten eine hohe Konzentration von zellfreier DNA/RNA enthielt, führte dies zu einer Verstärkung des Effekts. Es hat sich gezeigt, dass durch die Ausbildung der Proteinschicht in der Apheresesäule Bindungsstellen maskiert werden und die Leistung des Apheresematerials verringert wird. Der ursprüngliche Zustand konnte mit bekannten Maßnahmen wie weiteren Regenerationsversuchen mit einer Glycin/HCl-Lösung nicht wiederhergestellt werden. Mit fortschreitender Schädigung der Apheresesäule erhöht sich die Behandlungszeit für den Patienten und somit die Leidenszeit des Patienten. Zudem waren die beschädigten Apheresesäulen für einen weiteren Einsatz oft nicht mehr brauchbar, sodass die Behandlungskosten erheblich steigen. Weiterhin kann es durch die Proteinschicht bzw. Protein-DNA sowie Protein-RNA Schicht zu einer Verstopfung der feinen Poren kommen, wodurch der Systemdruck bei gleichbleibender Flussrate steigt. Eine weitere Erhöhung der Flussrate geht mit einer weiteren Druckerhöhung einher. Dies kann zu einem Abbruch der Behandlung führen. Diese Apheresesäulen waren ebenfalls für einen weiteren Einsatz nicht mehr brauchbar.

Bei der Regeneration nach Variante B, C und D mit Natriumhydroxid-Lösung hat sich gezeigt, dass einerseits eine basische Regeneration mit Natriumhydroxid-Lösung auch eine bereits geschädigte Adsorbermatrix regenerieren kann. Überraschenderweise hat sich gezeigt, dass bei der Verwendung von nur Natriumhydroxid-Lösung als Regenerationsmittel keine saure Proteinfällung stattfinden und damit die oben beschriebenen Nachteile der Regeneration mit Glycin/HCl-Lösung nicht auftreten. Als Neutralisationslösung wurde PBS-Lösung (Variante B), Citrat-Lösung (Variante C) bzw. Natriumchlorid-Lösung (Variante D) verwendet. Die Vorteile der Verwendung einer Citrat-Lösung gegenüber einer PBS-Lösung liegen in der verringerten Neutralisationszeit und in dem verringerten benötigten Spülvolumen.

### Beschreibung der Figuren

- **Fig. 1:**: **Schema-Zeichnung** einer Ausführungsform der erfindungsgemäßen Apheresevorrichtung (1) zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P4) von der Plasmaleitung (8B) ab. Zusätzlich mündet die Regenerationsleitung (14) zum Anschluss eines Flüssigkeitsbehälters (F2) in einem Bereich zwischen dem Knotenpunkt (P2) und der Apheresesäule (4) in die Plasmaleitung (8A). Alternativ kann die Regenerationsleitung (14) auch direkt in die Apheresesäule (4) führen (nicht dargestellt).
- **Fig. 2:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich mündet die Regenerationsleitung (14) an dem Knotenpunkt (P2) in die Plasmaleitung (8A).
- **Fig. 3:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P3) in die Zellleitung (9). Die Abfallleitung (13) zweigt an dem Knotenpunkt (P1) von der Plasmaleitung (8B) ab. Zusätzlich mündet die Regenerationsleitung (14), welche an dem Punkt (P5) von der Anschlussleitung (11) abzweigt, an dem Knotenpunkt (P2) in die Plasmaleitung (8A).
- **Fig. 4:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich münden eine erste Regenerationsleitung (14'), welche an dem Punkt (P5') von der Anschlussleitung (11') abzweigt, und eine zweite Regenerationsleitung (14"), welche an dem Punkt (P5") von der Anschlussleitung (11") abzweigt, beide an dem Knotenpunkt (P2) in die Plasmaleitung (8A).
- **Fig. 5:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. eine Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4) zur affinitätschromatographischen Entfernung von CRP aus dem Blut. Von dieser führt die Plasmaleitung (8B) zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Die Bypass-Leitung (12) zweigt an dem Knotenpunkt (P2) von der Plasmaleitung (8A) ab und mündet an dem Knotenpunkt (P6) in die Plasmaleitung (8B). Die Abfallleitung (13) zweigt an dem Knotenpunkt (P6) von der Plasmaleitung (8B) ab. Zusätzlich mündet die Regenerationsleitung (14), welche an dem Punkt (P5) von der Anschlussleitung (11) abzweigt, an dem Knotenpunkt (P2) in die Plasmaleitung (8A). Zur verbesserten Übersichtlichkeit ist eine zur erfindungsgemäßen Apheresevorrichtung gehörende zentrale Recheneinheit nicht dargestellt. Die Regenerationsleitung (14) weist einen zusätzlichen Anschluss für einen Flüssigkeitsbehälter (F2) auf, wobei dieser Anschluss in Flussrichtung hinter dem Zellseparator (7) liegt, so dass Flüssigkeit aus diesem zusätzlichen Flüssigkeitsbehälter (F2) nicht in den Zellseparator (7) geleitet und nicht vor dem Zellseparator (7) in die arterielle Leitung (5) geführt werden kann, sondern nur in die Plasmaleitung (8A) in Flussrichtung hinter dem Zellseparator (7) oder direkt in die Apheresesäule (4).
- **Fig. 6:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13") zweigt an dem Knotenpunkt (P8) von dem Bypass-Leitungsabschnitt (12') der Bypass-Leitung (12) ab und die Abfallleitung (13') zweigt an dem Knotenpunkt (P4) von der Plasmaleitung (8B). Zusätzlich mündet die Regenerationsleitung (14) zum Anschluss eines Flüssigkeitsbehälters (F2) an dem Knotenpunkt (P2) in das extrakorporale Zirkulationssystem (2).
- **Fig. 7:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13") zweigt an dem Knotenpunkt (P8) von dem Bypass-Leitungsabschnitt (12") der Bypass-Leitung (12) ab und die Abfallleitung (13') zweigt an dem Knotenpunkt (P4) von der Plasmaleitung (8B). Zusätzlich führt die Regenerationsleitung (14) zum Anschluss eines Flüssigkeitsbehälters (F2) bis zu dem Knotenpunkt (P7). Am Knotenpunkt (P7) zweigen zwei Leitungen (15', 15") ab. Die Leitung (15') mündet am Knotenpunkt (P2) in das extrakorporale Zirkulationssystem (2) und die Leitung (15") mündet in dem Bereich zwischen dem Knotenpunkt (P2) und der Apheresesäule (4").
- **Fig. 8:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A") laufen am Knotenpunkt (P2) auseinander und am Knotenpunkt (P6) laufen der Bypass-Leitungsabschnitt (12") der Bypass-Leitung (12) und die Plasmaleitung (8B") zusammen. Die Abfallleitung (13) zweigt an dem Knotenpunkt (P6) vom extrakorporalen Zirkulationssystem (2) ab. Zusätzlich mündet die Regenerationsleitung (14) zum Anschluss eines Flüssigkeitsbehälters (F2) an dem Knotenpunkt (P2) in das extrakorporale Zirkulationssystem (2).
- **Fig. 9:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator). Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11'), die in die arterielle Leitung (5) mündet aber auch direkt in den Zellseparator (7) hätte münden können, sowie eine Anschlussleitung (11"), die in den Zellseparator (7) mündet aber auch in die arterielle Leitung (5) hätte münden können. Der Bypass-Leitungsabschnitt (12') der Bypass-Leitung (12) und die Plasmaleitung (8A) laufen am Knotenpunkt (P2) auseinander und am Knotenpunkt (P6) laufen die Plasmaleitung (8B') und die Plasmaleitung (8B") zusammen. Die Abfallleitung (12) zweigt an dem Knotenpunkt (P6) vom extrakorporalen Zirkulationssystem (2) ab. Zusätzlich münden eine erste Regenerationsleitung (14'), welche an dem Punkt (P5') von der Anschlussleitung (11') abzweigt, und eine zweite Regenerationsleitung (14"), welche an dem Punkt (P5") von der Anschlussleitung (11") abzweigt, beide an dem Knotenpunkt (P2) in das extrakorporale Zirkulationssystem (2).
- **Fig. 10:**: **Schema-Zeichnung** einer Ausführungsform der erfindungsgemäßen Apheresevorrichtung zur extrakorporalen Entfernung von CRP aus Blut. Die arterielle Leitung (5), in der sich ein Mittel (3) zur Generierung und Regulation eines Flusses des Blutes (z.B. Peristaltikpumpe) befindet, führt das Blut eines Patienten zu dem Zellseparator (7, z.B. ein zentrifugaler Zellseparator Von diesem führt die Plasmaleitung (8A) zu der Apheresesäule (4') zur affinitätschromatographischen Entfernung von CRP. Der Bypass-Leitungsabschnitt (12') der Bypass-Leitung (12) zweigt von der Plasmaleitung (8A) ab führt zu der Apheresesäule (4") zur affinitätschromatographischen Entfernung von CRP aus dem Blut. Von der Apheresesäule (4") führt der Bypass-Leitungsabschnitt (12") der Bypass-Leitung (12) für CRP-abgereichertes Blutplasma zu dem Knotenpunkt (P1) und von der Apheresesäule (4') führt die Plasmaleitung (8B) für CRP-abgereichertes Blutplasma zu dem Knotenpunkt (P1). Eine weitere Leitung, die Zellleitung (9), führt von dem Zellseparator (7) zu dem Knotenpunkt (P1). Von dem Knotenpunkt (P1) geht ebenfalls die venöse Leitung (6) ab, die das behandelte Blut wieder zurück in den Patienten führt. Zusätzlich existiert eine Anschlussleitung (11) zum Anschluss eines Flüssigkeitsbehälters (F1), die in die arterielle Leitung (5) mündet oder alternativ direkt in den Zellseparator (7) führt (gestrichelte Linie). Der Bypass-Leitungsabschnitt (12') der Bypass-Leitung (12) und die Plasmaleitung (8A) laufen am Knotenpunkt (P2) auseinander und am Knotenpunkt (P6) laufen der Bypass-Leitungsabschnitt (12") der Bypass-Leitung (12) und die Plasmaleitung (8B) zusammen. Die Abfallleitung (13) zweigt an dem Knotenpunkt (P6) vom extrakorporalen Zirkulationssystem (2) ab. Zusätzlich mündet die Regenerationsleitung (14), welche an dem Punkt (P5) von der Anschlussleitung (11) abzweigt, an dem Knotenpunkt (P2) in das extrakorporale Zirkulationssystem (2). Die Zulaufleitung weist einen zusätzlichen Anschluss für einen Flüssigkeitsbehälter (F2) auf, wobei dieser Anschluss in Flussrichtung hinter dem Zellseparator (7) liegt, sodass Flüssigkeit aus diesem zusätzlichen Flüssigkeitsbehälter nicht in den Zellseparator (7) und nicht vor dem Zellseparator (7) in die arterielle Leitung (5) geführt werden kann, sondern nur in den Bypass-Leitungsabschnitt (12') der Bypass-Leitung (12) oder in die Plasmaleitung (8A) in Flussrichtung hinter dem Zellseparator (7) oder direkt in die Apheresesäule (4') oder direkt in die Apheresesäule (4").

### Verzeichnis der Bezugszeichen

- 1 -: Apheresevorrichtung
- 2 -: extrakorporales Zirkulationssystem
- 3 -: Mittel zur Generierung und Regulation eines Flusses von Blut (oder Blutplasma) in dem extrakorporalen Zirkulationssystem (Pumpe)
- 4 -: Apheresesäule zur affinitätschromatographischen Entfernung von CRP
- 4' -: Apheresesäule zur affinitätschromatographischen Entfernung von CRP
- 4" -: Apheresesäule zur affinitätschromatographischen Entfernung von CRP
- 5 -: arterielle Leitung
- 6 -: venöse Leitung
- 7 -: Zellseparator
- 8A -: Plasmaleitung (vor der Apheresesäule)
- 8B -: Plasmaleitung (nach der Apheresesäule)
- 9: Zellleitung
- 11 -: Anschlussleitung
- 12 -: Bypass-Leitung
- 12' -: Bypass-Leitungsabschnitt der Bypass-Leitung
- 12" -: Bypass-Leitungsabschnitt der Bypass-Leitung
- 13 -: Abfallleitung
- 13' -: Abfallleitung
- 13" -: Abfallleitung
- 14 -: Regenerationsleitung
- 14' -: Regenerationsleitung
- 14" -: Regenerationsleitung
- F -: Flüssigkeitsbehälter
- F1 -: Flüssigkeitsbehälter 1
- F2 -: Flüssigkeitsbehälter 2

- P1 -: Knotenpunkt, an dem die Plasmaleitung (8B) in die venöse Leitung (6)übergeht oder Knotenpunkt, an dem der Bypass-Leitungsabschnitt (12") der Bypass-Leitung (12) oder (8B) und die Zellleitung (9) zusammenlaufen und in die venöse Leitung (6) übergehen
- P2 -: Knotenpunkt, an dem die Bypass-Leitung (12) von der Plasmaleitung (8A) abzweigt oder Knotenpunkt, an dem der Bypass-Leitungsabschnitt (12') der Bypass-Leitung und die Plasmaleitung (8A) auseinanderlaufen
- P3 -: Knotenpunkt, an dem die Bypass-Leitung (12) in die Zellleitung (9) mündet
- P4 -: Knotenpunkt, an dem die Abfallleitung (13) von der Plasmaleitung (8B) abzweigt oder Knotenpunkt, an dem die Abfallleitung (13') von der Plasmaleitung (8B) abzweigt.
- P5 -: Knotenpunkt, an dem die Regenerationsleitung (14) von der Anschluss-Leitung (11) abzweigt
- P5, P5' -: Knotenpunkt, an dem die Regenerationsleitung (14) von der Anschlussleitung (11) bzw. (11') abzweigt.
- P6 -: Knotenpunkt, an dem die Bypass-Leitung (12) in die Plasmaleitung (8B) mündet oder Knotenpunkt, an dem der Bypass-Leitungsabschnitt (12") der Bypass-Leitung (12) und die Plasmaleitung (8B) zusammenlaufen und zusammen als Bypass-Leitungsabschnitt (12") der Bypass-Leitung (12) oder (8B) bis zum Punkt P1 laufen.
- P7 -: Knotenpunkt in der Regenerationsleitung (14), ab dem sich die Regenerationsleitung (14) die Leitungen (15') und (15") teilt
- P8 -: Knotenpunkt, an dem die Abfallleitung (13") von dem Bypass-Leitungsabschnitt (12") der Bypass-Leitung abzweigt.

## Patentansprüche

1. Verwendung einer Alkalihydroxid-Lösungzur Regeneration einer Apheresesäule.

2. Verwendung einer Alkalihydroxid-Lösung gemäß Anspruch 1, wobei es sich bei der Apheresesäule um eine Apheresesäule zur affinitätschromatographischen Entfernung von CRP handelt.

3. Verwendung einer Alkalihydroxid-Lösung gemäß Anspruch 1 oder 2, wobei es sich bei der Alkalihydroxid-Lösung um eine Lithiumhydroxid-Lösung, eine Natriumhydroxid-Lösung, eine Kaliumhydroxid-Lösung oder eine Mischung vorgenannter Lösungen handelt.

4. Verwendung einer Alkalihydroxid-Lösung gemäß einem der Ansprüche 1 - 3, wobei die Konzentration des Alkalihydroxids in der Alkalihydroxid-Lösung in einem Bereich von 0,01 mol/l bis 1 mol/l liegt.

5. Verwendung einer Alkalihydroxid-Lösung gemäß einem der Ansprüche 1 - 4, wobei die Alkalihydroxid-Lösung einen pH-Wert in einem Bereich von 12 bis 14 aufweist.

6. Verwendung einer Alkalihydroxid-Lösung gemäß einem der Ansprüche 1 - 5, wobei die Apheresesäule ein Matrixsubstratmaterial funktionalisiert mit mindestens einer ω-Phosphonooxyalkylammoniumgruppe und/oder mindestens einer ω-Ammoniumalkoxy-hydroxyphosphoryloxygruppe umfasst.

7. Verwendung einer Alkalihydroxid-Lösung gemäß Anspruch 6, wobei die mindestens eine ω-Phosphonooxyalkylammoniumgruppe einer Gruppe der folgenden allgemeinen Formel (I) entspricht 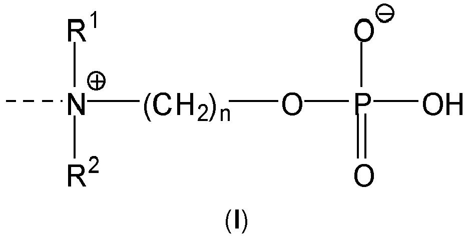 wobei
n ausgewählt wird aus 2 und 3;
R¹ und R² unabhängig voneinander ausgewählt werden aus: -H, -CH₃, -C₂H₅, -C₃H₇, -C₄H₉, -C₅H₁₁, -C₆H₁₃, oder R¹ und R² zusammen mit dem Stickstoffatom mit dem sie verbunden sind einen Heterozyklus bilden können, der ausgewählt wird aus: 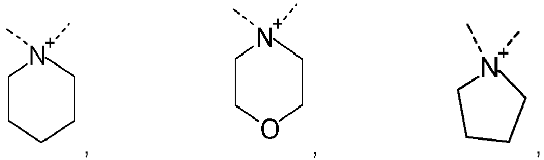 wobei ein oder mehrere Wasserstoffatom(e) durch (ein) Fluoratom(e) ersetzt werden können.

8. Verwendung einer Alkalihydroxid-Lösung gemäß Anspruch 6, wobei die mindestens eine ω-Ammoniumalkoxy-hydroxyphosphoryloxygruppe einer Gruppe der folgenden allgemeinen Formel (II) entspricht 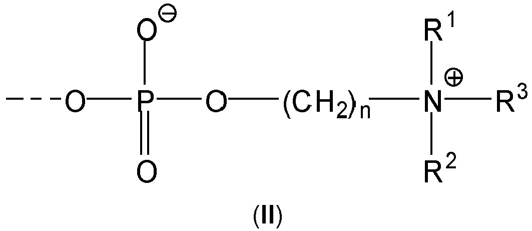 wobei
n ausgewählt wird aus 2 und 3;
R¹, R² und R³ unabhängig voneinander ausgewählt werden aus: -H, -CH₃, - C₂H₅,
-C₃H₇, -C₄H₉, -C₅H₁₁, -C₆H₁₃,
oder R¹ und R² zusammen mit dem Stickstoffatom mit dem sie verbunden sind einen Heterozyklus bilden können, der ausgewählt wird aus: 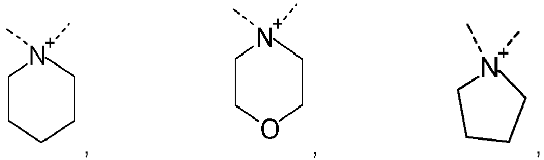 und
R³ ausgewählt wird aus: -H, -CH₃, -C₂H₅, -C₃H₇, -C₄H₉, -C₅H₁₁, -C₆H₁₃, und vorzugsweise -H;
wobei ein oder mehrere Wasserstoffatom(e) durch (ein) Fluoratom(e) ersetzt werden können.

9. Verwendung einer Alkalihydroxid-Lösung gemäß einem der Ansprüche 1 - 8, wobei die Apheresesäule Agarose enthält.

10. Verwendung einer Alkalihydroxid-Lösung gemäß einem der Ansprüche 1 - 9, wobei die Apheresesäule DNA und/oder RNA enthält.

11. Apheresevorrichtung (1) zur extrakorporalen Entfernung von CRP aus Blut umfassend:
ein extrakorporales Zirkulationssystem (2) für Blut,
Mittel (3) zur Generierung und Regulation eines Flusses des Blutes in dem extrakorporalen Zirkulationssystem (2),
einen Zellseparator (7) zur Auftrennung des Bluts in Blutplasma und zelluläre Bestandteile,
mindestens eine Apheresesäule (4) zur affinitätschromatographischen Entfernung von CRP aus dem Blut,
wobei das extrakorporale Zirkulationssystem (2) eine arterielle Leitung (5) zu dem Zellseparator (7), eine Plasmaleitung (8A) von dem Zellseparator (7) zur Apheresesäule (4), eine Plasmaleitung (8B) für CRP-abgereichertes Blutplasma von der Apheresesäule (4) bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F1) an die arterielle Leitung (5) oder den Zellseparator (7),
**dadurch gekennzeichnet, dass**
eine Bypass-Leitung (12) von der Plasmaleitung (8A) abzweigt und in die Plasmaleitung (8B) mündet,
eine Abfallleitung (13) direkt von der Apheresesäule (4) abgeht oder von der Plasmaleitung (8B) in Flussrichtung vor der Einmündung der Bypass-Leitung (12) abgeht, und
zumindest eine Regenerationsleitung (14), die in Flussrichtung an oder nach der Abzweigung der Bypass-Leitung (12) zu der Plasmaleitung (8A) führt oder direkt in die Apheresesäule (4) mündet, wobei die Apheresevorrichtung derart ausgestaltet ist, dass diese gegenüber einer Alkalihydroxid-Lösung beständig ist.

12. Die Apheresevorrichtung (1) gemäß Anspruch 11, **dadurch gekennzeichnet, dass** die zumindest eine Regenerationsleitung (14) von dem zumindest einen Flüssigkeitsbehälter (F1) oder der zumindest einen Anschlussleitung (11) abgeht und
die zumindest eine Regenerationsleitung (14) zumindest einen zusätzlichen Anschluss für einen Flüssigkeitsbehälter (F2) aufweist.

13. Apheresevorrichtung (II) zur extrakorporalen Entfernung von CRP aus Blut umfassend:
ein extrakorporales Zirkulationssystem (2) für Blut,
ein Mittel (3) zur Generierung und Regulation eines Flusses des Blutes in dem extrakorporalen Zirkulationssystem (2),
einen Zellseparator (7) zur Auftrennung von Blut in Blutplasma und zelluläre Bestandteile,
zwei Apheresesäulen (4', 4") zur affinitätschromatographischen Entfernung von CRP aus dem Blutplasma,
wobei das extrakorporale Zirkulationssystem (2) eine arterielle Leitung (5) zu dem Zellseparator (7), eine Plasmaleitung (8A) von dem Zellseparator (7) zu der Apheresesäule (4'), eine Plasmaleitung (8B) für CRP-abgereichertes Blutplasma von der Apheresesäule (4') bis zu einem Punkt (P1), eine Zellleitung (9) für die abgetrennten zellulären Bestandteile von dem Zellseparator (7) bis zu dem Punkt (P1) und eine venöse Leitung (6) ausgehend von dem Punkt (P1) umfasst,
zumindest eine Anschlussleitung (11) zum Anschluss von zumindest einem Flüssigkeitsbehälter (F) an die arterielle Leitung (5) oder den Zellseparator (7),
**dadurch gekennzeichnet, dass**
eine Bypass-Leitung (12) von der Plasmaleitung (8A) abzweigt und in die Plasmaleitung (8B) mündet, und die Bypass-Leitung (12) die zweite Apheresesäule (4") umfasst,
eine Abfallleitung (13) direkt von der Apheresesäule (4') abgeht oder von der Plasmaleitung (8B) in Flussrichtung vor der Einmündung der Bypass-Leitung (12) abgeht, und
zumindest eine Regenerationsleitung (14), die in Flussrichtung nach der Abzweigung der Bypass-Leitung (12) zu der Plasmaleitung (8A) führt oder direkt in die Apheresesäule (4') mündet, und
wobei eine zweite Apheresesäule (4") parallel zur ersten Apheresesäule (4') geschaltet ist und beide Apheresesäulen (4', 4") nicht zeitgleich zur CRP-Entfernung nutzbar sind, wobei die Apheresevorrichtung derart ausgestaltet ist, dass diese gegenüber einer Alkalihydroxid-Lösung beständig ist.

14. Verfahren zur Regeneration einer Apheresesäule (4) zur affinitätschromatographischen Entfernung von CRP in einer Apheresevorrichtung (1) gemäß einem der Ansprüche 11 oder 12, wobei das Verfahren die Regeneration im laufenden Betrieb ermöglicht und durch die folgenden Schritte gekennzeichnet ist:
(A) Beginn der Umleitung des abgetrennten Plasmas aus der Plasmaleitung (8A) in die Bypass-Leitung (12) und damit Stoppen der Einleitung des abgetrennten Plasmas aus der Plasmaleitung (8A) in die Apheresesäule (4),
(B) Beginn der Einleitung von Regenerationslösung über die zumindest eine Regenerationsleitung (14) in die Plasmaleitung (8A) oder direkt in die Apheresesäule (4), wobei die Regenerationslösung eine Alkalihydroxid-Lösung ist,
(C) Beginn der Umleitung des aus der Apheresesäule (4) austretenden Flüssigkeitsstromes von der Plasmaleitung (8B) in die Abfallleitung (13),
(D) Stoppen der Einleitung von Regenerationslösung,
(E) Beginn der Einleitung einer Neutralisationslösung,
(F) Stoppen der Einleitung der Neutralisationslösung und Stoppen der Umleitung des abgetrennten Plasmas aus der Plasmaleitung (8A) in die Bypass-Leitung (12) und damit Einleitung des abgetrennten Plasmas aus der Plasmaleitung (8A) in die Apheresesäule (4),
(G) Schließung der Abfallleitung (13).

15. Verfahren gemäß Anspruch 14 zur Regeneration einer Apheresesäule (4) zur affinitätschromatographischen Entfernung von CRP in einer Apheresevorrichtung (1) gemäß einem der Ansprüche 11 oder 12, wobei das Verfahren durch die folgenden Schritte gekennzeichnet ist:
(A) Beginn der Umleitung des abgetrennten Plasmas aus der Plasmaleitung (8A) in die Bypass-Leitung (12) und damit Stoppen der Leitung des abgetrennten Plasmas aus der Plasmaleitung (8A) in die Apheresesäule (4),
(B) Beginn der Einleitung einer Spüllösung über die zumindest eine Regenerationsleitung (14) in die Plasmaleitung (8A) oder direkt in die Apheresesäule (4),
(C) Beginn der Umleitung des aus der Apheresesäule (4) austretenden Flüssigkeitsstromes von der Plasmaleitung (8B) in die Abfallleitung (13),
(D) Stoppen der Einleitung der Spüllösung und Übergang zur Einleitung einer Regenerationslösung über die zumindest eine Regenerationsleitung (14) in die Plasmaleitung (8A) oder direkt in die Apheresesäule (4), wobei die Regenerationslösung eine Alkalihydroxid-Lösung ist,
(E) Stoppen der Einleitung der Regenerationslösung und Übergang zur Einleitung der Neutralisationslösung über die zumindest eine Regenerationsleitung (14) in die Plasmaleitung (8A) oder direkt in die Apheresesäule (4),
(F) Stoppen der Einleitung der Neutralisationslösung und Übergang zur Einleitung der Spüllösung über die zumindest eine Regenerationsleitung (14) in die Plasmaleitung (8A) oder direkt in die Apheresesäule (4),
(G) Stoppen der Einleitung der Spüllösung und Stoppen der Umleitung des abgetrennten Plasmas aus der Plasmaleitung (8A) in die Bypass-Leitung (12) und damit Leitung des abgetrennten Plasmas aus der Plasmaleitung (8A) in die Apheresesäule (4);
(H) Schließung der Abfallleitung (13).
